# EUROPEAN PATENT APPLICATION

(11) **EP 4 512 403 A1**
(43) Date of publication of application: **26.02.2025**
(21) Application number: 23192592.6
(22) Date of filing: 22.08.2023
(51) Int. Cl.: A61K 31/50, A61K 48/00, A61P 25/00, A61P 25/16, A61P 25/22, A61P 25/24

(54) **NEUROPEPTIDE B AND W-RECEPTOR AS A TARGET FOR TREATING MOOD DISORDERS AND/OR CHRONIC STRESS**

(71) Applicant: Friedrich-Schiller-Universität Jena, 07743 Jena (DE)
(72) Inventor: ENGMANN, Olivia, 07743 Jena (DE)
(74) Representative: Vossius & Partner Patentanwälte Rechtsanwälte mbB

(57) **Abstract**

Described is a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease. Moreover, described is a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia. Further, described is a method for assessing the activity of a candidate molecule suspected of being an antagonist/inhibitor or an agonist/activator of NPBWR1.

## Description

The present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease. Moreover, the present invention relates to a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia. Further, the present invention relates to a method for assessing the activity of a candidate molecule suspected of being an antagonist/inhibitor or an agonist/activator of NPBWR1.

Chronic stress is a main risk factor for mental illnesses including major depressive-disorder (depression, MDD), the leading cause for disability and suicide ^{1,2}. Chronic stress, and associated illnesses like anxiety disorders, are on the rise ³⁻⁹ and are among the largest categories of health care expenditure ^{10,11}. Neural signatures of chronic stress can be modeled reliably in mice ^{12,13}. Moreover, it has previously been observed that caffeine rapidly affects mood-related behavior. This effect was shown to be independent of locomotor changes ¹⁴. In the nucleus accumbens (NAc), the center of the brain reward system, caffeine altered binding of the CLOCK/BMAL transcription factor complex to the chromatin. This mechanism has been shown to be dependent on Thr75-phosphorylation of DARPP-32 and occurs in a diurnal manner ¹⁴. However, whether the affected genes are functionally relevant to mood and associated disorders, remains unknown. Moreover, it is little understood how chronic stress and depression can be ameliorated by rapid environmental interventions. Further, within a growing battery of antidepressant drugs, only the controlled substance ketamine has a fast-acting mechanism.

In light of the prior art, there is a need to provide further means and methods for treating, ameliorating or preventing a mood disorder/affective disorder and/or chronic stress and/or anxiety disorders.

The present invention meets this demand and is based on the surprising finding that the receptor for neuropeptides B and W (*Npbwr1,* also called GPR7) is a key mediator of depression and stress response and that corresponding antagonists like, e.g., CYM50769, are potential compounds to rapidly modulate this pathway in a beneficial manner, thereby identifying a new, fast-acting pathway, which rapidly ameliorates symptoms of stress and depression.

More specifically, as exemplified in the appended Examples below, transcriptomic signatures, which were rapidly altered by caffeine in the NAc in a T75-DARPP-32 and light-phase dependent manner were determined. Surprisingly, We the receptor for neuropeptides B and W (*Npbwr1,* also called GPR7) was identified as a key mediator of mood-related states in mice. This finding of the present invention is all the more surprising because Npbwr1 is a scarcely studied G-protein coupled receptor for neuropeptides B (NPB) and W (NPW) ¹⁵. *Npbwr1* is expressed in a variety of brain areas including the NAc ¹⁶. Incidental studies on neuropeptide B suggest an implication in sleep regulation, emotions and feeding behaviors ¹⁷. Npbwr1-/- mice do not have circadian abnormalities ¹⁸ or altered locomotor activity ¹⁸. Both, NPB and NPW are produced by brain areas projecting to the NAc, including the ventral tegmental area and the dorsal raphe nuclei ^{19,20}.
As exemplified in the appended Examples below, it has been shown that *Npbwr1* is reduced 24h after caffeine injection and increased by chronic variable stress (CVS). Using viral-mediated gene transfer, a causal link between *Npbwr1* and stress-related phenotypes has been demonstrated. Moreover, *NPBWR1* has been shown to be altered postmortem in NAc of depressed patients. RNA-sequencing after viral Npbwr1 overexpression provided a link to *Bdnf,* a key gene in antidepressant response ²¹. Microinjection with the synthetic *Npbwr1-*antagonist CYM5069 into the NAc reversed effects of chronic stress on behavior and altered *Bdnf* levels starting at 24h for up to 7 days after a single dose. On the other hand, microinjection of the natural agonist NPB mimicked chronic stress effects and had an opposing effect on *Bdnf.*
Moreover, the surprising finding of the present invention paves the way of plausibly providing antagonists of the receptor for neuropeptides B and W as potential compounds to treat Parkinson's disease (PD) which is the second most common neurodegenerative disorder. PD is characterized by the progressive loss of dopaminergic circuits.
Indeed, caffeine, a well-known adenosine receptor antagonist, has demonstrated neuroprotective properties and has been associated with a reduced risk of PD development in at least six prospective epidemiological studies as well as in animal models (Ren & Chen, Front Neurosci. 14: 602697 (2020)). This neuroprotective caffeine action is thought to be caused downstream of the adenosine 2-receptor. This receptor is highly expressed in dopaminergic neurons of the midbrain, including the NAc. Several diverging mechanisms contribute to the PD-protective caffeine effect, including mitochondrial regulation and antiinflammatory pathways.

As already outlined above and as exemplified in the present invention further below, Npbwr1 has been identified as a caffeine-regulated transcript that is altered via the adenosine 2-receptor - DARPP-32:CLOCK signaling cascade (see the experimental data below and Trautmann et al., Neuropharmacology 172, 108133 (2020)) and that this pathway is relevant to mood-related behaviors. Importantly, mood disorders including depression are often comorbid with PD, suggesting shared cell types or signaling events. Therefore, inhibition of Npbwr1 not only confers mood-elevating effects as seen after caffeine administration, but additionally exerts neuroprotective effects on those cell types that are affected by PD.

Thus, in summary, the present invention is based on the surprising identification of a previously unknown pathway which has rapid effects on stress- and depression-related behaviors wherein the receptor for neuropeptides B and W (*Npbwr1*) is the key mediator, paving the way to provide further means and methods for treating, ameliorating or preventing a mood disorder/affective disorder and/or chronic stress and/or anxiety disorders and/or Parkinson's disease by antagonizing the receptor for neuropeptides B and W (*Npbwr1*).

In view of the prior art, the technical problem underlying the present invention is the provision of further means and methods for treating, ameliorating or preventing a mood disorder/affective disorder and/or chronic stress and/or anxiety disorders and/or Parkinson's disease.

The technical problem is solved by provision of the embodiments characterized in the claims.

Therefore, in a first aspect, the present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease.

The neuropeptide B/W receptor (NPBWR1) is known in the prior art and is a member of the G-protein coupled receptor superfamily of integral membrane proteins, which bind the neuropeptides B and W. The receptor is known to be predominantly expressed in the CNS and is known to have a number of functions including regulation of the secretion of cortisol.
More specifically, NPBWR1 is, *inter alia,* known to have the following functions and activities:
NPBWR1 is known to regulate/inhibit Bdnf expression;
NPBWR1 is known to bind to neuropeptide B/W; and
NPBWR1 is known to bind to G-protein.
These functional capabilities of NPBWR1 can be assessed easily by the skilled person by utilizing routine methods and assays known in the art and as exemplified further below.

The neuropeptide B/W receptor (NPBWR1) is highly conserved across vertebrates with a homology from rodent to human of 66 to 100% and share structural and functional similarities.

The coding region of the neuropeptide B/W receptor (NPBWR1) is, *inter alia,* known in the art from human; see, e.g., UniProt accession number P48145. NPBWR1 is a classical 7 transmembrane receptor.

An exemplary neuropeptide B/W receptor (NPBWR1) may be the human neuropeptide B/W receptor (NPBWR1). The amino acid sequence of human neuropeptide B/W receptor (NPBWR1) is shown hereinbelow and in SEQ ID NO:1 and can be retrieved form the corresponding databases, such as NCBI, EMBL, Uniprot etc.:

MDNASFSEPWPANASGPDPALSCSNASTLAPLPAPLAVAVPVVYAVICAVGLAGNSAVLYVLLRAPRMK
TVTNLFILNLAIADELFTLVLPINIADFLLRQWPFGELMCKLIVAIDQYNTFSSLYFLTVMSADRYLVVLATAE
SRRVAGRTYSAARAVSLAVWGIVTLVVLPFAVFARLDDEQGRRQCVLVFPQPEAFWWRASRLYTLVLGF
AIPVSTICVLYTTLLCRLHAMRLDSHAKALERAKKRVTFLVVAILAVCLLCWTPYHLSTVVALTTDLPQTPLVI
AISYFITSLSYANSCLNPFLYAFLDASFRRNLRQLITCRAAA

The above sequence SEQ ID NO:1 was retrieved from UniProt with accession number P48145. The term "neuropeptide B/W receptor (NPBWR1)" as used herein also covers variants thereof, as well as orthologs, e.g., murine neuropeptide B/W receptor (NPBWR1). Corresponding nucleotide sequences can also be retrieved from the corresponding databases.

Thus, in particular, wild type human neuropeptide B/W receptor (NPBWR1) may be used in accordance with the present invention which is encoded by the nucleic acid sequence as represented by SEQ ID NO:2.

It is preferred herein that the patient/subject is human. Accordingly, it is preferred that the neuropeptide B/W receptor (NPBWR1) inhibitor/antagonist is an inhibitor/antagonist of human neuropeptide B/W receptor (NPBWR1), particularly when the patient/subject is human.

Accordingly, the neuropeptide B/W receptor (NPBWR1) molecules to be employed in the context of the present invention comprise, but are not limited to the molecules encoded by the nucleic acid molecules as described herein. Also envisaged are neuropeptide B/W receptor (NPBWR1) orthologs which are at least 60%, 70%, 80%, 90%, 95%, 96%, 97%, 98% or 99% identical to nucleic acid sequence as shown in SEQ ID NO: 2. These neuropeptide B/W receptor (NPBWR1) molecules as referred here are defined as molecules that are capable of acting as a neuropeptide B/W receptor (NPBWR1) as described herein above and below. These functions and activities include, *inter alia,* the capability of having neuropeptide B/W receptor (NPBWR1) activity as described herein above and below, i.e., the capability to regulate/inhibit Bdnf expression; the capability to bind to neuropeptide B/W; and/or the capability to bind to G-protein.
For testing the functional neuropeptide B/W receptor (NPBWR1) activity, assays provided herein below and as exemplified in the appended examples may be used.
As regards the capability of NPBWR1 to regulate/inhibit Bdnf expression, e.g., quantitative PCR to assay the expression of Bdnf can be used.
As regards the capability of NPBWR1 to bind to neuropeptide B/W, e.g., FRAP (fluorescence recovery after photobleaching) can be used wherein neuropeptide B or neuropeptide W is used as a substrate for the assessment of the binding to NBWBR1.
As regards the capability of NPBWR1 to bind to G-protein, e.g., FRAP (fluorescence recovery after photobleaching) can be used wherein G-protein subunits are used as a substrate for the assessment of the binding to NBWBR1.
Corresponding assays are known in the art and can easily be performed by the skilled person by utilizing routine methods.

Without being bound by theory, the Npbwr1 activity is frequently measured indirectly via the regulation/activation of the expression of the downstream protein Brain derived neurotophic factor (Bdnf). Bdnf RNA-levels can be assessed using quantitative PCR. An increase in the expression of Bdnf is an indication of the inhibition of Npbwr1. Correspondingly, a decrease in the expression of Bdnf is an indication of the activation of Npbwr1.
Alternatively, it is conceivable to design an activity assay based on the G-protein coupled nature of Npbwr1. Hereby, activity changes are translated into differential binding to a G-protein. Using Förster resonance energy transfer (FRET), changes in the interaction between Npbwr1 and its' G-protein can be visualized using a fusion of those components with fluorophores. Activation of fluorophore attached to Npbwr1 with a low wave length will emit a wave length that excites the fluorophore attached to G-protein if both are sufficiently close due to mutual binding. An increase in the fluorescence is an indication for the capability of NPBWR1 to bind to G-protein.

Furthermore, envisaged are neuropeptide B/W receptor (NPBWR1) orthologs which are at least 60%, 70%, 80%, 90%, 95%, 96%, 97%, 98% or 99% identical to the amino acid sequence as shown in SEQ ID NO:1 and being capable of acting as a neuropeptide B/W receptor (NPBWR1) as described herein above and below.

In order to determine whether a nucleic acid sequence has a certain degree of identity to a nucleic acid encoding neuropeptide B/W receptor (NPBWR1) orthologs or to an amino acid of a neuropeptide B/W receptor (NPBWR1), the skilled person can use means and methods well known in the art, e.g., alignments, either manually or by using computer programs such as those mentioned herein below.
In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (e.g., 60% or 65% identity, preferably, 70-95% identity, more preferably at least 95% identity with the nucleic acid sequences of, e.g., SEQ ID NO:2 or with the amino acid sequence of, e.g., SEQ ID NO:1 and being capable of acting as a functional neuropeptide B/W receptor (NPBWR1) as described herein above and below), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably, the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.
Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff (1989) PNAS 89:10915) uses alignments (B) of 50, expectation (E) of 10, M=5, N=4, and a comparison of both strands.

In one aspect, the present invention relates to the inhibition/antagonization of neuropeptide B/W receptor (NPBWR1) activity in a mechanism that blocks/inhibits downstream pathways of said receptor, e.g., of Brain derived neurotrophic factor (BDNF) signaling, paving the way for the use of inhibitors/antagonists of neuropeptide B/W receptor (NPBWR1) in a method of treating, ameliorating or preventing a mood disorder/affective disorder and/or chronic stress and/or anxiety disorders and/or Parkinson's disease of the present invention.

The present invention provides inhibitors/antagonists of neuropeptide B/W receptor (NPBWR1) for use in a method of treating, ameliorating or preventing a mood disorder/affective disorder and/or chronic stress and/or anxiety disorders and/or Parkinson's disease.
It is envisaged herein that these inhibitors/antagonists may be used as a medicament or as a pharmaceutical composition, i.e., the inhibitors of neuropeptide B/W receptor (NPBWR1) provided and described herein are for use in medicine (e.g., for use in the therapy/treatment/amelioration/prevention of a disease, in particular a disease associated with mood /affection and/or chronic stress and/or anxiety and/or Parkinson's disease, such as mood disorder/affective disorder and/or chronic stress and/or anxiety disorders and/or Parkinson's disease). The terms "medicament" and "pharmaceutical composition" are used interchangeably herein. Accordingly, definitions and explanations provided herein in relation to "pharmaceutical compositions", apply, *mutatis mutandis,* to the term "medicament".

The terms "antagonist" or "inhibitor" are used interchangeably herein. These terms are known in the art and relates to a compound/substance capable of fully or partially preventing or reducing the physiologic activity of (a) specific protein(s). In the context of the present invention said antagonist/inhibitor, therefore, may prevent or reduce or inhibit or inactivate the physiological activity of a protein such as neuropeptide B/W receptor (NPBWR1) upon binding of said compound/substance to said protein. Binding of an "antagonist/inhibitor" to a given protein, e.g., neuropeptide B/W receptor (NPBWR1), may compete with or prevent the binding of an endogenous activating molecules binding to said protein. As used herein, accordingly, the term "antagonist" also encompasses competitive antagonists, (reversible) non-competitive antagonists or irreversible antagonist, as described, *inter alia,* in Mutschler, "Arzneimittelwirkungen" (1986), Wissenschaftliche Verlagsgesellschaft mbH, Stuttgart, Germany. In addition thereto, however, an "antagonist" or "inhibitor" of neuropeptide B/W receptor (NPBWR1) in the context of the present invention may also be capable of preventing the function of a given protein, such as neuropeptide B/W receptor (NPBWR1), by preventing/reducing the expression of the nucleic acid molecule encoding for said protein. Thus, an antagonist/inhibitor of neuropeptide B/W receptor (NPBWR1) may lead to a decreased expression level of neuropeptide B/W receptor (NPBWR1) (e.g. decreased level of neuropeptide B/W receptor (NPBWR1) mRNA, neuropeptide B/W receptor (NPBWR1) protein) which is reflected in a decreased activity of neuropeptide B/W receptor (NPBWR1). This decreased activity can be measured/detected by methods known in the art and as described herein. An inhibitor of neuropeptide B/W receptor (NPBWR1) in the context of the present invention, accordingly, may also encompass transcriptional repressors of neuropeptide B/W receptor (NPBWR1) expression that are capable of reducing neuropeptide B/W receptor (NPBWR1) function. As described herein below in detail, the decreased expression and/or activity of neuropeptide B/W receptor (NPBWR1) by an antagonist/inhibitor of neuropeptide B/W receptor (NPBWR1) leads to a decreased activity (and/or expression) of neuropeptide B/W receptor (NPBWR1), thereby decreasing functional capability of neuropeptide B/W receptor (NPBWR1).

In line with the rationale of the present invention, a decrease in the activity of a functional neuropeptide B/W receptor (NPBWR1) is expected to have various medical implications.

As explained in the following, a decrease in the activity of a functional neuropeptide B/W receptor (NPBWR1) is expected to have medical implications, i.e., to have an impact on a disease/disorder associated with mood/affection and/or chronic stress and/or anxiety and/or Parkinson's disease, such as mood disorder/affective disorder and/or chronic stress and/or anxiety disorders and/or Parkinson's disease).

The term "inhibitor" and/or "antagonist" implies no specific mechanism of biological action whatsoever and is deemed to expressly include and encompass all possible pharmacological, physiological, and biochemical interactions with neuropeptide B/W receptor (NPBWR1) (signaling) whether direct or indirect. For the purpose of the present disclosure, it will be explicitly understood that the term "inhibitor" and/or "antagonist" encompasses all the previously identified terms, titles, and functional states and characteristics whereby neuropeptide B/W receptor (NPBWR1) itself, a biological activity of neuropeptide B/W receptor (NPBWR1) (including but not limited to its ability to regulate/inhibit Bdnf expression; the capability to bind to neuropeptide B/W; and/or the capability to bind to G-protein), or the consequences of the biological activity, are substantially nullified, decreased, or neutralized in any meaningful degree, e.g., by at least 5%, 10%, 20%, 50%, 70%, 85%, 90%, 100%, 150%, 200%, 300%, 500%, or by 2-fold, 3-fold, 4-fold, 5-fold, 10-fold, 20-fold, 50-fold, 100-fold or 1000-fold. An inhibitor/antagonist may decrease an abnormal level of a biological activity of neuropeptide B/W receptor (NPBWR1) that may cause an adverse effect and/or a disease in a subject to a level that corresponds to a level in a healthy subject thereby preventing, preventing progression and/or curing the adverse effect and/or disease.
Accordingly, the present invention relates in one aspect to an inhibitor/antagonist of neuropeptide B/W receptor (NPBWR1) for use in in a method of treating, ameliorating or preventing a mood disorder/affective disorder and/or chronic stress and/or anxiety disorders and/or Parkinson's disease, wherein the inhibitor decreases the biological activity of neuropeptide B/W receptor (NPBWR1) at least 2-fold, 3-fold, 4-fold, 5-fold, 10-fold, 20-fold, 50-fold, 100-fold or 1000-fold.
Accordingly, the present invention relates in one aspect to an inhibitor/antagonist of neuropeptide B/W receptor (NPBWR1) for use in in a method of treating, ameliorating or preventing a mood disorder/affective disorder and/or chronic stress and/or anxiety disorders and/or Parkinson's disease, wherein the inhibitor/antagonist decreases an abnormal level of a biological activity of neuropeptide B/W receptor (NPBWR1) that causes/and or promotes an adverse effect and/or a disease at least 2-fold, 3-fold, 4-fold, 5-fold, 10-fold, 20-fold, 50-fold, 100-fold or 1000-fold.
Accordingly, the present invention relates in one aspect to an inhibitor/antagonist of neuropeptide B/W receptor (NPBWR1) for use in in a method of treating, ameliorating or preventing a mood disorder/affective disorder and/or chronic stress and/or anxiety disorders and/or Parkinson's disease, wherein the inhibitor/antagonist decreases an abnormal level of a biological activity of neuropeptide B/W receptor (NPBWR1) that causes/and or promotes an adverse effect and/or a disease at least 3-fold.

Accordingly, the present invention relates in one aspect to an inhibitor/antagonist of neuropeptide B/W receptor (NPBWR1) for use in in a method of treating, ameliorating or preventing a mood disorder/affective disorder and/or chronic stress and/or anxiety disorders and/or Parkinson's disease, wherein the inhibitor/antagonist decreases an abnormal level of a biological activity of neuropeptide B/W receptor (NPBWR1) that causes/and or promotes mood disorder/affective disorder and/or chronic stress and/or anxiety disorders and/or Parkinson's disease.
Accordingly, the present invention relates in one aspect to an inhibitor/antagonist of neuropeptide B/W receptor (NPBWR1) for use in in a method of treating, ameliorating or preventing a mood disorder/affective disorder and/or chronic stress and/or anxiety disorders and/or Parkinson's disease, wherein the inhibitor decreases an abnormal level of a biological activity of neuropeptide B/W receptor (NPBWR1) that causes/and or promotes mood disorder/affective disorder and/or chronic stress and/or anxiety disorders and/or Parkinson's disease in a subject to a level comparable to a healthy subject.

According to the present invention the term "inhibitor/antagonist of neuropeptide B/W receptor (NPBWR1)" also means a compound or substance capable of fully or partially preventing or reducing the physiologic activity of the neuropeptide B/W receptor (NPBWR1). In the context of the present invention said inhibitor may therefore prevent, reduce, inhibit or inactivate the physiological activity of neuropeptide B/W receptor (NPBWR1), e.g., upon binding of said compound/substance (i.e., of the inhibitor/antagonist) to neuropeptide B/W receptor (NPBWR1).
As used herein, the term "inhibitor" also encompasses inhibitors that result in a reversible inhibition such as competitive inhibition, uncompetitive inhibition, non-competitive inhibition, mixed inhibition or in an irreversible inhibition of neuropeptide B/W receptor (NPBWR1), e.g., inhibition by covalent interaction.

A "inhibitor/antagonist of neuropeptide B/W receptor (NPBWR1)" may also be capable of preventing the function of neuropeptide B/W receptor (NPBWR1) by preventing/reducing the expression of the nucleic acid molecule encoding for neuropeptide B/W receptor (NPBWR1). Thus, an inhibitor/antagonist of neuropeptide B/W receptor (NPBWR1) may lead to a decreased expression level of neuropeptide B/W receptor (NPBWR1) gene products such as a decreased level of neuropeptide B/W receptor (NPBWR1) mRNA and/or neuropeptide B/W receptor (NPBWR1) protein.
An inhibitor/antagonist of neuropeptide B/W receptor (NPBWR1) may reduce an abnormal expression level of neuropeptide B/W receptor (NPBWR1) that may cause and/or promote an adverse effect and/or disease/disease associated with mood disorder/affective disorder and/or chronic stress and/or anxiety disorder and/or Parkinson's disease as outlined hereinabove and below to a level that corresponds to a level in a healthy subject thereby preventing, preventing progression or curing the adverse effect and/or disease/disease in a subject. This may be reflected in a decreased neuropeptide B/W receptor (NPBWR1) expression and/or a decreased abnormal neuropeptide B/W receptor (NPBWR1) expression thereby restoring a healthy level of neuropeptide B/W receptor (NPBWR1) expression. neuropeptide B/W receptor (NPBWR1) expression level may to some extent correlate with neuropeptide B/W receptor (NPBWR1) activity until saturation of the translation machinery and or a substance that binds neuropeptide B/W receptor (NPBWR1) is achieved. The expression level of neuropeptide B/W receptor (NPBWR1) may be measured/detected by methods known in the art.

Accordingly, the present invention relates in one aspect to an inhibitor/antagonist of neuropeptide B/W receptor (NPBWR1) for use in the treatment, amelioration or prevention of a disorder associated with mood disorder/affective disorder and/or chronic stress and/or anxiety disorder and/or Parkinson's disease in accordance with the present invention, wherein the inhibitor decreases the expression level of neuropeptide B/W receptor (NPBWR1) gene products.
Accordingly, the present invention relates in one aspect to an inhibitor/antagonist of neuropeptide B/W receptor (NPBWR1) for use in the treatment, amelioration or prevention of a disorder associated with mood disorder/affective disorder and/or chronic stress and/or anxiety disorder and/or Parkinson's disease in accordance with the present invention, wherein the inhibitor/antagonist decreases an abnormal expression level of neuropeptide B/W receptor (NPBWR1) gene products that causes and/or promotes an adverse effect and/or a disease/disorder.
Accordingly, the present invention relates in one aspect to an inhibitor/antagonist of neuropeptide B/W receptor (NPBWR1) for use in the treatment, amelioration or prevention of a disorder associated with mood disorder/affective disorder and/or chronic stress and/or anxiety disorder and/or Parkinson's disease in accordance with the present invention, wherein the inhibitor/antagonist decreases an abnormal expression level of neuropeptide B/W receptor (NPBWR1) gene products that causes and/or promotes the above mood disorder/affective disorder and/or chronic stress and/or anxiety disorder and/or Parkinson's disease.
Accordingly, the present invention relates in one aspect to an inhibitor/antagonist of neuropeptide B/W receptor (NPBWR1) for use in the treatment, amelioration or prevention of a disorder associated with mood disorder/affective disorder and/or chronic stress and/or anxiety disorder and/or Parkinson's disease in accordance with the present invention, wherein the inhibitor/antagonist decreases an abnormal expression level of neuropeptide B/W receptor (NPBWR1) gene products that causes and/or promotes mood disorder/affective disorder and/or chronic stress and/or anxiety disorder and/or Parkinson's disease in a subject to a level of expression comparable to a healthy subject.

An inhibitor/antagonist of neuropeptide B/W receptor (NPBWR1) may perform its inhibitory function by directly interacting the neuropeptide B/W receptor (NPBWR1) protein, i.e., with any part of the neuropeptide B/W receptor (NPBWR1) protein such as the ectodomain, transmembrane domain and/or cytoplasmic domain of neuropeptide B/W receptor (NPBWR1). An inhibitor/antagonist of neuropeptide B/W receptor (NPBWR1) may also perform any inhibitory effect on neuropeptide B/W receptor (NPBWR1) function by preventing, reducing, inhibiting or inactivating any upstream or downstream pathway components that essentially contribute to neuropeptide B/W receptor (NPBWR1) function. An inhibitor/antagonist of neuropeptide B/W receptor (NPBWR1) may perform any indirect inhibitory effect on any neuropeptide B/W receptor (NPBWR1) activating molecules such as nucleic acids, ribonucleic acid (RNA), double-stranded ribonucleic acid (dsRNA), chromatin reader proteins and/or ligands.

Efficacy of an inhibitor/antagonist of neuropeptide B/W receptor (NPBWR1) may be described using the half-maximal inhibitory concentration (IC50) value. In the sense of the present invention, an inhibitor/antagonist of neuropeptide B/W receptor (NPBWR1) preferably embodies a low IC50 value. The IC50 value of an inhibitor/antagonist of neuropeptide B/W receptor (NPBWR1) may be below 100µM, below 90 µM, below 80 µM, below 70 µM, below 60 µM, below 50 µM, below 40 µM, below 30 µM, below 20 µM or below 10 µM, wherein lower values are preferred over higher values. Preferably, the IC50 value of an inhibitor/antagonist of neuropeptide B/W receptor (NPBWR1) may be below 10µm, below 9 µM, below 8 µM, below 7 µM, below 6 µM, below 5 µM or below 4 µM. Preferably, the IC50 value of a an inhibitor/antagonist of neuropeptide B/W receptor (NPBWR1) may be below 4µM.

Accordingly, the invention relates to an inhibitor/antagonist of neuropeptide B/W receptor (NPBWR1) for use in the treatment, amelioration or prevention of a mood disorder/affective disorder and/or chronic stress and/or anxiety disorder and/or Parkinson's disease in accordance with the present invention, wherein the efficacy of the half-maximal inhibitory concentration (IC50) is below 100µM, below 90 µM, below 80 µM, below 70 µM, below 60 µM, below 50 µM, below 40 µM, below 30 µM, below 20 µM or below 10 µM, below 9 µM, below 8 µM, below 7 µM, below 6 µM, below 5 µM or below 4 µM, preferably below 4µM.

The skilled person is aware how to determine the IC50 value of an inhibitor/antagonist of neuropeptide B/W receptor (NPBWR1). It is envisioned herein, that an inhibitors/antagonists of neuropeptide B/W receptor (NPBWR1) may embody additional IC50 values and/or that other inhibitors/antagonists of neuropeptide B/W receptor (NPBWR1) with other IC50 values are identified.

Mood disorder/affective disorder and/or chronic stress and/or anxiety disorder and/or Parkinson's disease are disorders or syndromes that are known in the art and are medical indications that are classified in the ICD-system, i.e., the well-known and established medical classification list by the World Health Organization (WHO). ICD is the International Statistical Classification of Diseases and Related Health Problems (ICD). In the following, when referring to more specifically defined disorders or diseases, reference is made to the 10^{th} revision of said the International Statistical Classification of Diseases and Related Health Problems (ICD), i.e., "ICD 10".

The present invention is not limited to any particular mood disorder/affective disorder and/or chronic stress and/or anxiety disorder. Any mood disorder/affective disorder and/or chronic stress and/or anxiety disorder and/or Parkinson's disease can be treated, ameliorated and/or prevented by the antagonist/inhibitor of neuropeptide B/W receptor (NPBWR1) in accordance with the present invention.
Without being bound to theory, in particular preferred embodiments, the present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease wherein said mood disorder/affective disorder and/or chronic stress and/or anxiety disorder and/or Parkinson's disease is/are the following.

In a preferred embodiment, the present invention relates to a pharmaceutical composition comprising an antagonist/inhibitor of neuropeptide B/W receptor (NPBWR1) for use in in a method of treating, ameliorating or preventing a mood disorder/affective disorder, wherein said mood disorder/affective disorder is selected from the group consisting of:
(i) Manic episode (ICD-10 F30);
(ii) Bipolar affective disorder (ICD-10 F31);
(iii) Depressive episode (ICD-10 F32);
(iv) Recurrent depressive disorder (ICD-10 F33);
(v) Persistent mood affective disorders (ICD-10 F34).
(vi) Other mood affective disorders (ICD-10 F38); and
(vii) Unspecified mood affective disorder (ICD-10 F39).

Mood/affective disorders in terms of the present invention generally refer to disorders in which the fundamental disturbance is a change in affect or mood to depression (with or without associated anxiety) or to elation. The mood change is usually accompanied by a change in the overall level of activity; most of the other symptoms are either secondary to, or easily understood in the context of, the change in mood and activity. Most of these disorders tend to be recurrent and the onset of individual episodes can often be related to stressful events or situations.

More specific mood/affective disorders in terms of the present invention as well as corresponding subdivisions thereof are described in more detail in the following:

### Manic episode (ICD-10 F30):

All the subdivisions of this category should be used only for a single episode. Hypomanic or manic episodes in individuals who have had one or more previous affective episodes (depressive, hypomanic, manic, or mixed) should be coded as bipolar affective disorder (F31.-).

Subdivisions of Manic episode (ICD-10 F30) are explained in the following:
F30.0: Hypomania:
   A disorder characterized by a persistent mild elevation of mood, increased energy and activity, and usually marked feelings of well-being and both physical and mental efficiency. Increased sociability, talkativeness, over-familiarity, increased sexual energy, and a decreased need for sleep are often present but not to the extent that they lead to severe disruption of work or result in social rejection. Irritability, conceit, and boorish behaviour may take the place of the more usual euphoric sociability. The disturbances of mood and behaviour are not accompanied by hallucinations or delusions.
F30.1: Mania without psychotic symptoms:
   Mood is elevated out of keeping with the patient's circumstances and may vary from carefree joviality to almost uncontrollable excitement. Elation is accompanied by increased energy, resulting in overactivity, pressure of speech, and a decreased need for sleep. Attention cannot be sustained, and there is often marked distractibility. Self-esteem is often inflated with grandiose ideas and overconfidence. Loss of normal social inhibitions may result in behaviour that is reckless, foolhardy, or inappropriate to the circumstances, and out of character.
F30.2: Mania with psychotic symptoms:
   In addition to the clinical picture described in F30.1, delusions (usually grandiose) or hallucinations (usually of voices speaking directly to the patient) are present, or the excitement, excessive motor activity, and flight of ideas are so extreme that the subject is incomprehensible or inaccessible to ordinary communication.
   Mania with: mood-congruent psychotic symptoms or mood-incongruent psychotic symptoms Manic stupor
   F30.8: Other manic episodes:
   F30.9: Manic episode, unspecified
   Mania NOS

### Bipolar affective disorder (ICD-10 F31):

A disorder characterized by two or more episodes in which the patient's mood and activity levels are significantly disturbed, this disturbance consisting on some occasions of an elevation of mood and increased energy and activity (hypomania or mania) and on others of a lowering of mood and decreased energy and activity (depression). Repeated episodes of hypomania or mania only are classified as bipolar.

### Incl.:

### manic depression

### manic-depressive:

- illness
- psychosis
- reaction

### Excl. :

bipolar disorder, single manic episode (F30.-)
cyclothymia (F34.0)
Subdivisions of Bipolar affective disorder (ICD-10 F31) are explained in the following:
F31.0: Bipolar affective disorder, current episode hypomanic:
   The patient is currently hypomanic, and has had at least one other affective episode (hypomanic, manic, depressive, or mixed) in the past.
F31.1: Bipolar affective disorder, current episode manic without psychotic symptoms:
   The patient is currently manic, without psychotic symptoms (as in F30.1), and has had at least one other affective episode (hypomanic, manic, depressive, or mixed) in the past.
F31.2: Bipolar affective disorder, current episode manic with psychotic symptoms:
   The patient is currently manic, with psychotic symptoms (as in F30.2), and has had at least one other affective episode (hypomanic, manic, depressive, or mixed) in the past.
F31.3: Bipolar affective disorder, current episode mild or moderate depression:
   The patient is currently depressed, as in a depressive episode of either mild or moderate severity (F32.0 or F32.1), and has had at least one authenticated hypomanic, manic, or mixed affective episode in the past.
F31.4: Bipolar affective disorder, current episode severe depression without psychotic symptoms:
   The patient is currently depressed, as in severe depressive episode without psychotic symptoms (F32.2), and has had at least one authenticated hypomanic, manic, or mixed affective episode in the past.
F31.5: Bipolar affective disorder, current episode severe depression with psychotic symptoms: The patient is currently depressed, as in severe depressive episode with psychotic symptoms (F32.3), and has had at least one authenticated hypomanic, manic, or mixed affective episode in the past.
F31.6: Bipolar affective disorder, current episode mixed:
   The patient has had at least one authenticated hypomanic, manic, depressive, or mixed affective episode in the past, and currently exhibits either a mixture or a rapid alteration of manic and depressive symptoms.

### Excl.:

### single mixed affective episode (F38.0)

### F31.7: Bipolar affective disorder, currently in remission:

The patient has had at least one authenticated hypomanic, manic, or mixed affective episode in the past, and at least one other affective episode (hypomanic, manic, depressive, or mixed) in addition, but is not currently suffering from any significant mood disturbance, and has not done so for several months. Periods of remission during prophylactic treatment should be coded here.
F31.8: Other bipolar affective disorders
   Bipolar II disorder
   Recurrent manic episodes NOS
F31.9: Bipolar affective disorder, unspecified
   Manic depression NOS

### Depressive episode (ICD-10 F32):

In typical mild, moderate, or severe depressive episodes, the patient suffers from lowering of mood, reduction of energy, and decrease in activity. Capacity for enjoyment, interest, and concentration is reduced, and marked tiredness after even minimum effort is common. Sleep is usually disturbed and appetite diminished. Self-esteem and self-confidence are almost always reduced and, even in the mild form, some ideas of guilt or worthlessness are often present. The lowered mood varies little from day to day, is unresponsive to circumstances and may be accompanied by so-called "somatic" symptoms, such as loss of interest and pleasurable feelings, waking in the morning several hours before the usual time, depression worst in the morning, marked psychomotor retardation, agitation, loss of appetite, weight loss, and loss of libido. Depending upon the number and severity of the symptoms, a depressive episode may be specified as mild, moderate or severe.

### Incl.:

### single episodes of:

- depressive reaction
- psychogenic depression
- reactive depression

### Excl. :

adjustment disorder (F43.2)
recurrent depressive disorder (F33.-)
when associated with conduct disorders in F91.- (F92.0)
Subdivisions of Depressive episode (ICD-10 F32) are explained in the following:
F32.0: Mild depressive episode:
   Two or three of the above symptoms are usually present. The patient is usually distressed by these but will probably be able to continue with most activities.
F32.1: Moderate depressive episode:
   Four or more of the above symptoms are usually present and the patient is likely to have great difficulty in continuing with ordinary activities.
F32.2: Severe depressive episode without psychotic symptoms:
   An episode of depression in which several of the above symptoms are marked and distressing, typically loss of self-esteem and ideas of worthlessness or guilt. Suicidal thoughts and acts are common and a number of "somatic" symptoms are usually present.
   - Agitated depression
   - Major depression
   - Vital depression
   - Single episode without psychotic symptoms
F32.3: Severe depressive episode with psychotic symptoms:
   An episode of depression as described in F32.2, but with the presence of hallucinations, delusions, psychomotor retardation, or stupor so severe that ordinary social activities are impossible; there may be danger to life from suicide, dehydration, or starvation. The hallucinations and delusions may or may not be mood-congruent.
   Single episodes of:
      - major depression with psychotic symptoms
      - psychogenic depressive psychosis
      - psychotic depression
      - reactive depressive psychosis
F32.8: Other depressive episodes:

### Atypical depression

### Single episodes of "masked" depression NOS

F32.9: Depressive episode, unspecified:
Depression NOS
Depressive disorder NOS

### Recurrent depressive disorder (ICD-10 F33):

A disorder characterized by repeated episodes of depression as described for depressive episode (F32.-), without any history of independent episodes of mood elevation and increased energy (mania). There may, however, be brief episodes of mild mood elevation and overactivity (hypomania) immediately after a depressive episode, sometimes precipitated by antidepressant treatment. The more severe forms of recurrent depressive disorder (F33.2 and F33.3) have much in common with earlier concepts such as manic-depressive depression, melancholia, vital depression and endogenous depression. The first episode may occur at any age from childhood to old age, the onset may be either acute or insidious, and the duration varies from a few weeks to many months. The risk that a patient with recurrent depressive disorder will have an episode of mania never disappears completely, however many depressive episodes have been experienced. If such an episode does occur, the diagnosis should be changed to bipolar affective disorder (F31.-).

### Incl.:

recurrent episodes of:
- depressive reaction
- psychogenic depression
- reactive depression
seasonal depressive disorder

### Excl. :

### recurrent brief depressive episodes (F38.1)

Subdivisions of Recurrent depressive disorder (ICD-10 F33) are explained in the following:
F33.0: Recurrent depressive disorder, current episode mild:
   A disorder characterized by repeated episodes of depression, the current episode being mild, as in F32.0, and without any history of mania.
F33.1: Recurrent depressive disorder, current episode moderate:
   A disorder characterized by repeated episodes of depression, the current episode being of moderate severity, as in F32.1, and without any history of mania.
F33.2: Recurrent depressive disorder, current episode severe without psychotic symptoms:
   A disorder characterized by repeated episodes of depression, the current episode being severe without psychotic symptoms, as in F32.2, and without any history of mania.
   Endogenous depression without psychotic symptoms
   Major depression, recurrent without psychotic symptoms
   Manic-depressive psychosis, depressed type without psychotic symptoms
   Vital depression, recurrent without psychotic symptoms
F33.3: Recurrent depressive disorder, current episode severe with psychotic symptoms:
   A disorder characterized by repeated episodes of depression, the current episode being severe with psychotic symptoms, as in F32.3, and with no previous episodes of mania. Endogenous depression with psychotic symptoms
   Manic-depressive psychosis, depressed type with psychotic symptoms
   Recurrent severe episodes of:
      - major depression with psychotic symptoms
      - psychogenic depressive psychosis
      - psychotic depression
      - reactive depressive psychosis
F33.4: Recurrent depressive disorder, currently in remission:
   The patient has had two or more depressive episodes as described in F33.0-F33.3, in the past, but has been free from depressive symptoms for several months.
F33.8: Other recurrent depressive disorders:
F33.9: Recurrent depressive disorder, unspecified:
   Monopolar depression NOS

### Persistent mood affective disorders (ICD-10 F34):

Persistent and usually fluctuating disorders of mood in which the majority of the individual episodes are not sufficiently severe to warrant being described as hypomanic or mild depressive episodes. Because they last for many years, and sometimes for the greater part of the patient's adult life, they involve considerable distress and disability. In some instances, recurrent or single manic or depressive episodes may become superimposed on a persistent affective disorder.

Subdivisions of Persistent mood affective disorders (ICD-10 F34) are explained in the following:
F34.0: Cyclothymia:
   A persistent instability of mood involving numerous periods of depression and mild elation, none of which is sufficiently severe or prolonged to justify a diagnosis of bipolar affective disorder (F31.-) or recurrent depressive disorder (F33.-). This disorder is frequently found in the relatives of patients with bipolar affective disorder. Some patients with cyclothymia eventually develop bipolar affective disorder.
   Affective personality disorder
   Cycloid personality
   Cyclothymic personality
F34.1: Dysthymia:
   A chronic depression of mood, lasting at least several years, which is not sufficiently severe, or in which individual episodes are not sufficiently prolonged, to justify a diagnosis of severe, moderate, or mild recurrent depressive disorder (F33.-).
   Depressive:
      - neurosis
      - personality disorder
   Neurotic depression
   Persistent anxiety depression
   *Excl.:*
      anxiety depression (mild or not persistent) (F41.2)
F34.8: Other persistent mood [affective] disorders
F34.9: Persistent mood [affective] disorder, unspecified

### Other mood affective disorders (ICD-10 F38):

Any other mood disorders that do not justify classification to F30-F34, because they are not of sufficient severity or duration.

Subdivisions of Other mood affective disorders (ICD-10 F38) are explained in the following:
F38.0: Other single mood [affective] disorders:
   Mixed affective episode
F38.1: Other recurrent mood [affective] disorders:
   Recurrent brief depressive episodes
F38.8: Other specified mood [affective] disorders.

Unspecified mood affective disorder (ICD-10 F39):
*Incl.:*
Affective psychosis NOS

In another preferred embodiment, the present invention relates to a pharmaceutical composition comprising an antagonist/inhibitor of neuropeptide B/W receptor (NPBWR1) for use in in a method of treating, ameliorating or preventing chronic stress, wherein said chronic stress is selected from the group consisting of:
(i) Reaction to severe stress, and adjustment disorders (ICD-10 F43);
(ii) Acute stress reaction (ICD-10 F43.0);
(iii) Post-traumatic stress disorder (ICD-10 F43.1);
(iv) Adjustment disorders (ICD-10 F43.2);
(v) Other reactions to severe stress (ICD-10 F43.8); and
(vi) Reaction to severe stress, unspecified (ICD-10 F43.9).

Chronic stress in terms of the present invention is commonly understood as the physiological or psychological response induced by a long-term internal or external stressor. The stressor, either physically present or recollected, will produce the same effect and trigger a chronic stress response. There is a wide range of chronic stressors, but most entail relatively prolonged problems, conflicts and threats that people encounter on a daily basis.

More specific chronic stresses in terms of the present invention as well as corresponding subdivisions thereof are described in more detail in the following:

### Reaction to severe stress, and adjustment disorders (ICD-10 F43):

This category includes disorders identifiable on the basis of not only symptoms and course but also the existence of one or other of two causative influences: an exceptionally stressful life event producing an acute stress reaction, or a significant life change leading to continued unpleasant circumstances that result in an adjustment disorder. Although less severe psychosocial stress ("life events") may precipitate the onset or contribute to the presentation of a very wide range of disorders classified elsewhere in this chapter, its etiological importance is not always clear and in each case will be found to depend on individual, often idiosyncratic, vulnerability, i.e. the life events are neither necessary nor sufficient to explain the occurrence and form of the disorder. In contrast, the disorders brought together here are thought to arise always as a direct consequence of acute severe stress or continued trauma. The stressful events or the continuing unpleasant circumstances are the primary and overriding causal factor and the disorder would not have occurred without their impact. The disorders in this section can thus be regarded as maladaptive responses to severe or continued stress, in that they interfere with successful coping mechanisms and therefore lead to problems of social functioning.

Subdivisions of Severe Stress/Reaction to severe stress, and adjustment disorders (ICD-10 F43):are explained in the following:

### Acute stress reaction (ICD-10 F43.0):

A transient disorder that develops in an individual without any other apparent mental disorder in response to exceptional physical and mental stress and that usually subsides within hours or days. Individual vulnerability and coping capacity play a role in the occurrence and severity of acute stress reactions. The symptoms show a typically mixed and changing picture and include an initial state of "daze" with some constriction of the field of consciousness and narrowing of attention, inability to comprehend stimuli, and disorientation. This state may be followed either by further withdrawal from the surrounding situation (to the extent of a dissociative stupor - F44.2), or by agitation and over-activity (flight reaction or fugue). Autonomic signs of panic anxiety (tachycardia, sweating, flushing) are commonly present. The symptoms usually appear within minutes of the impact of the stressful stimulus or event, and disappear within two to three days (often within hours). Partial or complete amnesia (F44.0) for the episode may be present. If the symptoms persist, a change in diagnosis should be considered.

### Acute:

- crisis reaction
- reaction to stress

Combat fatigue
Crisis state
Psychic shock

### Post-traumatic stress disorder (ICD-10 F43.1):

Arises as a delayed or protracted response to a stressful event or situation (of either brief or long duration) of an exceptionally threatening or catastrophic nature, which is likely to cause pervasive distress in almost anyone. Predisposing factors, such as personality traits (e.g. compulsive, asthenic) or previous history of neurotic illness, may lower the threshold for the development of the syndrome or aggravate its course, but they are neither necessary nor sufficient to explain its occurrence. Typical features include episodes of repeated reliving of the trauma in intrusive memories ("flashbacks"), dreams or nightmares, occurring against the persisting background of a sense of "numbness" and emotional blunting, detachment from other people, unresponsiveness to surroundings, anhedonia, and avoidance of activities and situations reminiscent of the trauma. There is usually a state of autonomic hyperarousal with hypervigilance, an enhanced startle reaction, and insomnia. Anxiety and depression are commonly associated with the above symptoms and signs, and suicidal ideation is not infrequent. The onset follows the trauma with a latency period that may range from a few weeks to months. The course is fluctuating but recovery can be expected in the majority of cases. In a small proportion of cases the condition may follow a chronic course over many years, with eventual transition to an enduring personality change (F62.0).

### Traumatic neurosis

### Adjustment disorders (ICD-10 F43.2):

States of subjective distress and emotional disturbance, usually interfering with social functioning and performance, arising in the period of adaptation to a significant life change or a stressful life event. The stressor may have affected the integrity of an individual's social network (bereavement, separation experiences) or the wider system of social supports and values (migration, refugee status), or represented a major developmental transition or crisis (going to school, becoming a parent, failure to attain a cherished personal goal, retirement). Individual predisposition or vulnerability plays an important role in the risk of occurrence and the shaping of the manifestations of adjustment disorders, but it is nevertheless assumed that the condition would not have arisen without the stressor. The manifestations vary and include depressed mood, anxiety or worry (or mixture of these), a feeling of inability to cope, plan ahead, or continue in the present situation, as well as some degree of disability in 9the performance of daily routine. Conduct disorders may be an associated feature, particularly in adolescents. The predominant feature may be a brief or prolonged depressive reaction, or a disturbance of other emotions and conduct.
Culture shock
Grief reaction
Hospitalism in children
*Excl.:*
   separation anxiety disorder of childhood (F93.0)
   Other reactions to severe stress (ICD-10 F43.8):
      Reaction to severe stress, unspecified (ICD-10 F43.9).

In another preferred embodiment, the present invention relates to a pharmaceutical composition comprising an antagonist/inhibitor of neuropeptide B/W receptor (NPBWR1) for use in in a method of treating, ameliorating or preventing an anxiety disorder, wherein said anxiety disorder is selected from the group consisting of:
(i) Phobic anxiety disorders (ICD-10 F40); and
(ii) Other anxiety disorders (ICD-10 F41.1).

An anxiety disorder in terms of the present invention is a group of disorders in which anxiety is evoked only, or predominantly, in certain well-defined situations that are not currently dangerous. As a result these situations are characteristically avoided or endured with dread. The patient's concern may be focused on individual symptoms like palpitations or feeling faint and is often associated with secondary fears of dying, losing control, or going mad. Contemplating entry to the phobic situation usually generates anticipatory anxiety. Phobic anxiety and depression often coexist. Whether two diagnoses, phobic anxiety and depressive episode, are needed, or only one, is determined by the time course of the two conditions and by therapeutic considerations at the time of consultation.

More specific anxiety disorders in terms of the present invention as well as corresponding subdivisions thereof are described in more detail in the following:

### Agoraphobia (ICD-10 F40.0):

A fairly well-defined cluster of phobias embracing fears of leaving home, entering shops, crowds and public places, or travelling alone in trains, buses or planes. Panic disorder is a frequent feature of both present and past episodes. Depressive and obsessional symptoms and social phobias are also commonly present as subsidiary features. Avoidance of the phobic situation is often prominent, and some agoraphobics experience little anxiety because they are able to avoid their phobic situations.
- Agoraphobia without history of panic disorder
- Panic disorder with agoraphobia.

### Social phobias (ICD-10 F40.1)

Fear of scrutiny by other people leading to avoidance of social situations. More pervasive social phobias are usually associated with low self-esteem and fear of criticism. They may present as a complaint of blushing, hand tremor, nausea, or urgency of micturition, the patient sometimes being convinced that one of these secondary manifestations of their anxiety is the primary problem. Symptoms may progress to panic attacks.
- Anthropophobia
- Social neurosis

### Specific (isolated) phobias(ICD-10: F40.2):

Phobias restricted to highly specific situations such as proximity to particular animals, heights, thunder, darkness, flying, closed spaces, urinating or defecating in public toilets, eating certain foods, dentistry, or the sight of blood or injury. Though the triggering situation is discrete, contact with it can evoke panic as in agoraphobia or social phobia.
- Acrophobia
- Animal phobias
- Claustrophobia
- Simple phobia

### Excl.:

dysmorphophobia (nondelusional) (F45.2)
nosophobia (F45.2)
F40.8Other phobic anxiety disorders
F40.9Phobic anxiety disorder, unspecified
Phobia NOS
Phobic state NOS

### Other anxiety disorder (ICD-10 F41):

Disorders in which manifestation of anxiety is the major symptom and is not restricted to any particular environmental situation. Depressive and obsessional symptoms, and even some elements of phobic anxiety, may also be present, provided that they are clearly secondary or less severe.

### Panic disorder [episodic paroxysmal anxiety] (ICD-10 F41.0):

The essential feature is recurrent attacks of severe anxiety (panic), which are not restricted to any particular situation or set of circumstances and are therefore unpredictable. As with other anxiety disorders, the dominant symptoms include sudden onset of palpitations, chest pain, choking sensations, dizziness, and feelings of unreality (depersonalization or derealization).

There is often also a secondary fear of dying, losing control, or going mad. Panic disorder should not be given as the main diagnosis if the patient has a depressive disorder at the time the attacks start; in these circumstances the panic attacks are probably secondary to depression.

### Panic:

- attack
- state

### Excl.:

### panic disorder with agoraphobia (F40.0)

### Generalized anxiety disorder (ICD-10 F41.1):

Anxiety that is generalized and persistent but not restricted to, or even strongly predominating in, any particular environmental circumstances (i.e. it is "free-floating"). The dominant symptoms are variable but include complaints of persistent nervousness, trembling, muscular tensions, sweating, lightheadedness, palpitations, dizziness, and epigastric discomfort. Fears that the patient or a relative will shortly become ill or have an accident are often expressed.

### Anxiety:

- neurosis
- reaction
- state

### Excl.:

### neurasthenia (F48.0)

### Mixed anxiety and depressive disorder (ICD-10 F41.2):

This category should be used when symptoms of anxiety and depression are both present, but neither is clearly predominant, and neither type of symptom is present to the extent that justifies a diagnosis if considered separately. When both anxiety and depressive symptoms are present and severe enough to justify individual diagnoses, both diagnoses should be recorded and this category should not be used.

### Anxiety depression (mild or not persistent)

Other mixed anxiety disorders(ICD-10 F41.3):
Symptoms of anxiety mixed with features of other disorders in F42-F48. Neither type of symptom is severe enough to justify a diagnosis if considered separately.
Other specified anxiety disorders (ICD-10 F41.8):
   Anxiety hysteria
   Anxiety disorder, unspecified (ICD-10 F41.9):
   Anxiety NOS

In another preferred embodiment, the present invention relates to a pharmaceutical composition comprising an antagonist/inhibitor of neuropeptide B/W receptor (NPBWR1) for use in in a method of treating, ameliorating or preventing Parkinson's disease.

Parkinson's disease is known in the art as a chronic degenerative disorder of the central nervous system that affects both the motor system and non-motor systems. The symptoms usually emerge slowly, and as the disease worsens, non-motor symptoms become more common. Early symptoms are tremor, rigidity, slowness of movement, and difficulty with walking. Problems may also arise with cognition, behaviour, sleep, and sensory systems. Parkinson's disease dementia becomes common in advanced stages of the disease. Parkinson's disease is classified as ICD-10 G20.

In further preferred embodiments, the present invention relates to any pharmaceutical composition comprising an antagonist/inhibitor of neuropeptide B/W receptor (NPBWR1) for use in a method of treating, ameliorating or preventing a mood disorder/affective disorder and/or chronic stress and/or anxiety disorders and/or Parkinson's disease as described herein above and below, wherein said antagonist/inhibitor is selected from an NPBWR1 inhibitory peptide, NPBWR1 inhibitory small binding molecule, RNAi, siRNA, shRNA, aptamers and intramers specifically directed against NPBWR1, anti-NPBWR1 antisense molecules. Moreover, it is also envisaged that the antagonist/inhibitor of neuropeptide B/W receptor (NPBWR1) for use in a method of treating, ameliorating or preventing a mood disorder/affective disorder and/or chronic stress and/or anxiety disorders and/or Parkinson's disease as described herein above and below is an antibody.

Accordingly, compounds which may function as specific an "antagonist" or "inhibitor" of neuropeptide B/W receptor (NPBWR1) may comprise small binding molecules such as small (organic) compounds or ligands for neuropeptide B/W receptor (NPBWR1). The term "small molecule" in the context of drug discovery is known in the art and relates to medical compounds having a molecular weight of less than 2,500 Daltons, preferably less than 1,000 Daltons, more preferably between 50 and 350 Daltons. (Small) binding molecules comprise natural as well as synthetic compounds. The term "compound" in context of this invention comprises single substances or a plurality of substances. Said compound/binding molecules may be comprised in, for example, samples, e.g., cell extracts from, e.g., plants, animals or microorganisms. Furthermore, said compound(s) may be known in the art but hitherto not known to be capable of (negatively) influencing the activity neuropeptide B/W receptor (NPBWR1) or not known to be capable of influencing the expression of the nucleic acid molecule encoding for neuropeptide B/W receptor (NPBWR1), respectively.

Yet it is also envisaged in the context of the present invention that compounds including, *inter alia,* peptides, proteins, nucleic acids including cDNA expression libraries, small organic compounds, ligands, PNAs and the like can be used as an antagonist of neuropeptide B/W receptor (NPBWR1) function. Said compounds can also be functional derivatives or analogues. Methods for the preparation of chemical derivatives and analogues are well known to those skilled in the art and are described in, for example, Beilstein, "Handbook of Organic Chemistry", Springer Edition New York, or in "Organic Synthesis", Wiley, New York. Furthermore, said derivatives and analogues can be tested for their effects, i.e., their antagonistic effects of neuropeptide B/W receptor (NPBWR1) function according to methods known in the art. Furthermore, peptidomimetics and/or computer aided design of appropriate antagonists or inhibitors of neuropeptide B/W receptor (NPBWR1) can be used. Appropriate computer systems for the computer aided design of, e.g., proteins and peptides are described in the prior art, for example, in Berry (1994) Biochem. Soc. Trans. 22:1033-1036; Wodak (1987) , Ann. N. Y. Acad. Sci. 501:1-13; Pabo (1986), Biochemistry 25:5987-5991. The results obtained from the above-described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh (1996) Methods in Enzymology 267:220-234 and Dorner (1996) Bioorg. Med. Chem. 4:709-715. Furthermore, the three-dimensional and/or crystallographic structure of antagonists of neuropeptide B/W receptor (NPBWR1) can be used for the design of (peptidomimetic) antagonists of neuropeptide B/W receptor (NPBWR1).

The RNAi-approach is also envisaged in context of this invention for use in the preparation of a pharmaceutical composition for use in a method of treating, ameliorating or preventing a mood disorder/affective disorder and/or chronic stress and/or anxiety disorders and/or Parkinson's disease.

The term "RNA interference" or "inhibiting RNA" (RNAi/iRNA) describes the use of double-stranded RNA to target specific mRNAs for degradation, thereby silencing their expression. Preferred inhibiting RNA molecules may be selected from the group consisting of double-stranded RNA (dsRNA), RNAi, siRNA, shRNA and stRNA. dsRNA matching a gene sequence is synthesized in vitro and introduced into a cell. The dsRNA may also be introduced into a cell in form of a vector expressing a target gene sequence in sense and antisense orientation, for example in form of a hairpin mRNA. The sense and antisense sequences may also be expressed from separate vectors, whereby the individual antisense and sense molecules form double-stranded RNA upon their expression. It is known in the art that in some occasions the expression of a sequence in sense orientation or even of a promoter sequence suffices to give rise to dsRNA and subsequently to siRNA due to internal amplification mechanisms in a cell. Accordingly, all means and methods which result in a decrease in activity (which may be reflected in a lower expression of neuropeptide B/W receptor (NPBWR1)), in particular by taking advantage of neuropeptide B/W receptor (NPBWR1)-specific siRNAs (i.e., siRNAs that target specifically neuropeptide B/W receptor (NPBWR1) mRNA or a functional fragment thereof) are to be used in accordance with the present invention. For example sense constructs, antisense constructs, hairpin constructs, sense and antisense molecules and combinations thereof can be used to generate/introduce these siRNAs. The dsRNA feeds into a natural, but only partially understood process including the highly conserved nuclease dicer which cleaves dsRNA precursor molecules into short interfering RNAs (siRNAs). The generation and preparation of siRNA(s) as well as the method for inhibiting the expression of a target gene is, *inter alia,* described in WO 02/055693, Wei (2000) Dev. Biol. 15:239-255; La Count (2000) Biochem. Paras. 111:67-76; Baker (2000) Curr. Biol. 10:1071-1074; Svoboda (2000) Development 127:4147-4156 or Marie (2000) Curr. Biol. 10:289-292. These siRNAs built then the sequence specific part of an RNA-induced silencing complex (RISC), a multicomplex nuclease that destroys messenger RNAs homologous to the silencing trigger). Elbashir (2001) EMBO J. 20:6877-6888 showed that duplexes of 21 nucleotide RNAs may be used in cell culture to interfere with gene expression in mammalian cells. It is already known that RNAi is mediated very efficiently by siRNA in mammalian cells but the generation of stable cell lines or non-human transgenic animals was limited. However, new generations of vectors may be employed in order to stably express, e.g., short hairpin RNAs (shRNAs). Stable expression of siRNAs in Mammalian Cells is, *inter alia,* shown in Brummelkamp (2002) Science 296:550-553. Also Paul (2002) Nat. Biotechnol. 20:505-508 documented the effective expression of small interfering RNA in human cells. RNA interference by expression of short-interfering RNAs and hairpin RNAs in mammalian cells was also shown by Yu (2002) PNAS 99:6047-6052. The shRNA approach for gene silencing is well known in the art and may comprise the use of st (small temporal) RNAs; see, inter alia, Paddison (2002) Genes Dev. 16:948-958. These approaches may be vector-based, e.g. the pSUPER vector, or RNA pollll vectors may be employed as illustrated, inter alia, in Yu (2002), loc. cit.; Miyagishi (2002), loc. cit. or Brummelkamp (2002), loc. cit. It is envisaged that the regulatory sequences of the present invention are used in similar fashion as the systems based on pSUPER or RNA pollll vectors.

Methods to deduce and construct siRNAs are known in the art and are described in Elbashir (2002) Methods 26:199-213, at the internet web sites of commercial vendors of siRNA, e.g., Qiagen GmbH (https://www1.qiagen.com/GeneGlobe/Default.aspx); Dharmacon (www.dharmacon.com); Xeragon Inc. (http://www.dharmacon.com/Default.aspx), and Ambion (www.ambion.com), or at the web site of the research group of Tom Tuschl (http://www.rockefeller.edu/labheads/tuschl/sirna.html). In addition, programs are available online to deduce siRNAs from a given mRNA sequence (e.g., http://www.ambion.com/techlib/misc/siRNA_finder.html or http://katahdin.cshl.org:9331/RNAi/html/rnai.html). Uridine residues in the 2-nt 3' overhang can be replaced by 2'deoxythymidine without loss of activity, which significantly reduces costs of RNA synthesis and may also enhance resistance of siRNA duplexes when applied to mammalian cells (Elbashir (2001) loc. cit). The siRNAs may also be synthesized enzymatically using T7 or other RNA polymerases (Donze (2002) Nucleic Acids Res 30:e46). Short RNA duplexes that mediate effective RNA interference (esiRNA) may also be produced by hydrolysis with Escherichia coli RNase III (Yang (2002) PNAS 99:9942-9947). Furthermore, expression vectors have been developed to express double stranded siRNAs connected by small hairpin RNA loops in eukaryotic cells (e.g. (Brummelkamp (2002) Science 296:550-553). All of these constructs may be developed with the help of the programs named above. In addition, commercially available sequence prediction tools incorporated in sequence analysis programs or sold separately, e.g. the siRNA Design Tool offered by www.oligoEngine.com (Seattle, WA) may be used for siRNA sequence prediction.

Accordingly, specific interfering RNAs can be used in accordance with the present invention as antagonists (inhibitors) of neuropeptide B/W receptor (NPBWR1) (expression and/or function). These siRNAs are formed by an antisense and a sense strand, whereby the antisense/sense strand preferably comprises at least 10, more preferably at least 12, more preferably at least 14, more preferably at least 16, more preferably at least 18, more preferably at least 19, 20, 21 or 22 nucleotides.

As mentioned above, methods for preparing siRNAs to be used in accordance with the present invention are well known in the art. Based on the teaching provided herein, a skilled person in the art is easily in the position not only to prepare such siRNAs but also to assess whether a siRNA is capable of antagonizing/inhibiting neuropeptide B/W receptor (NPBWR1). It is envisaged herein that the above described siRNAs lead to a degradation of neuropeptide B/W receptor (NPBWR1) mRNA and thus to a decreased protein level of neuropeptide B/W receptor (NPBWR1).

In other words, siRNAs lead to a pronounced decrease in mRNA and/or protein levels of neuropeptide B/W receptor (NPBWR1) (i.e., to a reduced expression of neuropeptide B/W receptor (NPBWR1)). This decrease in expression may be reflected in a decreased activity of neuropeptide B/W receptor (NPBWR1). For example, neuropeptide B/W receptor (NPBWR1) -specific siRNAs may lead to a decreased capacity of neuropeptide B/W receptor (NPBWR1) and to inhibit neuropeptide B/W receptor (NPBWR1) activity. Hence, the use of potent antagonists/inhibitors of neuropeptide B/W receptor (NPBWR1) (such as the herein described siRNAs) will lead to a lower neuropeptide B/W receptor (NPBWR1) activity.

As used herein the term "small interfering RNA" (siRNA), sometimes known as short interfering RNA or silencing RNA, refers to a class of generally short and double-stranded RNA molecules that play a variety of roles in biology and, to an increasing extent, in treatment of a variety of diseases and conditions. As mentioned above, siRNAs are involved in the RNA interference (RNAi) pathway where the siRNA interferes with the expression of a specific gene (see, e.g., Zamore Nat Struct Biol 2001, 8(9):746-50; Tuschl T. CHEMBIOCHEM. 2001, 2:239-245; Scherrand Eder, Cell Cycle. 2007 Feb;6(4):444-9; Leung and Whittaker, Pharmacol Ther. 2005 Aug;107(2):222-39; de Fougerolles et al., Nat. Rev. Drug Discov. 2007, 6: 443-453).

Such siRNAs are generally 18-27 nt long, generally comprising a short (usually 19-21-nt) double-strand of RNA (dsRNA) with or without 2-nt 3' overhangs on either end. Each strand can have a 5' phosphate group and a 3' hydroxyl (-OH) group or the phosphate group can be absent on one or both strands. This structure is the result of processing by dicer, an enzyme that converts either long dsRNAs or small hairpin RNAs into siRNAs.

siRNAs can also be exogenously (artificially) introduced into cells by various transfection methods to bring about the specific knockdown of a gene of interest. In this context, other structures than those described above are also envisaged, provided they are capable of interfering with gene expression. Preferably, the double-stranded part has a length of about 12 to about 50 base pairs, more preferably 16 to 30, more preferably 18 to 25, more preferably 19 to 21 in length. Most preferably, the double-stranded part has a length of 19 base pairs. The siRNA of the invention may either have overhanging sequences of up to 10 bases, preferably not more than 5 bases in length at either end or at one end, or may be blunt-ended. Also preferred is that the complementarity to the target gene extends over the entire length of the double-stranded part. The region which is complementary to the target gene is at least 12 bases, preferably at least 15, 16, 17, 18, 19, 20, 21, 22, 23 or more bases in length. The siRNA of the invention may be fully complementary to the target gene. Alternatively, the siRNA may comprise up to 5%, 10%, 20% or 30% mismatches to the target gene. Furthermore, siRNAs and also antisense RNAs can be chemically modified e.g., on the backbone including the sugar residues. Preferred modifications of the siRNA molecules of the invention include linkers connecting the two strands of the siRNA molecule. Chemical modifications serve inter alia to improve the pharmacological properties of siRNAs and antisense RNAs such as in vivo stability and/or delivery to the target site within an organism. The skilled person is aware of such modified siRNAs as well as of means and methods of obtaining them, see, for example, Zhang et al., Curr Top Med Chem. 2006;6(9):893-900; Manoharan, Curr Opin Chem Biol. 2004 Dec;8(6):570-9.

Essentially any gene of which the sequence is known can thus be targeted based on sequence complementarity with an appropriately tailored siRNA. This has made siRNAs an important tool for gene function and drug target validation studies as well as for therapeutic intervention which is envisaged here. The siRNAs disclosed herein are capable of reducing or blocking the expression of neuropeptide B/W receptor (NPBWR1).

In a further aspect, it is envisaged that antisense molecules inhibit the expression or function of neuropeptide B/W receptor (NPBWR1), in particular of human neuropeptide B/W receptor (NPBWR1) and interact with neuropeptide B/W receptor (NPBWR1) as expressed by the coding regions, mRNAs/cDNAs as defined herein above as well as with neuropeptide B/W receptor (NPBWR1) as expressed by isoforms and variants of said neuropeptide B/W receptor (NPBWR1). Said isoforms or variants may, *inter alia,* comprise allelic variants or splice variants. Furthermore, it is also envisaged that the antisense molecules to be used in accordance with the present invention against neuropeptide B/W receptor (NPBWR1) expression or function interfere specifically with regulatory sequences of neuropeptide B/W receptor (NPBWR1) as defined herein below.

The term "variant" means in this context that the neuropeptide B/W receptor (NPBWR1) nucleotide sequence and the encoded neuropeptide B/W receptor (NPBWR1) amino acid sequence, respectively, differs from the distinct sequences available under the above-identified GenBank Accession numbers, by mutations, e.g. deletion, additions, substitutions, inversions etc.

Therefore, the antisense-molecule to be employed in accordance with the present invention specifically interacts with/hybridizes to one or more nucleic acid molecules encoding neuropeptide B/W receptor (NPBWR1). Preferably said nucleic acid molecule is RNA, i.e., pre m-RNA or mRNA. The term "specifically interacts with/hybridizes to one or more nucleic acid molecules encoding neuropeptide B/W receptor (NPBWR1)" relates, in context of this invention, to antisense molecules which are capable of interfering with the expression of neuropeptide B/W receptor (NPBWR1). Yet, highly mutated anti-neuropeptide B/W receptor (NPBWR1) antisense constructs, which are not capable of hybridizing to or specifically interacting with neuropeptide B/W receptor (NPBWR1)-coding nucleic acid molecules are not to be employed in the context of the present invention. The person skilled in the art can easily deduce whether an antisense construct specifically interacts with/hybridizes to neuropeptide B/W receptor (NPBWR1) encoding sequences. These tests comprise, but are not limited to hybridization assays, RNAse protection assays, Northern Blots, North-western blots, nuclear magnetic resonance and fluorescence binding assays, dot blots, micro- and macroarrays and quantitative PCR. In addition, such a screening may not be restricted to neuropeptide B/W receptor (NPBWR1) mRNA molecules, but may also include neuropeptide B/W receptor (NPBWR1) mRNA/protein (RNP) complexes (Hermann (2000) Angew Chem Int Ed Engl 39:1890-1904; DeJong (2002) CurrTrop Med Chem 2:289-302). Furthermore, functional tests including Western blots, immunohistochemistry, immunoprecipitation assay, and bioassays based on neuropeptide B/W receptor (NPBWR1)-responsive promoters are envisaged for testing whether a particular antisense construct is capable of specifically interacting with/hybridizing to the neuropeptide B/W receptor (NPBWR1) encoding nucleic acid molecules.

The term "antisense-molecule" as used herein comprises in particular antisense oligonucleotides. Said antisense oligonucleotides may also comprise modified nucleotides as well as modified internucleoside-linkage, as, *inter alia,* described in US 6,159,697.
Most preferably, the antisense oligonucleotides of the present invention comprise at least 8, more preferably at least 10, more preferably at least 12, more preferably at least 14, more preferably at least 16 nucleotides. The deduction as well as the preparation of antisense molecules is very well known in the art. The deduction of antisense molecules is, inter alia, described in Smith, 2000. Usual methods are "gene walking", Rnase H mapping, RNase L mapping (Leaman (1999) Meth Enzymol 18:252-265), combinatorial oligonucleotide arrays on solid support, determination of secondary structure analysis by computational methods (Walton (2000) Biotechnol Bioeng, 65:1-9), aptamer oligonucleotides targeted to structured nucleic acids (aptastruc), thetered oligonucleotide probes, foldback triplex-forming oligonucleotides (FTFOs) (Kandimalla (1994) Gene 149:115-121) and selection of sequences with minimized non-specific binding (Han (1994) Antisense Res Dev 4:53-65).

Preferably, the antisense molecules of the present invention are stabilized against degradation. Such stabilization methods are known in the art and, *inter alia,* described in US 6,159,697. Further methods described to protect oligonucleotides from degradation include oligonucleotides bridged by linkers (Vorobjev (2001) Antisense Nucleic Acid Drug Dev, 11:77-85), minimally modified molecules according to cell nuclease activity (Samani (2001) Antisense Nucleic Acid Drug Dev, 11:129-136), 2'O-DMAOE oligonucleotides (Prakash (2001) Nucleosides Nucleotides Nucleic Acids 20:829-832), 3'5'-Dipeptidyl oligonucleotides (Schwope (1999) J Org Chem 64:4749-4761), 3'methylene thymidine and 5-methyluridine/cytidine h-phosphonates and phosphonamidites (An (2001) J Org Chem, 66:2789-2801), as well as anionic liposome (De Oliveira (2000) Life Sci 67:1625-1637) or ionizable aminolipid (Semple (2001) Biochim Biophys Acta, 10:152-166) encapsulation.

In addition thereto, the antagonist/inhibitor of neuropeptide B/W receptor (NPBWR1) expression or function may also comprise intracellular binding partners of neuropeptide B/W receptor (NPBWR1). As used herein, the term "intracellular binding partner" relates to intracellular molecules capable of preventing or reducing neuropeptide B/W receptor (NPBWR1) activity. Such intracellular binding partners of neuropeptide B/W receptor (NPBWR1), *inter alia,* may relate to endogenous inhibitor/repressor proteins of neuropeptide B/W receptor (NPBWR1). In another embodiment of the invention the intracellular binding partner is an intracellular antibody. Intracellular antibodies are known in the art and can be used to modulate or inhibit the functional activity of the target molecule. This therapeutic approach is based on intracellular expression of recombinant antibody fragments, either Fab or single chain Fv, targeted to the desired cell compartment using appropriate targeting sequences (Teillaud (1999) Pathol Biol 47:771-775).

As mentioned herein above, the antagonist/inhibitor of neuropeptide B/W receptor (NPBWR1) expression orfunction may also comprise an aptamer. In the context of the present invention, the term "aptamer" comprises nucleic acids such as RNA, ssDNA (ss = single stranded), modified RNA, modified ssDNA or PNAs which bind a plurality of target sequences having a high specificity and affinity. Aptamers are well known in the art and, *inter alia,* described in Famulok (1998) Curr. Op. Chem. Biol. 2:320-327. The preparation of aptamers is well known in the art and may involve, *inter alia,* the use of combinatorial RNA libraries to identify binding sites (Gold (1995) Ann. Rev. Biochem. 64:763-797).

Accordingly, aptamers are oligonucleotides derived from an *in vitro* evolution process called SELEX (systematic evolution of ligands by exponential enrichment). Pools of randomized RNA or single stranded DNA sequences are selected against certain targets. The sequences of tighter binding with the targets are isolated and amplified. The selection is repeated using the enriched pool derived from the first round selection. Several rounds of this process lead to winning sequences that are called "aptamers". Aptamers have been evolved to bind proteins which are associated with a number of disease states. Using this method, many powerful antagonists of such proteins can be found. In order for these antagonists to work in animal models of disease and in humans, it is normally necessary to modify the aptamers. First of all, sugar modifications of nucleoside triphosphates are necessary to render the resulting aptamers resistant to nucleases found in serum. Changing the 2'OH groups of ribose to 2'F or 2'NH2 groups yields aptamers which are long lived in blood. The relatively low molecular weight of aptamers (8000-12000) leads to rapid clearance from the blood. Aptamers can be kept in the circulation from hours to days by conjugating them to higher molecular weight vehicles. When modified, conjugated aptamers are injected into animals, they inhibit physiological functions known to be associated with their target proteins. Aptamers may be applied systemically in animals and humans to treat organ specific diseases (Ostendorf (2001) J Am Soc Nephrol. 12:909-918). The first aptamerthat has proceeded to phase I clinical studies is NX-1838, an injectable angiogenesis inhibitor that can be potentially used to treat macular degeneration-induced blindness. (Sun (2000) Curr Opin Mol Ther 2:100-105). Cytoplasmatic expression of aptamers ("intramers") may be used to bind intracellular targets (Blind (1999) PNAS 96:3606-3610; Mayer (2001) PNAS 98:4961-4965). Said intramers are also envisaged to be employed in context of this invention.
Neuropeptide B/W receptor (NPBWR1) antagonists/inhibitors of neuropeptide B/W receptor (NPBWR1) function may be deduced by methods in the art. Such methods are described herein and, *inter alia,* may comprise, but are not limited to methods where a collection of substances is tested for interaction with neuropeptide B/W receptor (NPBWR1) or with (a) fragment(s) thereof and where substances which test positive for interaction in a corresponding readout system are further tested *in vivo, in vitro* or *in silica* for their inhibiting effects on neuropeptide B/W receptor (NPBWR1) expression or function.

Said "test for neuropeptide B/W receptor (NPBWR1) interaction" of the above described method may be carried out by specific immunological, molecular biological and/or biochemical assays which are well known in the art and which comprise, e.g., homogenous and heterogenous assays as described herein below. Neuropeptide B/W receptor (NPBWR1) ligands capable of inhibiting neuropeptide B/W receptor (NPBWR1) function may be identified by screening large compound libraries based on their capacity to interact with the neuropeptide B/W receptor (NPBWR1) protein. In a preferred embodiment, such antagonists or inhibitors of neuropeptide B/W receptor (NPBWR1) function are capable of binding the protein binding domain of neuropeptide B/W receptor (NPBWR1).

Besides molecules capable of binding to neuropeptide B/W receptor (NPBWR1), antagonists or inhibitors of neuropeptide B/W receptor (NPBWR1) function may be capable of preventing/reducing the expression of the nucleic acid molecule encoding the neuropeptide B/W receptor (NPBWR1) protein. The skilled person is readily capable of identifying regulatory sequences (such as promoter sequences, enhancer sequences, replication origins and other regulatory elements) of neuropeptide B/W receptor (NPBWR1) expression e.g., by using *in silica* gene prediction methods and experimental validation of functional sites (Elnitski (2006) Genome Res 16:1455-64).

As already mentioned above, it is also envisaged that the antagonist/inhibitor of neuropeptide B/W receptor (NPBWR1) for use in a method of treating, ameliorating or preventing a mood disorder/affective disorder and/or chronic stress and/or anxiety disorders and/or Parkinson's disease as described herein above and below is an antibody.

Preferably, the antibody is an antibody against human neuropeptide B/W receptor (NPBWR1), i.e. the antibody is an inhibitory antibody against human neuropeptide B/W receptor (NPBWR1). An antibody (interchangeably used in plural form) as used herein is an immunoglobulin molecule capable of specific binding to a target, such as a carbohydrate, polynucleotide, lipid, polypeptide, etc., through at least one antigen recognition site, located in the variable region of the immunoglobulin molecule. The preferred target herein is neuropeptide B/W receptor (NPBWR1), particularly human neuropeptide B/W receptor (NPBWR1). As used herein, the term "antibody" encompasses not only intact (i.e., full-length) monoclonal antibodies, but also antigen-binding fragments (such as Fab, Fab', F(ab')2, Fv, single chain variable fragment (scFv)), mutants thereof, fusion proteins comprising an antibody portion, humanized antibodies, chimeric antibodies, diabodies, linear antibodies, single chain antibodies, single domain antibodies (e.g., camel or llama VHH antibodies), multi-specific antibodies (e.g., bispecific antibodies) and any other modified configuration of the immunoglobulin molecule that comprises an antigen recognition site of the required specificity, including glycosylation variants of antibodies, amino acid sequence variants of antibodies, and covalently modified antibodies. An antibody includes an antibody of any class, such as IgD, IgE, IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.
An antibody that "specifically binds" (used interchangeably herein) to a target or an epitope is a term well understood in the art, and methods to determine such specific binding are also well known in the art. A molecule is said to exhibit "specific binding" if it reacts or associates more frequently, more rapidly, with greater duration and/or with greater affinity with a particular target antigen than it does with alternative targets. An antibody "specifically binds" to a target antigen if it binds with greater affinity, avidity, more readily, and/or with greater duration than it binds to other substances. For example, an antibody that specifically (or preferentially) binds to a neuropeptide B/W receptor (NPBWR1) epitope is an antibody that binds this neuropeptide B/W receptor (NPBWR1) epitope with greater affinity, avidity, more readily, and/or with greater duration than it binds to other neuropeptide B/W receptor (NPBWR1) epitopes or non-neuropeptide B/W receptor (NPBWR1) epitopes. It is also understood by reading this definition that, for example, an antibody that specifically binds to a first target antigen may or may not specifically or preferentially bind to a second target antigen. As such, "specific binding" or "preferential binding" does not necessarily require (although it can include) exclusive binding. Generally, but not necessarily, reference to binding means preferential binding.
An inhibitor of neuropeptide B/W receptor (NPBWR1) may be an anti-neuropeptide B/W receptor (NPBWR1) specific antibody. An anti-neuropeptide B/W receptor (NPBWR1) antibody is an antibody capable of binding to neuropeptide B/W receptor (NPBWR1), which may inhibit neuropeptide B/W receptor (NPBWR1) biological activity and/or downstream pathway components mediated by neuropeptide B/W receptor (NPBWR1). In some examples, an anti- neuropeptide B/W receptor (NPBWR1) antibody used in the methods described herein suppresses neuropeptide B/W receptor (NPBWR1) biological activity by at least 10%, at least 20%, at least 30%, at least 40%, at least 50%, at least 60%, at least 70%, at least 80%, at least 90%, at least 100%, or by at least 2-fold, at least 5-fold, at least 10-fold, at least 20-fold, at least 50-fold, at least 100-fold, or at least 1000-fold.

Accordingly, the present invention relates in one aspect to an anti-neuropeptide B/W receptor (NPBWR1) antibody for use in a method of treating, ameliorating or preventing a mood disorder/affective disorder and/or chronic stress and/or anxiety disorders and/or Parkinson's disease as described herein above and below.
Anti-neuropeptide B/W receptor (NPBWR1) antibodies are well known in the art and are commercially available. The skilled person is readily capable of choosing and/or generating suitable anti-neuropeptide B/W receptor (NPBWR1) antibodies.

In a particular embodiment, the present invention relates to an antibody and the use thereof that specifically binds to the polypeptide or fragments thereof as shown in SEQ ID NO:1.
As mentioned above, in context of the present invention, the term "antibody" as used herein relates in particular to full immunoglobulin molecules as well as to parts of such immunoglobulin molecules substantially retaining binding specificity. Furthermore, the term relates to modified and/or altered antibody molecules, like chimeric and humanized antibodies, CDR-grafted antibodies, recombinantly or synthetically generated/synthesized antibodies and to intact antibodies as well as to antibody fragments thereof, like, separated light and heavy chains, Fab, Fab/c, Fv, Fab', F(ab')₂. The term "antibody" also comprises bifunctional antibodies, trifunctional antibodies and antibody constructs, like single chain Fvs (scFv) or antibody-fusion proteins. Further "antibody" constructs are known in the art and comprised in the present invention.
Techniques for the production of antibodies are well known in the art and described, e.g., in Howard and Bethell (2000) Basic Methods in Antibody Production and Characterization, Crc. Pr. Inc. Antibodies directed against a polypeptide according to the present invention can be obtained, e.g., by direct injection of the polypeptide (or a fragment thereof) into an animal or by administering the polypeptide (or a fragment thereof) to an animal. The antibody so obtained will then bind polypeptide (or a fragment thereof) itself. In this manner, even a fragment of the polypeptide can be used to generate antibodies binding the whole polypeptide, as long as said binding is "specific" as defined above.

With the normal skill of the person skilled in the art and by routine methods, the person skilled in the art can easily deduce from the sequences provided herein relevant epitopes (also functional fragments) of the polypeptides of the present invention which are useful in the generation of antibodies like polyclonal and monoclonal antibodies. However, the person skilled in the art is readily in a position to also provide for engineered antibodies like CDR-grafted antibodies or also humanized and fully human antibodies and the like.

Particularly preferred in the context of the present invention are monoclonal antibodies. For the preparation of monoclonal antibodies, any technique which provides antibodies produced by continuous cell line cultures can be used. Examples for such techniques include the hybridoma technique, the trioma technique, the human B-cell hybridoma technique and the EBV-hybridoma technique to produce human monoclonal antibodies (Shepherd and Dean (2000), Monoclonal Antibodies: A Practical Approach, Oxford University Press, Goding and Goding (1996), Monoclonal Antibodies: Principles and Practice - Production and Application of Monoclonal Antibodies in Cell Biology, Biochemistry and Immunology, Academic Pr Inc, USA).

The antibody derivatives can also be produced by peptidomimetics. Further, techniques described for the production of single chain antibodies (see, inter alia, US Patent 4,946,778) can be adapted to produce single chain antibodies specifically recognizing the polypeptide of the invention. Also, transgenic animals may be used to express humanized antibodies to the polypeptide of the invention.

The production of a specific antibody against native polypeptides and recombinant polypeptides is based, for example, on the immunization of animals, like mice. However, also other animals for the production of antibody/antisera are envisaged within the present invention. For example, monoclonal and polyclonal antibodies can be produced by rabbit, mice, goats, donkeys and the like. The polynucleotide according to the invention as shown in SEQ ID NO:2 can be subcloned into an appropriated vector, wherein the recombinant polypeptide is to be expressed in an organism being able for an expression, for example in bacteria. Thus, the expressed recombinant protein can be intra-peritoneally injected into a mice and the resulting specific antibody can be, for example, obtained from the mice serum being provided by intra-cardiac blood puncture. The amount of obtained specific antibody can be quantified using an ELISA, which is also described herein below. Further methods for the production of antibodies are well known in the art, see, e.g. Harlow and Lane, "Antibodies, A Laboratory Manual", CSH Press, Cold Spring Harbor, 1988.

The term "specifically binds", as used herein, refers to a binding reaction that is determinative of the presence of the neuropeptide B/W receptor (NPBWR1) protein and antibody in the presence of a heterogeneous population of proteins and other biologics.

Thus, under designated assay conditions, the specified antibodies and neuropeptide B/W receptor (NPBWR1) proteins bind to one another and do not bind in a significant amount to other components present in a sample. Specific binding to a target analyte under such conditions may require a binding moiety that is selected for its specificity for a particular target analyte. A variety of immunoassay formats may be used to select antibodies specifically reactive with a particular antigen. For example, solid-phase ELISA immunoassays are routinely used to select monoclonal antibodies specifically immunoreactive with an analyte. See Shepherd and Dean (2000), Monoclonal Antibodies: A Practical Approach, Oxford University Press and/ or Howard and Bethell (2000) Basic Methods in Antibody Production and Characterization, Crc. Pr. Inc. for a description of immunoassay formats and conditions that can be used to determine specific immunoreactivity. Typically a specific or selective reaction will be at least twice background signal to noise and more typically more than 10 to 100 times greater than background. The person skilled in the art is in a position to provide for and generate specific binding molecules directed against the novel polypeptides. For specific binding-assays it can be readily employed to avoid undesired cross-reactivity, for example polyclonal antibodies can easily be purified and selected by known methods (see Shepherd and Dean, loc. cit.).

The term "purification or detection", as used herein, refers to a series of processes intended to isolate or detect a single type of protein from a complex mixture employing the "specifical binding" as defined above, that refers to a binding reaction that is determinative of the presence of the neuropeptide B/W receptor (NPBWR1) protein and antibody in the presence of a heterogeneous population of proteins and other biologics. Protein purification or detection is vital for the characterization of the function, structure and interactions of the protein of interest. The starting material, as a non-limiting example, can be a biological tissue or a microbial culture. The various steps in the purification or detection process may free the protein from a matrix that confines it, separate the protein and non-protein parts of the mixture, and finally separate the desired protein from all other proteins. Separation steps exploit differences in protein size, physico-chemical properties and binding affinity.

In further preferred embodiments, the present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease, wherein said antagonist/inhibitor is an NPBWR1 inhibitory small binding molecule and has the following chemical structure represented by Formula (1) 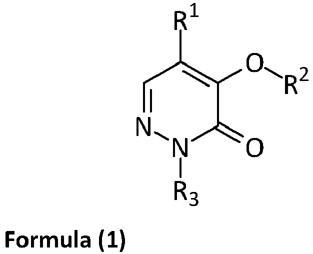 wherein:
R¹ is selected from F, Cl, Br, I and CN;
R² is selected from -(heterocyclyl with 5 to 10 ring atoms and optionally one or more substituents R^{2a}), and -(carbocyclyl with 6 to 10 ring atoms and optionally one or more substituents R^{2a});
R³ is selected from -(heterocyclyl with 5 to 20 ring atoms and optionally one or more substituents R^{3a}), -(C₁₋₄ alkylene which is optionally substituted with one or more substituents R^{Alk})-(heterocyclyl with 5 to 20 ring atoms and optionally one or more substituents R^{3a}), - (carbocyclyl with 6 to 20 ring atoms and optionally one or more substituents R^{3a}), and -(C₁₋₄ alkylene which is optionally substituted with one or more substituents R^{Alk})-(carbocyclyl with 6 to 20 ring atoms and optionally one or more substituents R^{3a}),
   wherein
each R^{2a} is independently selected from the group consisting of -halogen, -CN, -CF₃, -CHF₂, -CH₂F, -OCF₃, -OCHF₂, -OCH₂F, -NR*R*, -NR*COR*, -NR*C(O)NR*R*, -NR^{∗}S(O₂)NR^{∗}R^{∗}, - C(O)OR*, -C(O)NR*R*, -OH, or -O-C₁₋₆ alkyl, wherein each R* is independently selected from H or C₁₋₆ alkyl or C₁₋₆ cycloalkyl;
each R^{3a} is independently selected from the group consisting of -halogen, -CN, -CF₃, -CHF₂, -CH₂F, -OCF₃, -OCHF₂, -OCH₂F, -NR*R*, -NR*COR*, -NR*C(O)NR*R*, -NR^{∗}S(O₂)NR^{∗}R^{∗}, - C(O)OR*, -C(O)NR*R*, -OH, or -O-C₁₋₆ alkyl, wherein each R* is independently selected from H or C₁₋₆ alkyl or C₁₋₆ cycloalkyl;
each R^{Alk} is independently selected from the group consisting of-halogen and -CN;
or a pharmaceutically acceptable salt, solvate or prodrug thereof.

In further more preferred embodiments, the present invention relates to the aforementioned pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease, wherein said antagonist/inhibitor is an NPBWR1 inhibitory small binding molecule and has the chemical structure represented by Formula (1) as defined above, and wherein one or more of the following are fulfilled in Formula (1):
a) R¹ is selected from F, Cl, Br and CN; preferably from F, Cl and CN; more preferably from F and Cl; even more preferably from Cl;
b) R² is selected from -(heteroaryl with 5 to 10 ring atoms and optionally one or more substituents R^{2a}), and -(aryl with 6 to 10 ring atoms and optionally one or more substituents R^{2a}); preferably from -(heteroaryl with 5 to 7 ring atoms and optionally one or more substituents R^{2a}), and -(aryl with 6 or 10 ring atoms and optionally one or more substituents R^{2a}); more preferably from benzene, naphthalene, pyrrole, furan, imidazole, pyrazole, oxazole, thiazole and pyridine, any of which may optionally be substituted with one or more substituents R^{2a}; even more preferably from benzene, naphthalene, imidazolidine, oxazole and pyridine, any of which may optionally be substituted with one or more substituents R^{2a}; still more preferably from benzene, imidazolidine, oxazole and pyridine, any of which may optionally be substituted with one or more substituents R^{2a}; even still more preferably from benzene which may optionally be substituted with one or more substituents R^{2a}; still even more preferably from benzene which substituted with one or more substituents R^{2a}; most preferably from benzene being substituted at 4-position with R^{2a};
c) R³ is selected from -(heterocyclyl with 5 to 14 ring atoms and optionally one or more substituents R^{3a}), -(C₁₋₄ alkylene which is optionally substituted with one or more substituents R^{Alk})-(heterocyclyl with 5 to 14 ring atoms and optionally one or more substituents R^{3a}), - (carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); preferably from -(carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); more preferably from -(aryl with 6 to 14 ring atoms and optionally one or more substituents R^{3a}), and -(C₁₋₄ alkylene which is optionally substituted with one or more substituents R^{Alk})-(aryl with 6 to 14 ring atoms and optionally one or more substituents R^{3a}); even more preferably from -(aryl with 6 to 10 ring atoms and optionally one or more substituents R^{3a}), and -(C₁₋₄ alkylene which is optionally substituted with one or more substituents R^{Alk})-(aryl with 6 to 10 ring atoms and optionally one or more substituents R^{3a}); still more preferably from -(aryl with 6 or 10 ring atoms and optionally one or more substituents R^{3a}), and -(C₁₋₄ alkylene which is optionally substituted with one or more substituents R^{Alk})-(aryl with 6 or 10 ring atoms and optionally one or more substituents R^{3a}); even still more preferably from -(phenyl having one or more substituents R^{3a}), and -(C₁₋₄ alkylene which is optionally substituted with one or more substituents R^{Alk})-(naphthyl with optionally one or more substituents R^{3a}); still even more preferably 2,5-dimethylphenyl or 1-naphthylmethyl;
d) C₁₋₄ alkylene is preferably methylene or ethylene, more preferably methylene;
e) there are none, one, two or three substituents R^{2a}; preferably none, one or two substituents R^{2a}; more preferably one substituent R^{2a}; even more preferably one substituent R^{2a} in 4-position;
f) there are none, one, two or three substituents R^{3a;} preferably none, one or two substituents R^{3a}; more preferably two substituents R^{3a} in case R³ does not contain the -(C₁₋₄ alkylene) group, and more preferably one or no substituent R^{3a} in case R³ contains the -(C₁₋₄ alkylene) group;
g) there are none, one, two or three substituents R^{Alk;} preferably none, one or two substituents R^{Alk}; more preferably noen or one substituent R^{Alk}; even more preferably no substituent R^{Alk};
h) each R^{2a} is independently selected from the group consisting of -halogen, -CN, C₁₋₆ alkyl, C₁₋₆ haloalkyl, C₁₋₆ cycloalkyl, -NR*R*, -NR*COR*, -NR*C(O)NR*R*, -NR^{∗}S(O₂)NR^{∗}R^{∗}, - C(O)OR*, -C(O)NR*R*, -OH, -O-C₁₋₆ alkyl or -O-C₁₋₆ haloalkyl, wherein each R* is independently selected from H or C₁₋₆ alkyl or C₁₋₆ cycloalkyl; preferably each R^{2a} is independently selected from the group consisting of -halogen, -CN, C₁₋₃ alkyl, C₁₋₃ haloalkyl, cyclopropyl, -NR*R*, -NR*COR*, -NR*C(O)NR*R*, -C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; more preferably each R^{2a} is independently selected from the group consisting of -halogen, -CN, C₁₋₃ alkyl, -NR*R*, -NR*COR*, -C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; even more preferably each R^{2a} is independently selected from the group consisting of -halogen, -CN, C₁₋₃ alkyl or -O-C₁₋₃ alkyl; still more preferably each R^{2a} is independently selected from the group consisting of -halogen, -CN, or -O-C₁₋₃ alkyl; even more preferably each R^{2a} is independently -O-C₁₋₃ alkyl; still even more preferably each R^{2a} is independently methoxy or ethoxy, most preferably methoxy.
i) each R^{3a} is independently selected from the group consisting of -halogen, -CN, C₁₋₆ alkyl, C₁₋₆ haloalkyl, C₃₋₆ cycloalkyl, -NR*R*, -NR*COR*, -NR*C(O)NR*R*, -NR^{∗}S(O₂)NR^{∗}R^{∗}, -C(O)OR*, -C(O)NR*R*, -OH, -O-C₁₋₆ alkyl or -O-C₁₋₆ haloalkyl, wherein each R* is independently selected from H or C₁₋₆ alkyl or C₃₋₆ cycloalkyl; preferably each R^{3a} is independently selected from the group consisting of-halogen, -CN, C₁₋₆ alkyl, C₁₋₆ haloalkyl, C₃₋₆ cycloalkyl, -O-C₁₋₆ alkyl or -O-C₁₋₆ haloalkyl; more preferably each R^{3a} is independently selected from the group consisting of -halogen, -CN, C₁₋₆ alkyl, C₁₋₆ haloalkyl, or C₃₋₆ cycloalkyl; even more preferably each R^{3a} is independently selected from the group consisting of -halogen, -CN, C₁₋₃ alkyl, or C₁₋₃ haloalkyl; still more preferably each R^{3a} is independently selected from the group consisting of -halogen and C₁₋₃ alkyl; even still more preferably each R^{3a} is independently selected from the group consisting of C₁₋₃ alkyl, such as methyl or ethyl, more preferably methyl;
j) each R^{Alk} is independently selected from the group consisting of -F and -Cl; preferably each R^{Alk} is -F; more preferably each R^{Alk} is absent; and/or
k) the NPBWR1 inhibitory small binding molecule has the following chemical structure represented by Formula (1) or a pharmaceutically acceptable salt or solvate thereof.

In further even more preferred embodiments, the present invention relates to the aforementioned pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease,
wherein said antagonist/inhibitor is an NPBWR1 inhibitory small binding molecule and has one of the following chemical structures represented by Formulae (2) to (9): 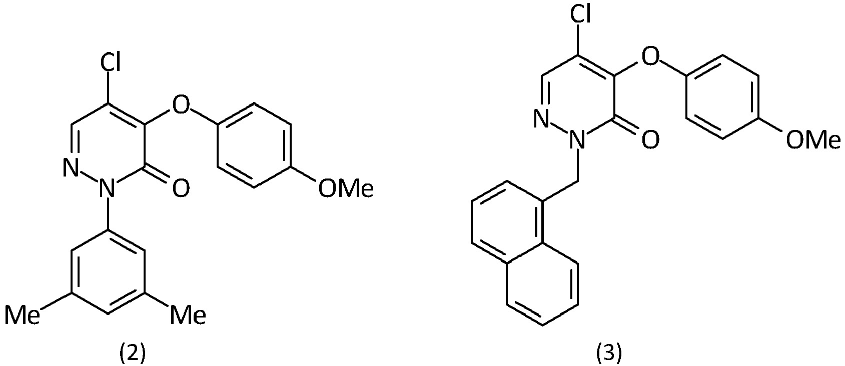 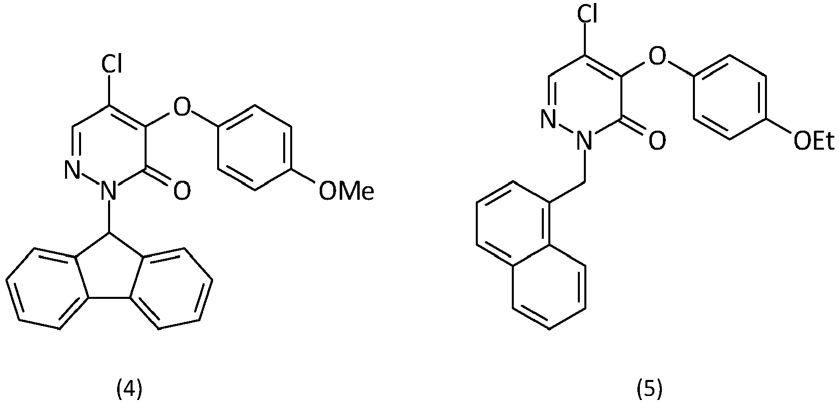 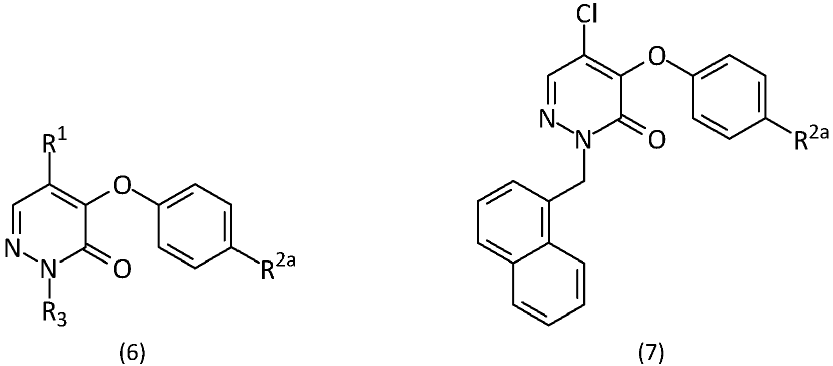 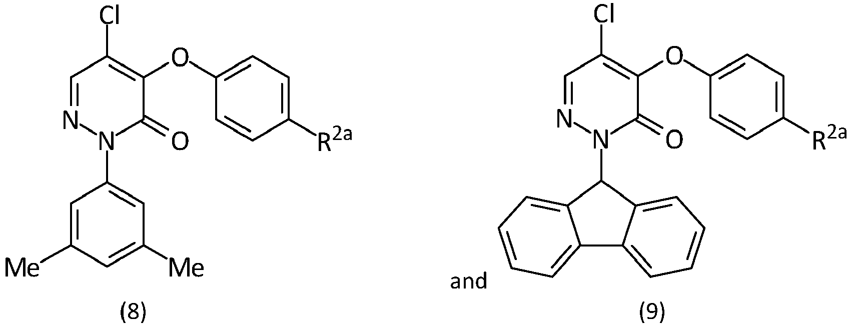 wherein R¹, R^{2a}, and R³ are as defined above.

Thus, in the context of the present invention, the NPBWR1 inhibitory small binding molecule preferably has the above chemical structure represented by **Formula (1)**
wherein:
R1 is selected from F, Cl, Br, I and CN;
R² is selected from -(heterocyclyl with 5 to 10 ring atoms and optionally one or more substituents R^{2a}), and -(carbocyclyl with 6 to 10 ring atoms and optionally one or more substituents R^{2a});
R³ is selected from -(heterocyclyl with 5 to 20 ring atoms and optionally one or more substituents R^{3a}), -(C₁₋₄ alkylene which is optionally substituted with one or more substituents R^{Alk})-(heterocyclyl with 5 to 20 ring atoms and optionally one or more substituents R^{3a}), - (carbocyclyl with 6 to 20 ring atoms and optionally one or more substituents R^{3a}), and -(C₁₋₄ alkylene which is optionally substituted with one or more substituents R^{Alk})-(carbocyclyl with 6 to 20 ring atoms and optionally one or more substituents R^{3a}),
   wherein
each R^{2a} is independently selected from the group consisting of -halogen, -CN, -CF₃, -CHF₂, -CH₂F, -OCF₃, -OCHF₂, -OCH₂F, -NR*R*, -NR*COR*, -NR*C(O)NR*R*, -NR^{∗}S(O₂)NR^{∗}R^{∗}, - C(O)OR*, -C(O)NR*R*, -OH, or -O-C₁₋₆ alkyl, wherein each R* is independently selected from H or C₁₋₆ alkyl or C₁₋₆ cycloalkyl;
each R^{3a} is independently selected from the group consisting of -halogen, -CN, -CF₃, -CHF₂, -CH₂F, -OCF₃, -OCHF₂, -OCH₂F, -NR*R*, -NR*COR*, -NR*C(O)NR*R*, -NR^{∗}S(O₂)NR^{∗}R^{∗}, - C(O)OR*, -C(O)NR*R*, -OH, or -O-C₁₋₆ alkyl, wherein each R* is independently selected from H or C₁₋₆ alkyl or C₁₋₆ cycloalkyl;
each R^{Alk} is independently selected from the group consisting of-halogen and -CN;
or a pharmaceutically acceptable salt, solvate or prodrug thereof.

One or more of the following are fulfilled in **Formula (1):**
a) R¹ is selected from F, Cl, Br and CN; preferably from F, Cl and CN; more preferably from F and Cl; even more preferably from Cl;
b) R² is selected from -(heteroaryl with 5 to 10 ring atoms and optionally one or more substituents R^{2a}), and -(aryl with 6 to 10 ring atoms and optionally one or more substituents R^{2a}); preferably from -(heteroaryl with 5 to 7 ring atoms and optionally one or more substituents R^{2a}), and -(aryl with 6 or 10 ring atoms and optionally one or more substituents R^{2a}); more preferably from benzene, naphthalene, pyrrole, furan, imidazole, pyrazole, oxazole, thiazole and pyridine, any of which may optionally be substituted with one or more substituents R^{2a}; even more preferably from benzene, naphthalene, imidazolidine, oxazole and pyridine, any of which may optionally be substituted with one or more substituents R^{2a}; still more preferably from benzene, imidazolidine, oxazole and pyridine, any of which may optionally be substituted with one or more substituents R^{2a}; even still more preferably from benzene which may optionally be substituted with one or more substituents R^{2a}; still even more preferably from benzene which substituted with one or more substituents R^{2a}; most preferably from benzene being substituted at 4-position with R^{2a};
c) R³ is selected from -(heterocyclyl with 5 to 14 ring atoms and optionally one or more substituents R^{3a}), -(C₁₋₄ alkylene which is optionally substituted with one or more substituents R^{Alk})-(heterocyclyl with 5 to 14 ring atoms and optionally one or more substituents R^{3a}), - (carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); preferably from -(carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); more preferably from -(aryl with 6 to 14 ring atoms and optionally one or more substituents R^{3a}), and -(C₁₋₄ alkylene which is optionally substituted with one or more substituents R^{Alk})-(aryl with 6 to 14 ring atoms and optionally one or more substituents R^{3a}); even more preferably from -(aryl with 6 to 10 ring atoms and optionally one or more substituents R^{3a}), and -(C₁₋₄ alkylene which is optionally substituted with one or more substituents R^{Alk})-(aryl with 6 to 10 ring atoms and optionally one or more substituents R^{3a}); still more preferably from -(aryl with 6 or 10 ring atoms and optionally one or more substituents R^{3a}), and -(C₁₋₄ alkylene which is optionally substituted with one or more substituents R^{Alk})-(aryl with 6 or 10 ring atoms and optionally one or more substituents R^{3a}); even still more preferably from -(phenyl having one or more substituents R^{3a}), and -(C₁₋₄ alkylene which is optionally substituted with one or more substituents R^{Alk})-(naphthyl with optionally one or more substituents R^{3a}); still even more preferably 2,5-dimethylphenyl or 1-naphthylmethyl;
d) C₁₋₄ alkylene is preferably methylene or ethylene, more preferably methylene;
e) there are none, one, two or three substituents R^{2a}; preferably none, one or two substituents R^{2a}; more preferably one substituent R^{2a}; even more preferably one substituent R^{2a} in 4-position;
f) there are none, one, two or three substituents R^{3a}; preferably none, one or two substituents R^{3a}; more preferably two substituents R^{3a} in case R³ does not contain the -(C₁₋₄ alkylene) group, and more preferably one or no substituent R^{3a} in case R³ contains the -(C₁₋₄ alkylene) group;
g) there are none, one, two or three substituents R^{Alk;} preferably none, one or two substituents R^{Alk}; more preferably noen or one substituent R^{Alk}; even more preferably no substituent R^{Alk};
h) each R^{2a} is independently selected from the group consisting of -halogen, -CN, C₁₋₆ alkyl, C₁₋₆ haloalkyl, C₁₋₆ cycloalkyl, -NR*R*, -NR*COR*, -NR*C(O)NR*R*, -NR^{∗}S(O₂)NR^{∗}R^{∗}, - C(O)OR*, -C(O)NR*R*, -OH, -O-C₁₋₆ alkyl or -O-C₁₋₆ haloalkyl, wherein each R* is independently selected from H or C₁₋₆ alkyl or C₁₋₆ cycloalkyl; preferably each R^{2a} is independently selected from the group consisting of -halogen, -CN, C₁₋₃ alkyl, C₁₋₃ haloalkyl, cyclopropyl, -NR*R*, -NR*COR*, -NR*C(O)NR*R*, -C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; more preferably each R^{2a} is independently selected from the group consisting of -halogen, -CN, C₁₋₃ alkyl, -NR*R*, -NR*COR*, -C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; even more preferably each R^{2a} is independently selected from the group consisting of -halogen, -CN, C₁₋₃ alkyl or -O-C₁₋₃ alkyl; still more preferably each R^{2a} is independently selected from the group consisting of -halogen, -CN, or -O-C₁₋₃ alkyl; even more preferably each R^{2a} is independently -O-C₁₋₃ alkyl; still even more preferably each R^{2a} is independently methoxy or ethoxy, most preferably methoxy.
i) each R^{3a} is independently selected from the group consisting of -halogen, -CN, C₁₋₆ alkyl, C₁₋₆ haloalkyl, C₃₋₆ cycloalkyl, -NR*R*, -NR*COR*, -NR*C(O)NR*R*, -NR^{∗}S(O₂)NR^{∗}R^{∗}, -C(O)OR*, -C(O)NR*R*, -OH, -O-C₁₋₆ alkyl or -O-C₁₋₆ haloalkyl, wherein each R* is independently selected from H or C₁₋₆ alkyl or C₃₋₆ cycloalkyl; preferably each R^{3a} is independently selected from the group consisting of-halogen, -CN, C₁₋₆ alkyl, C₁₋₆ haloalkyl, C₃₋₆ cycloalkyl, -O-C₁₋₆ alkyl or -O-C₁₋₆ haloalkyl; more preferably each R^{3a} is independently selected from the group consisting of -halogen, -CN, C₁₋₆ alkyl, C₁₋₆ haloalkyl, or C₃₋₆ cycloalkyl; even more preferably each R^{3a} is independently selected from the group consisting of -halogen, -CN, C₁₋₃ alkyl, or C₁₋₃ haloalkyl; still more preferably each R^{3a} is independently selected from the group consisting of -halogen and C₁₋₃ alkyl; even still more preferably each R^{3a} is independently selected from the group consisting of C₁₋₃ alkyl, such as methyl or ethyl, more preferably methyl;
j) each R^{Alk} is independently selected from the group consisting of -F and -Cl; preferably each R^{Alk} is -F; more preferably each R^{Alk} is absent; and/or
k) the NPBWR1 inhibitory small binding molecule has the following chemical structure represented by Formula (1) or a pharmaceutically acceptable salt or solvate thereof.

Moreover, in the context of the present invention, the NPBWR1 inhibitory small binding molecule more preferably has one of the above chemical structures represented by **Formulae (2) to (9):**
wherein R¹, R^{2a}, and R³ are as defined above.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. The term "alkyl" preferably refers to a "C₁₋₆ alkyl". A "C₁₋₆ alkyl" denotes an alkyl group having 1 to 6 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" more preferably refers to C₁₋₄ alkyl, more preferably to methyl or ethyl, and even more preferably to methyl.

"Halogen" represents F, Cl, Br and I, more preferably F or Cl, and, unless otherwise specified, even more preferably F.

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. In the case where haloalkyl is a substituent at an oxygen, the carbon (of the haloalkyl) next to the oxygen preferably has no halogen directly bound to it.

The term "aryl" preferably refers to an aromatic monocyclic ring containing 5 or 6 carbon atoms, an aromatic bicyclic ring system containing 10 carbon atoms or an aromatic tricyclic ring system containing 14 carbon atoms. Examples are phenyl, naphthyl or anthracenyl, preferably phenyl.

The term "heteroaryl" preferably refers to a five or six-membered aromatic ring wherein one or more of the carbon atoms in the ring have been replaced by 1, 2, 3, or 4 (for the five membered ring) or 1, 2, 3, 4, or 5 (for the six membered ring) of the same or different heteroatoms. The heteroatoms are preferably selected from O, N and S. Examples of the heteroaryl group are given below.

The term "heterocyclyl" covers any mono-, bi- or polycyclic ring system which includes one or more heteroatoms in the ring system, whereby the heteroatoms are the same or different and are selected from O, N and S. Preferably the ring system includes 3 to 15 ring atoms. More preferably the ring system is mono- or bicyclic and has 5 to 10 ring atoms, even more preferably the ring system is monocyclic and has 5 or 6 ring atoms. Typically the ring system can include 1 to 4, more typically 1 or 2 heteroatoms at available positions. The term "heterocyclyl" also covers heteroaryl rings. Examples include azetidine, pyrrole, pyrrolidine, oxolane, furan, imidazolidine, imidazole, pyrazole, oxazolidine, oxazole, thiazole, piperidine, pyridine, morpholine, piperazine, and dioxolane.

The term "carbocyclyl" covers any mono-, bi- or polycyclic ring system which does not include heteroatoms in the ring. Preferably the ring system includes 3 to 15 ring atoms. More preferably the ring system is mono- or bicyclic and has 5 to 10 ring atoms, even more preferably the ring system is monocyclic and has 5 or 6 ring atoms. The term "carbocyclic ring" also covers aryl rings.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, or adamantyl. Unless defined otherwise, "cycloalkyl" preferably refers to a C₃₋₁₁ cycloalkyl, and more preferably refers to a C₃₋₈ cycloalkyl. A particularly preferred "cycloalkyl" is a monocyclic saturated hydrocarbon ring having 3 to 8 ring members, most preferably 3 ring members.

If a compound or moiety is referred to as being "optionally substituted" it can in each instance include one or more of the indicated substituents, whereby the substituents can be the same or different.

As used herein, unless explicitly indicated otherwise or contradicted by context, the terms "a", "an" and "the" are used interchangeably with "one or more" and "at least one". Thus, for example, a composition comprising "a" compound of the present invention (in particular of formula (1)) can be interpreted as referring to a composition comprising "one or more" compounds of the present invention.

As used herein, the term "comprising" (or "comprise", "comprises", "contain", "contains", or "containing"), unless explicitly indicated otherwise or contradicted by context, has the meaning of "containing, *inter alia*", i.e., "containing, among further optional elements, ...". In addition thereto, this term also includes the narrower meanings of "consisting essentially of" and "consisting of". For example, the term "A comprising B and C" has the meaning of "A containing, *inter alia,* B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Moreover, unless indicated otherwise, any reference to an industry standard, a pharmacopeia, or a manufacturer's manual refers to the corresponding latest version that was available at the priority date (i.e., at the earliest filing date) of the present specification.

The scope of the invention embraces all pharmaceutically acceptable salt forms of the compounds provided herein, particularly the compounds of the present invention (in particular of formula (1)), which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts.

Moreover, the scope of the invention embraces the compounds provided herein, particularly the compounds of the present invention (in particular of formula (1)), in any solvated form, including, e.g., solvates with water (i.e., as a hydrate) or solvates with organic solvents such as, e.g., methanol, ethanol or acetonitrile (i.e., as a methanolate, ethanolate or acetonitrilate), or in any crystalline form (i.e., as any polymorph), or in amorphous form. It is to be understood that such solvates of the compounds provided herein, particularly the compounds of the present invention, also include solvates of pharmaceutically acceptable salts of the corresponding compounds.

Furthermore, the compounds provided herein, particularly the compounds of formula (1), may exist in the form of different isomers, in particular stereoisomers (including, e.g., geometric isomers (or cis/trans isomers), enantiomers and diastereomers) or tautomers. All such isomers of the compounds provided herein are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds provided herein.

The scope of the invention also embraces the compounds provided herein, particularly the compounds of formula (1), in which one or more atoms are replaced by a specific isotope of the corresponding atom. For example, the invention encompasses compounds of formula (1), in which one or more hydrogen atoms (or, e.g., all hydrogen atoms) are replaced by deuterium atoms (i.e., ²H; also referred to as "D"). Accordingly, the invention also embraces compounds of formula (1) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (1) can be increased using deuteration techniques known in the art. For example, a compound of formula (1)or a reactant or precursor to be used in the synthesis of the compound of formula (1)can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; or Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (1) is not enriched in deuterium. Accordingly, the presence of naturally occurring hydrogen atoms or ¹H hydrogen atoms in the compounds of formula (1)is preferred.

The present invention also embraces the compounds provided herein, particularly the compounds of formula (1), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers or imaging probes in positron emission tomography (PET). The invention thus includes (1) compounds of formula (1), in which one or more fluorine atoms (or, e.g., all fluorine atoms) are replaced by ¹⁸F atoms, (ii) compounds of formula (1), in which one or more carbon atoms (or, e.g., all carbon atoms) are replaced by ¹¹C atoms, (iii) compounds of formula (1), in which one or more nitrogen atoms (or, e.g., all nitrogen atoms) are replaced by ¹³N atoms, (iv) compounds of formula (1), in which one or more oxygen atoms (or, e.g., all oxygen atoms) are replaced by ¹⁵O atoms, (v) compounds of formula (1), in which one or more bromine atoms (or, e.g., all bromine atoms) are replaced by ⁷⁶Br atoms, (vi) compounds of formula (1), in which one or more bromine atoms (or, e.g., all bromine atoms) are replaced by ⁷⁷Br atoms, (vii) compounds of formula (1), in which one or more iodine atoms (or, e.g., all iodine atoms) are replaced by ¹²⁰I atoms, and (viii) compounds of formula (1), in which one or more iodine atoms (or, e.g., all iodine atoms) are replaced by ¹²⁴I atoms. In general, it is preferred that none of the atoms in the compounds of formula (1) are replaced by specific isotopes.

Pharmaceutically acceptable prodrugs of the compounds provided herein, particularly the compounds of formula (1), are derivatives which have chemically or metabolically cleavable groups and become, by solvolysis or under physiological conditions, the compounds of the invention which are pharmaceutically active *in vivo.* Prodrugs of the compounds according to the the present invention may be formed in a conventional manner with a functional group of the compounds such as, e.g., with an amino, hydroxy or carboxy group. The prodrug form often offers advantages in terms of solubility, tissue compatibility or delayed release in a mammalian organism (see, Bundgaard, H., Design of Prodrugs, pp. 7-9, 21-24, Elsevier, Amsterdam 1985). Prodrugs include acid derivatives, such as, e.g., esters prepared by reaction of the parent acidic compound with a suitable alcohol, or amides prepared by reaction of the parent acid compound with a suitable amine. If a compound of the present invention has a carboxyl group, an ester derivative prepared by reacting the carboxyl group with a suitable alcohol or an amide derivative prepared by reacting the carboxyl group with a suitable amine is exemplified as a prodrug. An especially preferred ester derivative as a prodrug is methylester, ethylester, n-propylester, isopropylester, n-butylester, isobutylester, tert-butylester, morpholinoethylester, N,N-diethylglycolamidoester or α-acetoxyethylester. If a compound of the present invention has a hydroxy group, an acyloxy derivative prepared by reacting the hydroxyl group with a suitable acylhalide or a suitable acid anhydride is exemplified as a prodrug. An especially preferred acyloxy derivative as a prodrug is -OC(=O)-CH₃, -OC(=O)-C₂H₅, -OC(=O)-(tert-Bu), -OC(=O)-C₁₁H₃₁, -OC(=O)-(m-COONa-Ph), -OC(=O)-CH₂CH₂COONa, -O(C=O)-CH(NH₂)CH₃ or -OC(=O)-CH₂-N(CH₃)₂. If a compound of the present invention has an amino group, an amide derivative prepared by reacting the amino group with a suitable acid halide or a suitable mixed anhydride is exemplified as a prodrug. An especially preferred amide derivative as a prodrug is -NHC(=O)-(CH₂)₂OCH₃ or - NHC(=O)-CH(NH₂)CH₃.

In further preferred embodiments, the present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease, wherein said antagonist/inhibitor is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional repressor domain and an epigenetic modification domain capable of repressing the expression of NPBWR1.

CRISPR/Cas systems are known in the art and can be used to target the CRISPR-Cas endonuclease to a very specific target site of interest in a highly specific manner by a specifically designed guide RNA. In the present case, the specific target sequence is a genomic sequence of the NBPWR1 locus.

The human NBPWR1 gene is located on chromosome 8 forward strand, from base pair 52,939,182 to base pair 52,943,734 (Genome Reference Consortium - Human GRCh38/h38). The genomic locus of the NBPWR1 gene is known in the art and can, e.g., be retrieved under the following accession number: ENSG00000288611.

Its cytogenetic location is at the cell membrane.

While CRISPR/Cas systems are often used to cleave the genomic (target) DNA by the nuclease activity of the site-directed Cas endonuclease, a cleavage is not envisaged and desired in the context of the present invention in connection with the use of the antagonist/inhibitor of neuropeptide B/W receptor (NPBWR1). Instead, as will be described in more detail further below, in the context of the present invention, it is desired that the CRISPR/Cas system is modified to lack its nuclease activity and said Cas-protein is fused to an effector domain selected from the group consisting of a transcriptional repressor domain and an epigenetic modification domain capable of repressing the expression of NPBWR1. Accordingly, while the CRISPR/Cas system as used in the context of the present invention utilizes the capability of the CRISPR/Cas system to target the CRISPR-Cas endonuclease to a very specific target site of interest in a highly specific manner by a specifically designed guide RNA, a cleavage of the genomic target is not desired. Instead, the CRISPR/Cas system as used in the context of the present invention utilizes the capability of the CRISPR/Cas system to target/to bind to the CRISPR-Cas endonuclease to a very specific target site of interest in a highly specific manner by a specifically designed guide RNA wherein the CRISPR/Cas system is modified to lack its nuclease activity. The CRISPR/Cas system is then used to exert its function at the specific genomic target (i.e., the CRISPR/Cas system is specifically designed in the present case to be directed against NPBWR1) by an effector domain selected from the group consisting of a transcriptional repressor domain and an epigenetic modification domain capable of repressing the expression of NPBWR1 which is fused to the Cas-protein of the CRISPR/Cas system.

In general, in the following, the CRISPR/Cas system is described in the following while further below, the more specific use of the CRISPR/Cas9 system in the context of the present invention is described in more detail.

In general, it is known in the art that a CRISPR (Clustered Regularly Interspaced Short Palindromic Repeats) genomic locus can be found in the genomes of many prokaryotes (*e.g*., bacteria and archaea). In prokaryotes, the CRISPR locus encodes products that function as a type of immune system to help defend the prokaryotes against foreign invaders, such as virus and phage. Five types of CRISPR systems (*e.g*., Type I, Type II, Type III, Type U, and Type V) have been identified.

A CRISPR locus includes a number of short repeating sequences referred to as "repeats." The repeats can form hairpin structures and/or comprise unstructured single-stranded sequences. The repeats usually occur in clusters and frequently diverge between species. The repeats are regularly interspaced with unique intervening sequences referred to as "spacers," resulting in a repeat-spacer-repeat locus architecture. The spacers are identical to or have high homology with known foreign invader sequences. A spacer-repeat unit encodes a crisprRNA (crRNA), which is processed into a mature form of the spacer-repeat unit. A crRNA comprises a "seed" or spacer sequence that is involved in targeting a target nucleic acid (in the naturally occurring form in prokaryotes, the spacer sequence targets the foreign invader nucleic acid). A spacer sequence is located at the 5' or 3' end of the crRNA.

A CRISPR locus also comprises polynucleotide sequences encoding CRISPR Associated (Cas) genes. Cas genes encode endonucleases involved in the biogenesis and the interference stages of crRNA function in prokaryotes. Some Cas genes comprise homologous secondary and/or tertiary structures.

### Type II CRISPR Systems

crRNA biogenesis in a Type II CRISPR system in nature requires a trans-activating CRISPR RNA (tracrRNA). The tracrRNA is modified by endogenous RNaseIII, and then hybridizes to a crRNA repeat in the pre-crRNA array. Endogenous RNaselll is recruited to cleave the pre-crRNA. Cleaved crRNAs are subjected to exoribonuclease trimming to produce the mature crRNA form (*e.g*., 5' trimming). The tracrRNA remains hybridized to the crRNA, and the tracrRNA and the crRNA associate with a site-directed polypeptide (*e.g*., Cas9). The crRNA of the crRNA-tracrRNA-Cas9 complex guides the complex to a target nucleic acid to which the crRNA can hybridize. Hybridization of the crRNA to the target nucleic acid activates Cas9 for targeted nucleic acid cleavage (in case the Cas9 is enzymatically active which is not envisaged in the context of the present invention as mentioned above and as explained further below). The target nucleic acid in a Type II CRISPR system is referred to as a protospacer adjacent motif (PAM). In nature, the PAM is essential to facilitate binding of a site-directed polypeptide (e.g., Cas9) to the target nucleic acid. Type II systems are further subdivided into Type II-A (CASS4) and II-B (CASS4a). Jinek et al., Science, 337(6096):816-821 (2012) showed that the CRISPR/Cas9 system is useful for RNA-programmable genome editing, and international patent application publication number WO2013/176772 provides numerous examples and applications of the CRISPR/Cas endonuclease system for site-specific gene editing.

### Cas Genes/Polypeptides and Protospacer Adjacent Motifs

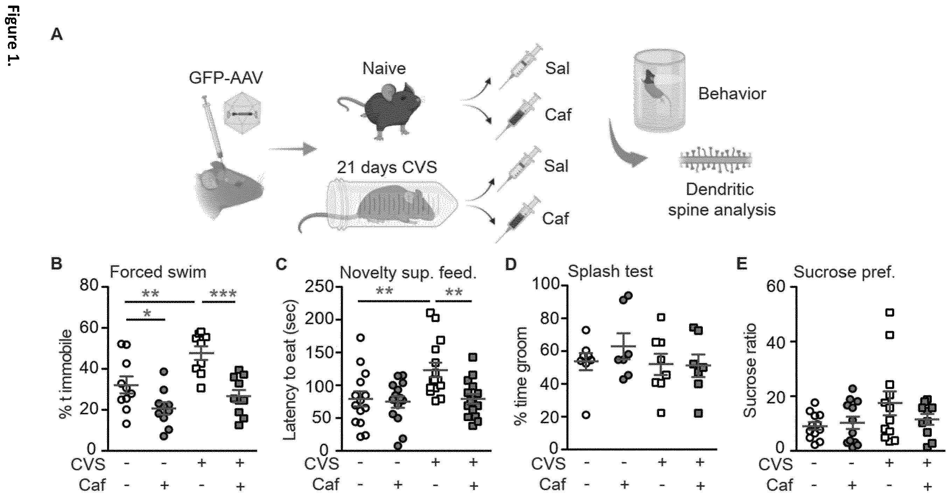

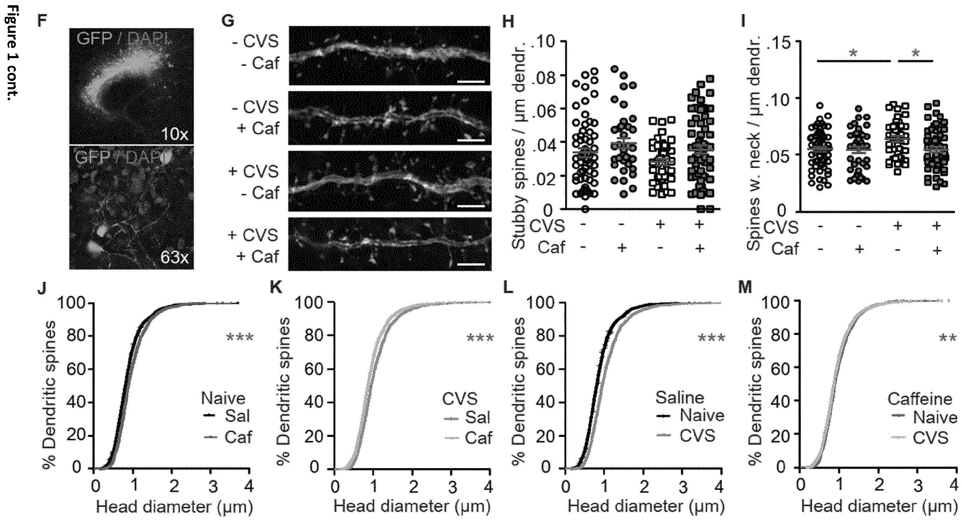

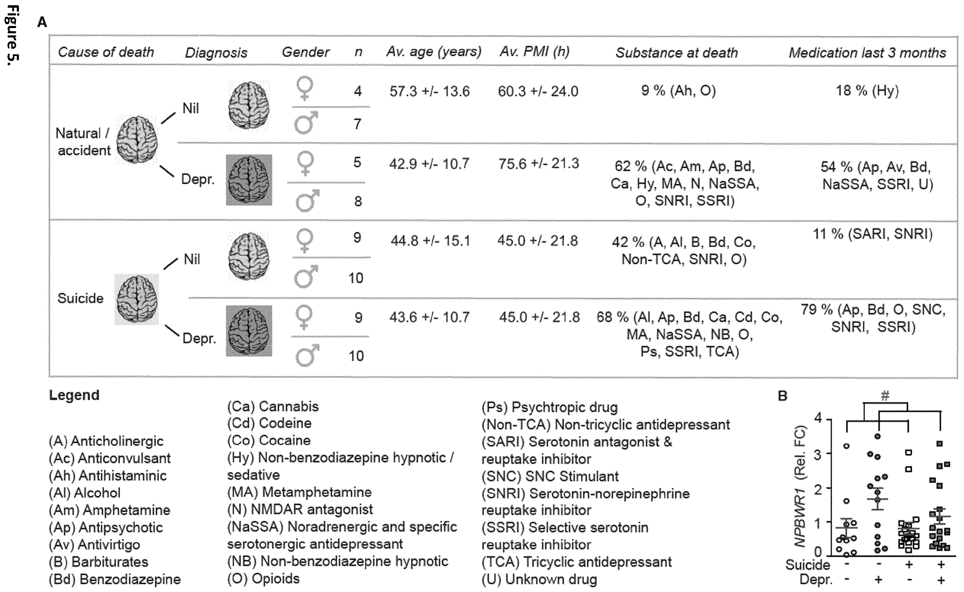

Exemplary CRISPR/Cas polypeptides include the Cas9 polypeptides in Fig. 1 of Fonfara et al., Nucleic Acids Research, 42: 2577-2590 (2014). The CRISPR/Cas gene naming system has undergone extensive rewriting since the Cas genes were discovered. Fig. 5 of Fonfara, *supra,* provides PAM sequences for the Cas9 polypeptides from various species.

### Site-Directed DNA Endonucleases

In general terms, a site-directed endonuclease is a nuclease used in genome editing to cleave DNA. The site-directed endonuclease may be administered to a cell or a patient as either: one or more polypeptides, or one or more mRNAs encoding the polypeptide.

In the context of a CRISPR/Cas system, the site-directed DNA endonuclease can bind to a guide RNA that, in turn, specifies the site in the target DNA to which the polypeptide is directed.

It is generally known that a DNA endonuclease comprises a plurality of nucleic acid-cleaving (*i.e*., nuclease) domains. In fact, naturally-occurring wild-type Cas9 enzymes are known to comprise two nuclease domains, a HNH nuclease domain and a RuvC domain. Herein, the "Cas9" refers to both naturally-occurring and recombinant Cas9s. Cas9 enzymes contemplated herein comprises a HNH or HNH-like nuclease domain, and/or a RuvC or RuvC-like nuclease domain. HNH or HNH-like domains comprise a McrA-like fold. HNH or HNH-like domains comprises two antiparallel β-strands and an α-helix. HNH or HNH-like domains comprises a metal binding site (*e.g*., a divalent cation binding site). HNH or HNH-like domains can cleave one strand of a target nucleic acid (*e.g*., the complementary strand of the crRNA targeted strand). RuvC or RuvC-like domains comprise an RNaseH or RNaseH-like fold. RuvC/RNaseH domains are involved in a diverse set of nucleic acid-based functions including acting on both RNA and DNA. The RNaseH domain comprises 5 β-strands surrounded by a plurality of α-helices. RuvC/RNaseH or RuvC/RNaseH-like domains comprise a metal binding site (*e.g*., a divalent cation binding site). RuvC/RNaseH or RuvC/RNaseH-like domains can cleave one strand of a target nucleic acid (*e.g*., the non-complementary strand of a double-stranded target DNA).

In general, it is known that DNA endonucleases can introduce double-strand breaks (or single-strand breaks) in nucleic acids, *e.g*., genomic DNA. The double-strand break can stimulate a cell's endogenous DNA-repair pathways (*e.g*., homology-dependent repair (HDR) or non-homologous end joining (NHEJ) or alternative non-homologous end joining (A-NHEJ) or microhomology-mediated end joining (MMEJ)). NHEJ can repair cleaved target nucleic acid without the need for a homologous template. This can sometimes result in small deletions or insertions (indels) in the target nucleic acid at the site of cleavage, and can lead to disruption or alteration of gene expression.

In some embodiments of the present invention, the DNA endonuclease, preferably the Cas (more preferably the Cas9) enzyme, comprises a nucleotide sequence that encodes an amino acid sequence having at least 10%, at least 15%, at least 20%, at least 30%, at least 40%, at least 50%, at least 60%, at least 70%, at least 75%, at least 80%, at least 85%, at least 90%, at least 95%, at least 99%, or 100% amino acid sequence identity to a wild-type exemplary site-directed polypeptide [*e.g.*, Cas9 from S. *pyogenes*, US2014/0068797 Sequence ID No. 8 or Sapranauskas et al., Nucleic Acids Res, 39(21): 9275-9282 (2011)], and various other site-directed polypeptides).

In some embodiments, the DNA endonuclease, preferably the Cas (more preferably the Cas9) enzyme, comprises a nucleotide sequence that encodes an amino acid sequence having at least 10%, at least 15%, at least 20%, at least 30%, at least 40%, at least 50%, at least 60%, at least 70%, at least 75%, at least 80%, at least 85%, at least 90%, at least 95%, at least 99%, or 100% amino acid sequence identity to the nuclease domain of a wild-type exemplary site-directed polypeptide (*e.g.,* Cas9 from S. *pyogenes, supra).*

In some embodiments, the DNA endonuclease, preferably the Cas (more preferably the Cas9) protein/enzyme, comprises a nucleotide sequence that encodes an amino acid sequence at least 70, 75, 80, 85, 90, 95, 97, 99, or 100% identity to a wild-type site-directed polypeptide (*e.g.,* Cas9 from *S*. *pyogenes, supra)* over 10 contiguous amino acids. In some embodiments, the DNA endonuclease, preferably the Cas (more preferably the Cas9) enzyme, comprises a nucleotide sequence that encodes an amino acid sequence comprises at most: 70, 75, 80, 85, 90, 95, 97, 99, or 100% identity to a wild-type site-directed polypeptide (*e.g*., Cas9 from S. *pyogenes, supra)* over 10 contiguous amino acids. In some embodiments, the DNA endonuclease, preferably the Cas (more preferably the Cas9) enzyme, comprises a nucleotide sequence that encodes an amino acid sequence at least: 70, 75, 80, 85, 90, 95, 97, 99, or 100% identity to a wild-type site-directed polypeptide (*e.g*., Cas9 from *S*. *pyogenes, supra)* over 10 contiguous amino acids in a HNH nuclease domain of the encoded site-directed polypeptide. In some embodiments, the DNA endonuclease, preferably the Cas (more preferably the Cas9) enzyme, comprises a nucleotide sequence that encodes an amino acid sequence at most: 70, 75, 80, 85, 90, 95, 97, 99, or 100% identity to a wild-type site-directed polypeptide (*e.g*., Cas9 from *S*. *pyogenes, supra)* over 10 contiguous amino acids in a HNH nuclease domain of the encoded site-directed polypeptide. In some embodiments, the DNA endonuclease, preferably the Cas (more preferably the Cas9) enzyme, comprises a nucleotide sequence that encodes an amino acid sequence at least: 70, 75, 80, 85, 90, 95, 97, 99, or 100% identity to a wild-type site-directed polypeptide (*e.g*., Cas9 from *S*. *pyogenes, supra)* over 10 contiguous amino acids in a RuvC nuclease domain of the encoded site-directed polypeptide. In some embodiments, the DNA endonuclease, preferably the Cas (more preferably the Cas9) enzyme, comprises a nucleotide sequence that encodes an amino acid sequence at most: 70, 75, 80, 85, 90, 95, 97, 99, or 100% identity to a wild-type site-directed polypeptide (*e.g*., Cas9 from *S*. *pyogenes, supra*) over 10 contiguous amino acids in a RuvC nuclease domain of the encoded site-directed polypeptide.

As already mentioned above, in the present invention, the DNA endonuclease, preferably the Cas (more preferably the Cas9) protein/enzyme, "is modified to lack its nuclease activity". This means that the DNA endonuclease preferably the Cas (more preferably the Cas9) protein/enzyme, encodes a site-directed polypeptide comprising a modified form of a wild-type exemplary site-directed polypeptide. The modified form of the wild-type exemplary site-directed polypeptide comprises a mutation that reduces the nucleic acid-cleaving activity of the site-directed polypeptide. In some embodiments, the modified form of the wild-type exemplary site-directed polypeptide has less than 90%, less than 80%, less than 70%, less than 60%, less than 50%, less than 40%, less than 30%, less than 20%, less than 10%, less than 5%, or less than 1% of the nucleic acid-cleaving activity of the wild-type exemplary site-directed polypeptide (*e.g*., Cas9 from *S*. *pyogenes, supra).* In most preferred embodiments, the DNA endonuclease preferably the Cas (more preferably the Cas9) protein/enzyme, in its modified form of the site-directed polypeptide, can have no substantial nucleic acid-cleaving activity. When a site-directed polypeptide is a modified form that has no substantial nucleic acid-cleaving activity, it is referred to herein as "enzymatically inactive". A corresponding Cas enzyme is the most preferred embodiment of a Cas-protein that "is modified to lack its nuclease activity" in terms of the present invention.

In some embodiments, a Cas-protein that "is modified to lack its nuclease activity" is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from S. pyogenes converts Cas9 from a nuclease that cleaves both strands to a nickase (cleaves a single strand). Other examples of mutations that render Cas9 a nickase include, without limitation, H840A, N854A, and N863A. As a further example, two or more catalytic domains of Cas9 (RuvC I, RuvC II, and RuvC III or the HNH domain) may be mutated to produce a mutated Cas9 substantially lacking all DM A cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0,1%, 0.01%, or lower with respect to its non-mutated form. Where the enzyme is not Cas9 from S. pyogenes (SpCas9), mutations may be made at any or all residues corresponding to positions 10, 762, 840, 854, 863 and/or 986 of SpCas9 (which may be ascertained for instance by standard sequence comparison tools. In particular, any or all of the following mutations are preferred in SpCas9: D10A, E762A, H840A, N854A, N863A and/or D986A; as well as conservative substitution for any of the replacement amino acids is also envisaged. The same (or conservative substitutions of these mutations) at corresponding positions in other Cas9s are also preferred. Particularly preferred are D10 and H840 in SpCas9. However, in other Cas9s, residues corresponding to SpCas9 D10 and H840 are also preferred.

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium (*e.g., S. pyogenes),* a nucleic acid binding domain, and two nucleic acid cleaving domains (*i.e*., a HNH domain and a RuvC domain).

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium *(e.g., S. pyogenes),* and two nucleic acid cleaving domains (*i.e*., a HNH domain and a RuvC domain).

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium *(e.g., S. pyogenes),* and two nucleic acid cleaving domains, wherein one or both of the nucleic acid cleaving domains comprise at least 50% amino acid identity to a nuclease domain from Cas9 from a bacterium (*e.g.*, S. *pyogenes*)*.*

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium *(e.g., S. pyogenes),* two nucleic acid cleaving domains (*i.e*., a HNH domain and a RuvC domain), and non-native sequence (for example, a nuclear localization signal) or a linker linking the site-directed polypeptide to a non-native sequence.

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium *(e.g., S. pyogenes),* two nucleic acid cleaving domains (i.e., a HNH domain and a RuvC domain), wherein the site-directed polypeptide comprises a mutation in one or both of the nucleic acid cleaving domains that reduces the cleaving activity of the nuclease domains by at least 50%.

In some embodiments, a Cas-protein that "is modified to lack its nuclease activity", preferably, the DNA endonuclease, preferably the Cas (more preferably the Cas9) protein/enzyme, i.e., the above-described site-directed polypeptide comprises an amino acid sequence comprising at least 15% amino acid identity to a Cas9 from a bacterium *(e.g., S. pyogenes),* and two nucleic acid cleaving domains (*i.e*., a HNH domain and a RuvC domain), wherein one of the nuclease domains comprises mutation of aspartic acid 10, and/or wherein one of the nuclease domains comprises mutation of histidine 840, and wherein the mutation reduces the cleaving activity of the nuclease domain(s) by at least 50%.

In some embodiments, a Cas-protein that "is modified to lack its nuclease activity", preferably, the DNA endonuclease, preferably the Cas (more preferably the Cas9) protein/enzyme, i.e., the above-described site-directed polypeptide (Cas9 protein) is from *S*. *lugdunensis* (SluCas9). In some embodiments, the Cas9 protein are from *Staphylococcus aureus* (SaCas9). In some embodiments, a suitable Cas9 protein for use in the present disclosure is any disclosed in WO2019/183150 and WO2019/118935, each of which is incorporate herein by reference.

A Type-II CRISPR/Cas system component are from a Type-IIA, Type-IIB, or Type-IIC system. Cas9 and its orthologs are encompassed. Non-limiting exemplary species that the Cas9 nuclease or other components are from include *Streptococcus pyogenes, Streptoccoccus lugdunensis, Streptococcus thermophilus, Streptococcus sp., Staphylococcus aureus, Listeria innocua, Lactobacillus gasseri, Francisella novicida, Wolinella succinogenes, Sutterella wadsworthensis, Gamma proteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium crobaticum, Ammonifex degensii, Caldicelulosiruptor becscii, Candidatus Desulforudis, Clostridium botulinum, Clostridium difficile, Finegoldic magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus holophilus, Nitrosococcus wai-soni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria,* or *Acaryochloris marina.* In some embodiments, the Cas9 protein are from *Streptococcus pyogenes* (SpCas9). In some embodiments, the Cas9 protein is from *S*. *lugdunensis* (SluCas9). In some embodiments, the Cas9 protein are from *Staphylococcus aureus* (SaCas9). In some embodiments, a suitable Cas9 protein for use in the present disclosure is any disclosed in WO2019/183150 and WO2019/118935, each of which is incorporate herein by reference.

### Guide RNAs

A guide RNA (or 'gRNA") comprises at least a spacer sequence that hybridizes to a target nucleic acid sequence of interest, and a CRISPR repeat sequence. In Type II systems, the gRNA also comprises a tracrRNA sequence. In the Type II guide RNA, the CRISPR repeat sequence and tracrRNA sequence hybridize to each other to form a duplex. In the Type V guide RNA, the crRNA forms a duplex. In both systems, the duplex binds a site-directed polypeptide, such that the guide RNA and site-direct polypeptide form a complex. The guide RNA provides target specificity to the complex by virtue of its association with the site-directed polypeptide. The guide RNA thus directs the activity of the site-directed polypeptide, i.e., in the present invention, a Cas-protein that "is modified to lack its nuclease activity", preferably, the DNA endonuclease, preferably the Cas (more preferably the Cas9) protein/enzyme as described herein-above.

In some embodiments, the guide RNA is double-stranded. The first strand comprises in the 5' to 3' direction, an optional spacer extension sequence, a spacer sequence and a minimum CRISPR repeat sequence. The second strand comprises a minimum tracrRNA sequence (complementary to the minimum CRISPR repeat sequence), a 3' tracrRNA sequence and an optional tracrRNA extension sequence.

In some embodiments, the guide RNA is single-stranded guide. A single-molecule guide RNA in a Type II system comprises, in the 5' to 3' direction, an optional spacer extension sequence, a spacer sequence, a minimum CRISPR repeat sequence, a single-stranded guide linker, a minimum tracrRNA sequence, a 3' tracrRNA sequence and an optional tracrRNA extension sequence. The optional tracrRNA extension may comprise elements that contribute additional functionality (*e.g*., stability) to the guide RNA. The single-stranded guide linker links the minimum CRISPR repeat and the minimum tracrRNA sequence to form a hairpin structure. The optional tracrRNA extension comprises one or more hairpins.

By way of illustration, guide RNAs used in the CRISPR/Cas system, or other smaller RNAs can be readily synthesized by chemical means, as described in the art. While chemical synthetic procedures are continually expanding, purifications of such RNAs by procedures such as high performance liquid chromatography (HPLC, which avoids the use of gels such as PAGE) tends to become more challenging as polynucleotide lengths increase significantly beyond a hundred or so nucleotides. One approach used for generating RNAs of greater length is to produce two or more molecules that are ligated together. Much longer RNAs, such as those encoding a Cas9 endonuclease, are more readily generated enzymatically. Various types of RNA modifications can be introduced during or after chemical synthesis and/or enzymatic generation of RNAs, *e.g*., modifications that enhance stability, reduce the likelihood or degree of innate immune response, and/or enhance other attributes, as described in the art.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-WlieelerTransform (e.g. the Burrows Wheeler Aligner), ClustalW, Clustal X, BLAT, Novoalign (Novocraft Technologies; available at wwnv.novocraft.com), ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21,22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay known in the art. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

Thus, the skilled person is easily in a position to design a CRISPR/Cas system "specifically directed against NPBWR1" by designing a corresponding guide RNA by methods known in the art. Moreover, the skilled person is easily in a position to test whether the respectively designed guide RNA of the CRISPR/Cas system is "specifically directed against NPBWR1" in that it bind to a certain specific location in the NPBWR1 target.

In a preferred embodiment, without being bound by theory, the guide RNA that is "specifically directed against NPBWR1" can, e.g., have one of the sequences as shown in any one of SEQ ID NOs:3 to 5.

The above-described components of the CRISPR/Cas system (which may form part of the pharmaceutical composition in accordance with the present invention) can be expressed and delivered by means and methods known in the art as briefly described in the following. Accordingly, the present disclosure provides a nucleic acid comprising a nucleotide sequence encoding one or more guide RNAs, and one or more a DNA endonucleases.

In some embodiments, the nucleic acid encoding one or more guide RNAs and a DNA endonuclease comprises a vector (*e.g*., a recombinant expression vector). The term "vector" refers to a nucleic acid molecule capable of transporting another nucleic acid to which it has been linked. One type of vector is a "plasmid", which refers to a circular double-stranded DNA loop into which additional nucleic acid segments can be ligated. Another type of vector is a viral vector, wherein additional nucleic acid segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome.

In some embodiments, vectors are capable of directing the expression of nucleic acids to which they are operatively linked. Such vectors are referred to herein as "recombinant expression vectors", or more simply "expression vectors", which serve equivalent functions. The term "operably linked" means that the nucleotide sequence of interest is linked to regulatory sequence(s) in a manner that allows for expression of the nucleotide sequence. The term "regulatory sequence" is intended to include, for example, promoters, enhancers and other expression control elements (*e.g*., polyadenylation signals). Such regulatory sequences are well known in the art and are described, for example, in Goeddel; Gene Expression Technology: Methods in Enzymology 185, Academic Press, San Diego, CA (1990). Regulatory sequences include those that direct constitutive expression of a nucleotide sequence in many types of host cells, and those that direct expression of the nucleotide sequence only in certain host cells (*e.g*., tissue-specific regulatory sequences). It will be appreciated by those skilled in the art that the design of the expression vector can depend on such factors as the choice of the target cell, the level of expression desired, and the like.

Expression vectors contemplated include, but are not limited to, viral vectors based on vaccinia virus, poliovirus, adenovirus, adeno-associated virus, SV40, herpes simplex virus, human immunodeficiency virus, retrovirus (*e.g*., Murine Leukemia Virus, spleen necrosis virus, and vectors derived from retroviruses such as Rous Sarcoma Virus, Harvey Sarcoma Virus, avian leukosis virus, a lentivirus, human immunodeficiency virus, myeloproliferative sarcoma virus, and mammary tumor virus) and other recombinant vectors. Other vectors contemplated for eukaryotic target cells include, but are not limited to, the vectors pXT1, pSG5, pSVK3, pBPV, pMSG, and pSVLSV40 (Pharmacia). Other vectors may be used so long as they are compatible with the host cell.

In some embodiments, a vector comprises one or more transcription and/or translation control elements. Depending on the host/vector system utilized, any of a number of suitable transcription and translation control elements, including constitutive and inducible promoters, transcription enhancer elements, transcription terminators, etc. may be used in the expression vector. In some embodiments, the vector is a self-inactivating vector that either inactivates the viral sequences or the components of the CRISPR machinery or other elements.

Non-limiting examples of suitable eukaryotic promoters (*i.e.*, promoters functional in a eukaryotic cell) include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-actin promoter (CAG), murine stem cell virus promoter (MSCV), phosphoglycerate kinase-1 locus promoter (PGK), and mouse metallothionein-I.

For expressing small RNAs, including guide RNAs used in connection with Cas endonuclease, various promoters such as RNA polymerase III promoters, including for example U6 and H1, can be advantageous. Descriptions of and parameters for enhancing the use of such promoters are known in art, and additional information and approaches are regularly being described; see, *e.g.,* Ma, H. et al., Molecular Therapy - Nucleic Acids 3, e161 (2014) doi:10.1038/mtna.2014.12.

The expression vector may also contain a ribosome binding site for translation initiation and a transcription terminator. The expression vector may also include appropriate sequences for amplifying expression. The expression vector may also include nucleotide sequences encoding non-native tags (*e.g*., histidine tag, hemagglutinin tag, green fluorescent protein, etc.) that are fused to the site-directed polypeptide, thus resulting in a fusion protein.

In some embodiments, a promoter is an inducible promoter (*e.g*., a heat shock promoter, tetracycline-regulated promoter, steroid-regulated promoter, metal-regulated promoter, estrogen receptor-regulated promoter, etc.). In some embodiments, a promoter is a constitutive promoter (*e.g*., CMV promoter, UBC promoter). In some embodiments, the promoter is a spatially restricted and/or temporally restricted promoter (*e.g*., a tissue specific promoter, a cell type specific promoter, etc.).

In some embodiments, the nucleic acid encoding one or more guide RNAs and/or DNA endonuclease are packaged into or on the surface of delivery vehicles for delivery to cells. Delivery vehicles contemplated include, but are not limited to, nanospheres, liposomes, quantum dots, nanoparticles, polyethylene glycol particles, hydrogels, and micelles. A variety of targeting moieties can be used to enhance the preferential interaction of such vehicles with desired cell types or locations.

Introduction of the complexes, polypeptides, and nucleic acids of the disclosure into cells can occur by viral or bacteriophage infection, transfection, conjugation, protoplast fusion, lipofection, electroporation, nucleofection, calcium phosphate precipitation, polyethyleneimine (PEI)-mediated transfection, DEAE-dextran mediated transfection, liposome-mediated transfection, particle gun technology, calcium phosphate precipitation, direct micro-injection, nanoparticle-mediated nucleic acid delivery, and the like.

### Delivery

Guide RNA polynucleotides (RNA or DNA) and/or endonuclease polynucleotide(s) (RNA or DNA) can be delivered by viral or non-viral delivery vehicles known in the art. Alternatively, endonuclease polypeptide(s) may be delivered by non-viral delivery vehicles known in the art, such as electroporation or lipid nanoparticles. In further alternative embodiments, the DNA endonuclease may be delivered as one or more polypeptides, either alone or pre-complexed with one or more guide RNAs.

Polynucleotides may be delivered by non-viral delivery vehicles including, but not limited to, nanoparticles, liposomes, ribonucleoproteins, positively charged peptides, small molecule RNA-conjugates, aptamer-RNA chimeras, and RNA-fusion protein complexes. Some exemplary non-viral delivery vehicles are described in Peer and Lieberman, Gene Therapy, 18: 1127-1133 (2011) (which focuses on non-viral delivery vehicles for siRNA that are also useful for delivery of other polynucleotides).

Polynucleotides, such as guide RNA, sgRNA, and mRNA encoding an endonuclease, may be delivered to a cell or a patient by a lipid nanoparticle (LNP).

A LNP refers to any particle having a diameter of less than 1000 nm, 500 nm, 250 nm, 200 nm, 150 nm, 100 nm, 75 nm, 50 nm, or 25 nm. Alternatively, a nanoparticle may range in size from 1-1000 nm, 1-500 nm, 1-250 nm, 25-200 nm, 25-100 nm, 35-75 nm, or 25-60 nm.

LNPs may be made from cationic, anionic, or neutral lipids. Neutral lipids, such as the fusogenic phospholipid DOPE or the membrane component cholesterol, may be included in LNPs as 'helper lipids' to enhance transfection activity and nanoparticle stability. Limitations of cationic lipids include low efficacy owing to poor stability and rapid clearance, as well as the generation of inflammatory or anti-inflammatory responses.

LNPs may also be comprised of hydrophobic lipids, hydrophilic lipids, or both hydrophobic and hydrophilic lipids.

Any lipid or combination of lipids that are known in the art may be used to produce a LNP. Examples of lipids used to produce LNPs are: DOTMA, DOSPA, DOTAP, DMRIE, DC-cholesterol, DOTAP-cholesterol, GAP-DMORIE-DPyPE, and GL67A-DOPE-DMPE-polyethylene glycol (PEG). Examples of cationic lipids are: 98N12-5, C12-200, DLin-KC2-DMA (KC2), DLin-MC3-DMA (MC3), XTC, MD1, and 7C1. Examples of neutral lipids are: DPSC, DPPC, POPC, DOPE, and SM. Examples of PEG-modified lipids are: PEG-DMG, PEG-CerC14, and PEG-CerC20.

The lipids may be combined in any number of molar ratios to produce a LNP. In addition, the polynucleotide(s) may be combined with lipid(s) in a wide range of molar ratios to produce a LNP.

As stated previously, the DNA endonuclease and guide RNA may each be administered separately to a cell or a patient. On the other hand, the DNA endonuclease may be pre-complexed with one or more guide RNAs. The pre-complexed material may then be administered to a cell or a patient. Such pre-complexed material is known as a ribonucleoprotein particle (RNP).

RNA is capable of forming specific interactions with RNA or DNA. While this property is exploited in many biological processes, it also comes with the risk of promiscuous interactions in a nucleic acid-rich cellular environment. One solution to this problem is the formation of ribonucleoprotein particles (RNPs), in which the RNA is pre-complexed with an endonuclease. Another benefit of the RNP is protection of the RNA from degradation.

The DNA endonuclease in the RNP may be modified or unmodified. Likewise, the gRNA may be modified or unmodified. Numerous modifications are known in the art and may be used. The DNA endonuclease and gRNA can be generally combined in a 1:1 molar ratio. However, a wide range of molar ratios may be used to produce a RNP.

In some embodiments, an AAV vector is used for delivery. Exemplary AAV serotypes include, but are not limited to, AAV-1, AAV-2, AAV-3, AAV-4, AAV-5, AAV-6, AAV-7, AAV-8, AAV-9, AAV-10, AAV-11, AAV-12, AAV-13 and AAV rh.74. See also **Table 1.**

**Table 1**

| AAV Serotype | Genbank Accession No. |
|---|---|
| AAV-1 | NC_002077.1 |
| AAV-2 | NC_001401.2 |
| AAV-3 | NC_001729.1 |
| AAV-3 B | AF028705.1 |
| AAV-4 | NC_001829.1 |
| AAV-5 | NC_006152.1 |
| AAV-6 | AF028704.1 |
| AAV-7 | NC_006260.1 |
| AAV-8 | NC_006261.1 |
| AAV-9 | AX753250.1 |
| AAV-10 | AY631965.1 |
| AAV-11 | AY631966.1 |
| AAV-12 | DQ813647.1 |
| AAV-13 | EU285562.1 |

A method of generating a packaging cell involves creating a cell line that stably expresses all of the necessary components for AAV particle production. For example, a plasmid (or multiple plasmids) comprising a rAAV genome lacking AAV rep and cap genes, AAV rep and cap genes separate from the rAAV genome, and a selectable marker, such as a neomycin resistance gene, are integrated into the genome of a cell. AAV genomes have been introduced into bacterial plasmids by procedures such as GC tailing (Samulski et al., 1982, Proc. Natl. Acad. S6. USA, 79:2077-2081), addition of synthetic linkers containing restriction endonuclease cleavage sites (Laughlin et al., 1983, Gene, 23:65-73) or by direct, blunt-end ligation (Senapathy & Carter, 1984, J. Biol. Chem., 259:4661-4666). The packaging cell line is then infected with a helper virus, such as adenovirus. The advantages of this method are that the cells are selectable and are suitable for large-scale production of rAAV. Other examples of suitable methods employ adenovirus or baculovirus, rather than plasmids, to introduce rAAV genomes and/or rep and cap genes into packaging cells.

General principles of rAAV production are reviewed in, for example, Carter, 1992, Current Opinions in Biotechnology, 1533-539; and Muzyczka, 1992, Curr. Topics in Microbial. and Immunol., 158:97-129). Various approaches are described in Ratschin et al., Mol. Cell. Biol. 4:2072 (1984); Hermonat et al., Proc. Natl. Acad. Sci. USA, 81:6466 (1984); Tratschin et al., Mo1. Cell. Biol. 5:3251 (1985); McLaughlin et al., J. Virol., 62:1963 (1988); and Lebkowski et al., 1988 Mol. Cell. Biol., 7:349 (1988). Samulski et al. (1989, J. Virol., 63:3822-3828); U.S. Patent No. 5,173,414; WO 95/13365 and corresponding U.S. Patent No. 5,658.776 ; WO 95/13392; WO 96/17947; PCT/US98/18600; WO 97/09441 (PCT/US96/14423); WO 97/08298 (PCT/US96/13872); WO 97/21825 (PCT/US96/20777); WO 97/06243 (PCT/FR96/01064); WO 99/11764; Perrin et al. (1995) Vaccine 13:1244-1250; Paul et al. (1993) Human Gene Therapy 4:609-615; Clark et al. (1996) Gene Therapy 3:1124-1132; U.S. Patent. No. 5,786,211; U.S. Patent No. 5,871,982; and U.S. Patent. No. 6,258,595.

In addition to adeno-associated viral vectors, other viral vectors may be used in the practice of the invention. Such viral vectors include, but are not limited to, lentivirus, alphavirus, enterovirus, pestivirus, baculovirus, herpesvirus, Epstein Barr virus, papovavirusr, poxvirus, vaccinia virus, and herpes simplex virus.

Options are available to deliver the Cas9 nuclease as a DNA plasmid, as mRNA or as a protein. The guide RNA can be expressed from the same DNA, or can also be delivered as an RNA. The RNA can be chemically modified to alter or improve its half-life, or decrease the likelihood or degree of immune response. The endonuclease protein can be complexed with the gRNA prior to delivery. Viral vectors allow efficient delivery; split versions of Cas9 and smaller orthologs of Cas9 can be packaged in AAV, as can donors for HDR. A range of non-viral delivery methods also exist that can deliver each of these components, or non-viral and viral methods can be employed in tandem. For example, nano-particles can be used to deliver the protein and guide RNA, while AAV can be used to deliver a donor DNA.

As outlined above, the present invention relates to pharmaceutical compositions comprising an antagonist/inhibitor of NPBWR1 for use in the treatment, amelioration and/or prevention of certain diseases. Thus, the present invention is directed to the use of the pharmaceutical compositions comprising an antagonist/inhibitor of NPBWR1 in medical settings.

This also particularly applies to the CRISPR/Cas system. Accordingly, in this context, the guide RNA(s) of the invention can be formulated with pharmaceutically acceptable excipients such as carriers, solvents, stabilizers, adjuvants, diluents, etc., depending upon the particular mode of administration and dosage form. Guide RNA compositions are generally formulated to achieve a physiologically compatible pH, and range from a pH of about 3 to a pH of about 11, about pH 3 to about pH 7, depending on the formulation and route of administration. In alternative embodiments, the pH is adjusted to a range from about pH 5.0 to about pH 8. In some embodiments, the compositions comprise a therapeutically effective amount of at least one compound as described herein, together with one or more pharmaceutically acceptable excipients. Optionally, the compositions comprise a combination of the compounds described herein, or may include a second active ingredient useful in the treatment or prevention of bacterial growth (for example and without limitation, anti-bacterial or anti-microbial agents), or may include a combination of reagents of the invention.

Suitable excipients include, for example, carrier molecules that include large, slowly metabolized macromolecules such as proteins, polysaccharides, polylactic acids, polyglycolic acids, polymeric amino acids, amino acid copolymers, and inactive virus particles. Other exemplary excipients include antioxidants (for example and without limitation, ascorbic acid), chelating agents (for example and without limitation, EDTA), carbohydrates (for example and without limitation, dextrin, hydroxyalkylcellulose, and hydroxyalkylmethylcellulose), stearic acid, liquids (for example and without limitation, oils, water, saline, glycerol and ethanol), wetting or emulsifying agents, pH buffering substances, and the like.

"Administered" refers to the delivery of a composition described herein comprising the guide ribonucleic acid (gRNAs) and the one or more DNA endonucleases (or a vector comprising a polynucleotide that encodes the gRNA and the one or more DNA endonucleases) into a subject by a method or route that results in at least partial localization of the composition at a desired site. A composition can be administered by any appropriate route that results in effective treatment in the subject, *i.e*., administration results in delivery to a desired location in the subject where at least a portion of the composition delivered, are delivered to the desired site for a period of time. Modes of administration include injection, infusion, instillation, or ingestion. "Injection" includes, without limitation, intravenous, intramuscular, intra-arterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the route is intravenous. For the delivery of cells, administration by injection or infusion is generally preferred.

In some aspects, the invention provides methods which may comprise delivering one or more polynucleotides, such as or one or more vectors as described herein, one or more transcripts thereof, and/or one or proteins transcribed therefrom, to a host cell. In some aspects, the invention further provides cells produced by such methods, and animals which may comprise or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR. system to cells in culture, or in a host organism, Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808813 (1992); Nabel & Feigner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10): 1149-1154 (1988); Vigne, Restorative Neurology and Neuroscience 8:35-36 (1995); Kremer & Perricaudet, British Medical Bulletin 51 (1):31 -44 (1995); Haddada et al., in Current Topics in Microbiology and Immunology Doerfler and Bohm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid nucleic acid conjugates, naked DNA, artificial virions, and agent-enhanced uptake of DMA. Lipofection is described in e.g., U.S. Pat. Nos. 5,049,386, 4,946,787; and 4,897,355) and lipofection reagents are sold commercially (e.g., Transfectam^{™} and Lipofectin^{™}), Cationic and neutral lipids that are suitable for efficient receptor-recognition lipofection of polynucleotides include those of Feigner, WO 91/17424; WO 91/16024. Delivery can be to cells (e.g. in vitro or ex vivo administration) or target tissues (e.g. in vivo administration).

The preparation of lipid nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et ah, Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (*in vivo*) or they can be used to treat cells *in vitro,* and the modified cells may optionally be administered to patients (*ex vivo*)*.* Conventional viral based systems could include retroviral, lentivirus, adenoviral, adeno-associated and herpes simplex vims vectors for gene transfer.

Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency vims (SIV), human immuno deficiency vims (HIV), and combinations thereof (see, e.g., Buchscher et al., J. Virol. 66:2731-2739 (1992); Johann et al., J. Virol. 66:1635-1640 (1992); Sommnerfelt et al, Virol. 176:58-59 (1990); Wilson et al., J. Virol. 63:2374-2378 (1989); Miller et al, J. Virol. 65:2220-2224 (1991); PCT/US94/05700).

As mentioned above, the present invention relates to a pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease, wherein said antagonist/inhibitor is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional repressor domain and an epigenetic modification domain capable of repressing the expression of NPBWR1.

Thus, in accordance with the present invention, the Cas-protein is fused to an effector domain selected from the group consisting of a transcriptional repressor domain and an epigenetic modification domain capable of repressing the expression of NPBWR1.

Accordingly, the CRISPR enzyme, preferably the Cas9 protein, is part of a fusion protein which may comprise one or more heterologous protein domains (e.g., about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains.

A heterologous protein domain in terms of the present invention is a transcriptional repressor domain or an epigenetic modification domain capable of repressing the expression of NPBWR1.

Corresponding examples of protein domains that may be fused to a CRISPR enzyme are described in the following.

As explained above, the aim of the transcriptional repressor domain or an epigenetic modification domain capable of repressing the expression of NPBWR1 which is fused to the Cas-protein is to modulate transcription of a target nucleic acid, i.e., the transcription of the NPBWR1 gene by decreasing (preferably completely inhibiting) its transcription.

As outlined, this generally involves contacting the target nucleic acid with an enzymatically inactive Cas9 polypeptide and a guide RNA.

A corresponding transcriptional modulation finds use therapeutic applications in terms of the present invention.

In preferred embodiments, some cases, a transcription modulation, preferably the transcriptional repression, provides for selective modulation (e.g., reduction or increase (an increase is envisaged in the context of an agonistic/activating arm of the present invention described further below)) of a target nucleic acid, i.e., the NPBWR1 gene. For example, "selective" reduction of transcription of a target nucleic acid reduces transcription of the target nucleic acid by at least about 10%, at least about 20%, at least about 30%, at least about 40%, at least about 50%, at least about 60%, at least about 70%, at least about 80%, at least about 90%, or greater than 90%, compared to the level of transcription of the target nucleic acid in the absence of a DNA-targeting RNA/variant Cas9 polypeptide complex. Selective reduction of transcription of a target nucleic acid reduces transcription of the target nucleic acid, but does not substantially reduce transcription of a non-target nucleic acid, e.g., transcription of a non-target nucleic acid is reduced, if at all, by less than 10% compared to the level of transcription of the non-target nucleic acid in the absence of the DNA-targeting RNA/variant Cas9 polypeptide complex.

As outlined further below, an increased transcription is envisaged in the context of an agonistic/activating arm of the present invention described further below."Selective" increased transcription of a target DNA can increase transcription of the target DNA by at least about 1.1 fold (e.g., at least about 1.2 fold, at least about 1.3 fold, at least about 1.4 fold, at least about 1.5 fold, at least about 1.6 fold, at least about 1.7 fold, at least about 1.8 fold, at least about 1.9 fold, at least about 2 fold, at least about 2.5 fold, at least about 3 fold, at least about 3.5 fold, at least about 4 fold, at least about 4.5 fold, at least about 5 fold, at least about 6 fold, at least about 7 fold, at least about 8 fold, at least about 9 fold, at least about 10 fold, at least about 12 fold, at least about 15 fold, or at least about 20-fold) compared to the level of transcription of the target DNA in the absence of a DNA-targeting RNA/variant Cas9 polypeptide complex. Selective increase of transcription of a target DNA increases transcription of the target DNA, but does not substantially increase transcription of a non-target DNA, e.g., transcription of a non-target DNA is increased, if at all, by less than about 5-fold (e.g., less than about 4-fold, less than about 3-fold, less than about 2-fold, less than about 1.8-fold, less than about 1.6-fold, less than about 1.4-fold, less than about 1.2-fold, or less than about 1.1-fold) compared to the level of transcription of the non-targeted DNA in the absence of the DNA targeting RNA/variant Cas9 polypeptide complex.

As a non-limiting example, increased can be achieved by fusing dCas9 (dead Cas9, i.e., a Cas-protein that is "modified to lack its nuclease activity" in terms of the present invention) to a heterologous sequence. Suitable fusion partners include, but are not limited to, a polypeptide that provides an activity that indirectly increases transcription by acting directly on the target DNA or on a polypeptide (e.g., a histone or other DNA-binding protein) associated with the target DNA. Suitable fusion partners include, but are not limited to, a polypeptide that provides for methyltransferase activity, demethylase activity, acetyltransferase activity, deacetylase activity, kinase activity, phosphatase activity, ubiquitin ligase activity, deubiquitinating activity, adenylation activity, deadenylation activity, SUMOylating activity, deSUMOylating activity, ribosylation activity, deribosylation activity, myristoylation activity, or demyristoylation activity.

Additional suitable fusion partners include, but are not limited to, a polypeptide that directly provides for increased transcription of the target nucleic acid (e.g., a transcription activator or a fragment thereof, a protein or fragment thereof that recruits a transcription activator, a small molecule/drug-responsive transcription regulator, etc.).

A non-limiting example of a subject method using a dCas9 fusion protein to increase transcription in a eukaryotes includes fusion of dCas9 to an activation domain (AD) (e.g., GAL4, herpesvirus activation protein VP16 or VP64, human nuclear factor NF-kB p65 subunit, etc.). To render the system inducible, expression of the dCas9 fusion protein can be controlled by an inducible promoter (e.g., Tet-ON, Tet-OFF, etc.). The DNA-targeting RNA can be design to target known transcription response elements (e.g., promoters, enhancers, etc.), known upstream activating sequences (UAS), sequences of unknown or known function that are suspected of being able to control expression of the target DNA, etc.

### Additional fusion partners

Non-limiting examples of fusion partners to accomplish increased or decreased transcription include transcription activator and transcription repressor domains (e.g., the Kruppel associated box (KRAB or SKD); the Mad mSIN3 interaction domain (SID); the ERF repressor domain (ERD), etc). In some such cases, the dCas9 fusion protein is targeted by the DNA-targeting RNA to a specific location (i.e., sequence) in the target DNA and exerts locus-specific regulation such as blocking RNA polymerase binding to a promoter (which selectively inhibits transcription activator function), and/or modifying the local chromatin status (e.g., when a fusion sequence is used that modifies the target DNA or modifies a polypeptide associated with the target DNA). In some cases, the changes are transient (e.g., transcription repression or activation). In some cases, the changes are inheritable (e.g., when epigenetic modifications are made to the target DNA or to proteins associated with the target DNA, e.g., nucleosomal histones).

In some embodiments, the heterologous sequence can be fused to the C-terminus of the dCas9 polypeptide. In some embodiments, the heterologous sequence can be fused to the N-terminus of the dCas9 polypeptide. In some embodiments, the heterologous sequence can be fused to an internal portion (i.e., a portion other than the N- or C- terminus) of the dCas9 polypeptide.

The biological effects of a method using a subject dCas9 fusion protein can be detected by any convenient method (e.g., gene expression assays; chromatin-based assays, e.g., Chromatin immunoPrecipitation (ChiP), Chromatin in vivo Assay (CiA), etc.; and the like).

In preferred embodiments in which the effector domain of the fusion
protein is a transcriptional activation domain or a transcriptional repressor domain, the guide RNA directs the fusion protein to a specific chromosomal sequence, wherein the transcriptional activation domain or a transcriptional repressor domain activates or represses expression, respectively, of the targeted chromosomal sequence.

In alternate preferred embodiments in which the effector domain of the fusion protein is an epigenetic modification domain, the guide RNA directs the fusion protein to a specific chromosomal sequence, wherein the epigenetic modification domain modifies the structure of the targeted the chromosomal sequence. Epigenetic modifications include acetylation, methylation of histone proteins and/or nucleotide methylation. In some instances, structural modification of the chromosomal sequence leads to changes in expression of the chromosomal sequence.

In preferred embodiments, the transcriptional activation domain is, e. g. VP64. In another aspect the transcriptional repressor domain, e.g.a KRAB domain, a SID domain or a SID4X domain.

Furthermore, in line with the rationale of the present invention and the experimental evidence of the appended Examples, as also explained in the following, an increase in the activity of a functional neuropeptide B/W receptor (NPBWR1) is expected to have medical implications, in particular, in certain specific disorders or diseases.

Accordingly, the present invention not only relates to the above-described pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease but also, in a different aspect of the present invention, to a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia.

Thus, in further embodiments, the present invention also relates to a pharmaceutical composition comprising an agonist/activator of neuropeptide B/W receptor (NPBWR1) **for use in** in a method of treating, ameliorating or preventing a bipolar affective disorder (ICD-10 F31) during the manic phase, appetitive disorders, preferably anorexia or bulimia.

Bipolar affective disorders (ICD-10 F31) during the manic phase, appetitive disorders, preferably anorexia or bulimia are disorders or syndromes that are known in the art and are medical indications that are classified in the ICD-system, i.e., the well-known and established medical classification list by the World Health Organization (WHO). ICD is the International Statistical Classification of Diseases and Related Health Problems (ICD). In the following, when referring to more specifically defined disorders or diseases, reference is made to the 10^{th} revision of said the International Statistical Classification of Diseases and Related Health Problems (ICD), i.e., "ICD 10".

The present invention is not limited to a bipolar affective disorders (ICD-10 F31) during the manic phase, appetitive disorder, preferably anorexia or bulimia. Any bipolar affective disorders (ICD-10 F31) during the manic phase, appetitive disorder, preferably anorexia or bulimia can be treated, ameliorated and/or prevented by the agonist/activator of neuropeptide B/W receptor (NPBWR1) in accordance with the present invention.

Without being bound to theory, in particular preferred embodiments, the present invention relates a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein said bipolar affective disorder (ICD-10 F31) during the manic phase, appetitive disorder, preferably anorexia or bulimia is/are the following.

In a preferred embodiment, the present invention relates to a bipolar affective disorder (ICD-10 F31) during the manic phase. The term "bipolar affective disorders (ICD-10 F31)" is already described above. Accordingly, the same applies, *mutatis mutandis,* as regards the definition of this disorder or disease as has been set forth above, with the exception that the agonist/activator in terms of the present invention is to be used during the manic phase of said disease.

The terms "agonist" or "activator" are used interchangeably herein. These terms are known in the art and relates to a compound/substance capable of fully or partially activating or enhancing the physiologic activity of (a) specific protein(s). In the context of the present invention said agonist/activator, therefore, may activate or enhance the physiological activity of a protein such as neuropeptide B/W receptor (NPBWR1) upon binding of said compound/substance to said protein. Binding of an "agonist/activator" to a given protein, e.g., neuropeptide B/W receptor (NPBWR1), may enhance the binding of an endogenous activating molecules binding to said protein. As used herein, accordingly, the term "agonist" also encompasses enhancing agonists.

In addition thereto, however, an "agonist" or "activator" of neuropeptide B/W receptor (NPBWR1) in the context of the present invention may also be capable of activating or enhancing the function of a given protein, such as neuropeptide B/W receptor (NPBWR1), by activating or enhancing the expression of the nucleic acid molecule encoding for said protein. Thus, an agonist/activator of neuropeptide B/W receptor (NPBWR1) may lead to an increased expression level of neuropeptide B/W receptor (NPBWR1) (e.g. increased level of neuropeptide B/W receptor (NPBWR1) mRNA, neuropeptide B/W receptor (NPBWR1) protein) which is reflected in an increased activity of neuropeptide B/W receptor (NPBWR1). This increased activity can be measured/detected by methods known in the art and as described herein. An activator of neuropeptide B/W receptor (NPBWR1) in the context of the present invention, accordingly, may also encompass transcriptional activators of neuropeptide B/W receptor (NPBWR1) expression that are capable of increasing neuropeptide B/W receptor (NPBWR1) function. As described herein below in detail, the increased expression and/or activity of neuropeptide B/W receptor (NPBWR1) by an agonist/activator of neuropeptide B/W receptor (NPBWR1) leads to an increased activity (and/or expression) of neuropeptide B/W receptor (NPBWR1), thereby increasing functional capability of neuropeptide B/W receptor (NPBWR1).

In line with the rationale of the present invention, an increase in the activity of a functional neuropeptide B/W receptor (NPBWR1) is expected to have various medical implications.

As explained in the following, an increase in the activity of a functional neuropeptide B/W receptor (NPBWR1) is expected to have medical implications, i.e., to have an impact on a disease/disorder associated with a bipolar affective disorder (ICD-10 F31) during the manic phase, appetitive disorders, preferably anorexia or bulimia.

The term "activator" and/or "agonist" implies no specific mechanism of biological action whatsoever and is deemed to expressly include and encompass all possible pharmacological, physiological, and biochemical interactions with neuropeptide B/W receptor (NPBWR1) (signaling) whether direct or indirect. For the purpose of the present disclosure, it will be explicitly understood that the term "activator" and/or "agonist" encompasses all the previously identified terms, titles, and functional states and characteristics whereby neuropeptide B/W receptor (NPBWR1) itself, a biological activity of neuropeptide B/W receptor (NPBWR1) (including but not limited to its ability to to regulate/inhibit Bdnf expression; the capability to bind to neuropeptide B/W; and/or the capability to bind to G-protein), or the consequences of the biological activity, are substantially increased or enhanced in any meaningful degree, e.g., by at least 5%, 10%, 20%, 50%, 70%, 85%, 90%, 100%, 150%, 200%, 300%, 500%, or by 2-fold, 3-fold, 4-fold, 5-fold, 10-fold, 20-fold, 50-fold, 100-fold or 1000-fold. An activator/agonist may increase an abnormal level of a biological activity of neuropeptide B/W receptor (NPBWR1) that may cause an adverse effect and/or a disease in a subject to a level that corresponds to a level in a healthy subject thereby preventing, preventing progression and/or curing the adverse effect and/or disease.

Accordingly, the present invention relates in one aspect to an inhibitor/antagonist of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein the activator increases the biological activity of neuropeptide B/W receptor (NPBWR1) at least 2-fold, 3-fold, 4-fold, 5-fold, 10-fold, 20-fold, 50-fold, 100-fold or 1000-fold.

Accordingly, the present invention relates in one aspect to an activator/agonist of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein the activator/agonist increases an abnormal level of a biological activity of neuropeptide B/W receptor (NPBWR1) that causes/and or promotes an adverse effect and/or a disease at least 2-fold, 3-fold, 4-fold, 5-fold, 10-fold, 20-fold, 50-fold, 100-fold or 1000-fold.

Accordingly, the present invention relates in one aspect to an activator/agonist of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein the inhibitor/antagonist decreases an abnormal level of a biological activity of neuropeptide B/W receptor (NPBWR1) that causes/and or promotes an adverse effect and/or a disease at least 3-fold.

Accordingly, the present invention relates in one aspect to an activator/agonist of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein the activator/agonist increases an abnormal level of a biological activity of neuropeptide B/W receptor (NPBWR1) that causes/and or promotes a bipolar affective disorder (ICD-10 F31) during the manic phase, appetitive disorders, preferably anorexia or bulimia.

Accordingly, the present invention relates in one aspect to an activator/agonist of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein the inhibitor increases an abnormal level of a biological activity of neuropeptide B/W receptor (NPBWR1) that causes/and or promotes a bipolar affective disorder (ICD-10 F31) during the manic phase, appetitive disorders, preferably anorexia or bulimia in a subject to a level comparable to a healthy subject.

According to the present invention the term "activator/agonist of neuropeptide B/W receptor (NPBWR1)" also means a compound or substance capable of increasing or enhancing the physiologic activity of the neuropeptide B/W receptor (NPBWR1). In the context of the present invention said activator may therefore activate or enhance the physiological activity of neuropeptide B/W receptor (NPBWR1), e.g., upon binding of said compound/substance (i.e. of the activator/agonist) to neuropeptide B/W receptor (NPBWR1).

A "activator/agonist of neuropeptide B/W receptor (NPBWR1)" may also be capable of increasing or activating the function of neuropeptide B/W receptor (NPBWR1) by increasing or activating or enhancing the expression of the nucleic acid molecule encoding for neuropeptide B/W receptor (NPBWR1). Thus, an activator/agonist of neuropeptide B/W receptor (NPBWR1) may lead to an increased expression level of neuropeptide B/W receptor (NPBWR1) gene products such as a increased level of neuropeptide B/W receptor (NPBWR1) mRNA and/or neuropeptide B/W receptor (NPBWR1) protein.

An activator/agonist of neuropeptide B/W receptor (NPBWR1) may increase or enhance an abnormal expression level of neuropeptide B/W receptor (NPBWR1) that may cause and/or promote an adverse effect and/or disease/disease associated with a bipolar affective disorder (ICD-10 F31) during the manic phase, appetitive disorders, preferably anorexia or bulimia as outlined hereinabove and below to a level that corresponds to a level in a healthy subject thereby preventing, preventing progression or curing the adverse effect and/or disease/disease in a subject. This may be reflected in an increased neuropeptide B/W receptor (NPBWR1) expression and/or a increased abnormal neuropeptide B/W receptor (NPBWR1) expression thereby restoring a healthy level of neuropeptide B/W receptor (NPBWR1) expression. Neuropeptide B/W receptor (NPBWR1) expression level may to some extent correlate with neuropeptide B/W receptor (NPBWR1) activity until saturation of the translation machinery and or a substance that binds neuropeptide B/W receptor (NPBWR1) is achieved. The expression level of neuropeptide B/W receptor (NPBWR1) may be measured/detected by methods known in the art.

Accordingly, the present invention relates in one aspect to an activator/agonist of neuropeptide B/W receptor (NPBWR1) for use in the treatment, amelioration or prevention of a disorder associated with a bipolar affective disorder (ICD-10 F31) during the manic phase, appetitive disorders, preferably anorexia or bulimia in accordance with the present invention, wherein the activator increases the expression level of neuropeptide B/W receptor (NPBWR1) gene products.

Accordingly, the present invention relates in one aspect to an activator/agonist of neuropeptide B/W receptor (NPBWR1) for use in the treatment, amelioration or prevention of a disorder associated with a bipolar affective disorder (ICD-10 F31) during the manic phase, appetitive disorders, preferably anorexia or bulimia in accordance with the present invention, wherein the activator/agonist increases an abnormal expression level of neuropeptide B/W receptor (NPBWR1) gene products that causes and/or promotes an adverse effect and/or a disease/disorder.

Accordingly, the present invention relates in one aspect to an activator/agonist of neuropeptide B/W receptor (NPBWR1) for use in the treatment, amelioration or prevention of a disorder associated with a bipolar affective disorder (ICD-10 F31) during the manic phase, appetitive disorders, preferably anorexia or bulimia in accordance with the present invention, wherein the activator/agonist increases an abnormal expression level of neuropeptide B/W receptor (NPBWR1) gene products that causes and/or promotes the above a bipolar affective disorder (ICD-10 F31) during the manic phase, appetitive disorders, preferably anorexia or bulimia.

Accordingly, the present invention relates in one aspect to an activator/agonist of neuropeptide B/W receptor (NPBWR1) for use in the treatment, amelioration or prevention of a disorder associated with a bipolar affective disorder (ICD-10 F31) during the manic phase, appetitive disorders, preferably anorexia or bulimia in accordance with the present invention, wherein the activator/agonist increases an abnormal expression level of neuropeptide B/W receptor (NPBWR1) gene products that causes and/or promotes a bipolar affective disorder (ICD-10 F31) during the manic phase, appetitive disorders, preferably anorexia or bulimia in a subject to a level of expression comparable to a healthy subject.

An activator/agonist of neuropeptide B/W receptor (NPBWR1) may perform its activating function by directly interacting the neuropeptide B/W receptor (NPBWR1) protein, i.e., with any part of the neuropeptide B/W receptor (NPBWR1) protein such as the ectodomain, transmembrane domain and/or cytoplasmic domain of neuropeptide B/W receptor (NPBWR1). An activator/agonist of neuropeptide B/W receptor (NPBWR1) may also perform any inhibitory effect on neuropeptide B/W receptor (NPBWR1) function by activating or enhancing or increasing any upstream or downstream pathway components that essentially contribute to neuropeptide B/W receptor (NPBWR1) function. An activator/agonist of neuropeptide B/W receptor (NPBWR1) may perform any indirect activating effect on any neuropeptide B/W receptor (NPBWR1) activating molecules such as nucleic acids, ribonucleic acid (RNA), double-stranded ribonucleic acid (dsRNA), chromatin reader proteins and/or ligands.

Efficacy of an activator/agonist of neuropeptide B/W receptor (NPBWR1) may be described using the half-maximal inhibitory concentration (IC50) value. In the sense of the present invention, an activator/agonist of neuropeptide B/W receptor (NPBWR1) preferably embodies a high IC50 value. The IC50 value of an activator/agonist of neuropeptide B/W receptor (NPBWR1) may be higher than 100µM, higher than 90 µM, higher than 80 µM, higher than 70 µM, higher than 60 µM, higher than 50 µM, higher than 40 µM, higher than 30 µM, higher than 20 µM or higher than 10 µM, wherein higher values are preferred over lower values. Accordingly, the invention relates to an inhibitor/antagonist of neuropeptide B/W receptor (NPBWR1) for use in the treatment, amelioration or prevention of a bipolar affective disorder (ICD-10 F31) during the manic phase, appetitive disorders, preferably anorexia or bulimia in accordance with the present invention, wherein the efficacy of the half-maximal inhibitory concentration (IC50) is higherthan 100µM, higher than 90 µM, higher than 80 µM, higherthan 70 µM, higher than 60 µM, higher than 50 µM, higher than 40 µM, higher than 30 µM, higher than 20 µM or higher than 10 µM, higher than 9 µM, higher than 8 µM, higher than 7 µM, higher than 6 µM, higher than 5 µM or higher than 4 µM, preferably higher than 4µM.

The skilled person is aware how to determine the IC50 value of an activator/agonist of neuropeptide B/W receptor (NPBWR1). It is envisioned herein, that an activators/agonists of neuropeptide B/W receptor (NPBWR1) may embody additional IC50 values and/or that other activators/agonists of neuropeptide B/W receptor (NPBWR1) with other IC50 values are identified.

In preferred embodiments, the present invention relates to a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia as defined herein-above, wherein said agonist/activator is selected from an NPBWR1 activating peptide, NPBWR1 activating small binding molecule and NPBWR1 RNA molecules.

In a preferred embodiment, said agonist/activator is an NPBWR1 RNA molecule encoding NPBWR1. Therefore, the NPBWR1 RNA molecule encoding NPBWR1 is a sense-molecule to be employed in accordance with the present invention that is a nucleic acid molecule encoding neuropeptide B/W receptor (NPBWR1). Preferably said nucleic acid molecule is RNA, i.e., pre m-RNA or mRNA.

In preferred embodiments, said NPBWR1 RNA molecule encoding NPBWR1 not only comprises a coding region encoding a NPBWR1 polypeptide (preferably, including a start codon at its 5' end), but also upstream of said coding sequence a UTR.

A ribonucleic acid (RNA) molecule as used in accordance with the present invention relates to a polymeric molecule which is assembled as a chain of the nucleotides termed G, A, U, and C. Each nucleotide in RNA contains a ribose sugar, with carbons numbered 1' through 5'. A nitrogenous base is attached to the 1' position, in general, adenine (A), cytosine (C), guanine (G), or uracil (U). In a polymeric RNA molecule a phosphate group is attached to the 3' position of one ribose and the 5' position of the next. Thus, the nucleotides in a polymeric RNA molecule are covalently linked to each other wherein the phosphate group from one nucleotide binds to the 3' carbon on the subsequent nucleotide, thereby forming a phosphodiester bond. Accordingly, an RNA strand has a 5' end and a 3' end, so named for the carbons on the ribose ring. By convention, upstream and downstream relate to the 5' to 3' direction in which RNA transcription takes place. Preferably, the RNA molecule is a messenger RNA (mRNA) molecule. mRNA is a large family of RNA molecules that convey genetic information from DNA to the ribosome, where they specify the amino acid sequence of the protein products of gene expression. Following transcription of primary transcript mRNA (known as pre-mRNA) by RNA polymerase, processed, mature mRNA is translated into a polymer of amino acids: a protein, as summarized in the central dogma of molecular biology. As in DNA, mRNA genetic information is in the sequence of nucleotides, which are arranged into codons consisting of three bases each. Each codon encodes for a specific amino acid, except the stop codons, which terminate protein synthesis.

The RNA molecule may also comprise a UTR at its 3' end. Thus, the RNA molecule of the present invention resembles with respect to its structure a "normal" mRNA molecule which occurs in nature, harbouring a coding region as well as (5' and 3') untranslated regions (UTRs) and, optionally, a poly-A tail.

The term "coding region including a start codon at its 5' end" as used in accordance with the present invention relates to a sequence which is composed of codons, which are decoded and translated into protein by the ribosome in accordance with the information provided by the genetic code. Coding regions commonly begin with a start codon at their 5' end and end with a stop codon. In general, the start codon is an AUG triplet and the stop codon is UAA, UAG, or UGA. In addition to being protein-coding, portions of coding regions may serve as regulatory sequences in the pre-mRNA as exonic splicing enhancers or exonic splicing silencers. The coding region of a gene coding for a polypeptide or a protein as used in accordance with the present invention is also known as the coding sequence or CDS (from coding DNA sequence) and is that portion of a gene's DNA or RNA, composed of exons, that codes for a polypeptide or protein. The coding region in mRNA is flanked by the 5'-untranslated region (5' UTR) and the 3'-untranslated region (3' UTR) which are also parts of the exons. Moreover, mRNA molecules may further comprise a so-called 5' cap and a poly-A tail. The 5' cap, the 5' UTR, the 3' UTR and the poly-A tail are regions of an mRNA molecule which are not translated into protein.

The term "untranslated region" or "UTR" as used in accordance with the present invention relates to sections of the mRNA upstream of the start codon and downstream of the stop codon that are not translated, and are, therefore, termed the five prime untranslated region (5' UTR) and three prime untranslated region (3' UTR), respectively. These regions are transcribed with the coding region and thus are exonic as they are present in the mature mRNA.

An RNA molecule of the present invention may also contain a poly-A tail. A poly-A tail is a long sequence of adenine nucleotides (often several hundred) added to the 3' end of the pre-mRNA by a process called polyadenylation. This tail promotes export from the nucleus and translation, and protects the mRNA from degradation. Polyadenylation is the addition of a poly(A) tail to a messenger RNA. The poly(A) tail consists of multiple adenosine monophosphates; in other words, it is a stretch of RNA that has only adenine bases. In eukaryotes, polyadenylation is part of the process that produces mature messenger RNA (mRNA) for translation.

The RNA molecules of the present invention containing the above-described UTR(s) may be generated/synthesized recombinantly (e.g., in an in vivo or an in vitro system) or synthetically by methods known to the person skilled in the art.

*In vitro* transcription of RNA usually requires a linear DNA template containing a double-stranded promoter region where the DNA-dependent RNA-polymerase binds and initiates RNA synthesis while the coding region may be double-stranded or single-stranded. In case the linear DNA template contains a single-stranded coding region, the antisense strand (i.e., the strand which is read by the DNA-dependent polymerase) of the coding region is part of the template. Common DNA-dependent RNA-polymerases are the T7 polymerase, the T3 polymerase, SP6 polymerase and the K11 polymerase.

Transcription templates for an in vitro transcription include, for example, cDNA templates synthesized from an RNA precursor, templates generated by PCR, chemically synthesized oligonucleotides and plasmid constructs. Many widely used plasmid cloning vectors harbour phage polymerase promoters located on each side of the multiple cloning site to allow transcription of either strand of a nucleotide sequence inserted into the multiple cloning site. Commonly used cloning vectors include for example Invitrogen's pCRll, Promega's pGEM and Stratagene's pBluescript vectors. Ambion's pTRIPLEscript family of vectors contain all three phage polymerase promoters in tandem (on the same side of the multiple cloning site), allowing any of the three polymerases, SP6, T7 or T3 to be used.

The RNA molecules of the present invention may be produced recombinantly in in vivo systems by methods known to the person skilled in the art.

Alternatively, the RNA molecules of the present invention may be produced in an in vitro system using, for example, an in vitro transcription system. In vitro transcription systems are commonly known and usually require a purified linear DNA template containing a DNA sequence "encoding" the RNA molecule wherein said DNA sequence is under the control of an appropriate promoter. Moreover, an in vitro transcription system also commonly requires ribonucleoside triphosphates, a buffer system that includes DTT and magnesium ions, and an appropriate RNA polymerase which provides the enzymatic activity for the in vitro transcription of the DNA sequence into a corresponding RNA molecule of the present invention.

Furthermore, the RNA molecules may be chemically synthesized, e.g., by conventional chemical synthesis on an automated nucleotide sequence synthesizer using a solid-phase support and standard techniques or by chemical synthesis of the respective DNA-sequences and subsequent in vitro or in vivo transcription of the same.

In further, more preferred embodiments, the present invention relates to a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia, wherein said agonist/activator is neuropeptide B or neuropeptide W.

The Neuropeptide B is known in the art and is a short biologically active peptide whose precursor in humans is encoded by the *NBP* gene. Neuropeptide B is a ligand of and acts via its binding to two G protein-coupled receptors, neuropeptide B/W receptors (collectively referred to in the context of the present invention as neuropeptide B/W receptor (NPBWR1)), called NPBW1 and NPBW2 encoded by the genes NPBWR1 and *NPBWR2,* respectively. Neuropeptide B is described in the art to be associated with the regulation of feeding, neuroendocrine system, memory, learning and in the afferent pain pathway. It is expressed throughout the CNS with high levels in the substantia nigra, hypothalamus, hippocampus, and spinal cord.

The amino acid sequence of human neuropeptide B can be retrieved under the UniProt accession number: Q8NG41

The amino acid sequence of human neuropeptide B is as follows:

MARSATLAAAALALCLLLAPPGLAWYKPAAGHSSYSVGRAAGLLSGLRRSPYARRSQPYRGAEPPGGAG
A SPELQLHPRLRSLAVCVQDVAPNLQRCERLPDGRGTYQCKANVFLSLRAADCLAA (SEQ ID NO:6).

Neuropeptide W is a short human neuropeptide. Neuropeptide W acts as a ligand for two neuropeptide B/W receptors (collectively referred to in the context of the present invention as neuropeptide B/W receptor (NPBWR1)), NPBWR1 and NPBWR2, which are integrated in GPCRs family of alpha-helical transmembrane proteins.

The amino acid sequence of neuropeptide W can be retrieved under the UniProt accession number:. Q8N729

The amino acid sequence of the human neuropeptide W is as follows:

MAWRPGERGAPASRPRLALLLLLLLLPLPSGAWYKHVASPRYHTVGRAAGLLMGLRRSPYLWRRALRAA
AGPLARDTLSPEPAAREAPLLLPSWVQELWETRRRSSQAGIPVRAPRSPRAPEPALEPESLDFSGAGQRLR
RDVSRPAVDPAANRLGLPCLAPGPF (SEQ ID NO:7).

However, neuropeptide B and neuropeptide W as used in the present invention is/are not particularly limited to the above specific amino acid sequences but may also be a modified version of the above neuropeptide B and neuropeptide W, respectively, amino acid sequence, as long as it is capable of binding to and/or activating the neuropeptide B/W receptor (NPBWR1) and, accordingly, is capable of transducing its signals comparable to a non-modified neuropeptide B and neuropeptide W.

Thus, in preferred embodiments, the neuropeptide B and neuropeptide W as used in the present invention is a neuropeptide which comprises a sequence which shows 1 to 8 substitutions, deletions, or insertions in comparison to SEQ ID NO:6 and SEQ ID NO:7, respectively. The neuropeptide sequence may also be a sequence which comprises a sequence which shows 1 to 7 substitutions, deletions, or insertions in comparison to SEQ ID NO:6 and SEQ ID NO:7, respectively. The neuropeptide sequence may also be a sequence which comprises a sequence which shows 1 to 6 substitutions, deletions, or insertions in comparison to SEQ ID NO:6 and SEQ ID NO:7, respectively. The neuropeptide sequence may also be a sequence which comprises a sequence which shows 1 to 5 substitutions, deletions, or insertions in comparison to SEQ ID NO:6 and SEQ ID NO:7, respectively. The neuropeptide sequence may also be a sequence which comprises a sequence which shows 1 to 4 substitutions, deletions, or insertions in comparison to SEQ ID NO:6 and SEQ ID NO:7, respectively. The neuropeptide sequence may also be a sequence which comprises a sequence which shows 1 to 3 substitutions, deletions, or insertions in comparison to SEQ ID NO:6 and SEQ ID NO:7, respectively. The neuropeptide sequence may also be a sequence which comprises a sequence which shows 1 to 2 substitutions, deletions, or insertions in comparison to SEQ ID NO:6 and SEQ ID NO:7, respectively. Most preferably, the neuropeptide sequence may also be a sequence which comprises a sequence which shows 1 substitution, deletion, or insertion in comparison to SEQ ID NO:6 and SEQ ID NO:7, respectively.

Preferably, the position(s) of the above amino acid substitution(s), deletion(s) or insertion(s) in comparison to SEQ ID NO:6 and SEQ ID NO:7, respectively are performed at (a) position(s) which is/are less conserved in the sequence of SEQ ID NO:6 and SEQ ID NO:7, respectively, in comparison with corresponding neuropeptide B and W sequences, respectively, from organisms or species other than human.

In order to determine whether a certain amino acid position is less conserved and, therefore, preferably subject to a modification as outlined above, the skilled person can use means and methods well known in the art, e.g., alignments, either manually or by using computer programs known to the person skilled in the art. Such an alignment can, e.g., be done with means and methods known to the skilled person, e.g. by using a known computer algorithm such as the Lipman-Pearson method (Science 227 (1985), 1435) or the CLUSTAL algorithm. It is preferred that in such an alignment maximum homology is assigned to conserved amino acid residues present in the amino acid sequences. Preferably, ClustalW2 is used for the comparison of amino acid sequences. In the case of pairwise comparisons/alignments, the following settings are preferably chosen: Protein weight matrix: BLOSUM 62; gap open: 10; gap extension: 0.1. In the case of multiple comparisons/alignments, the following settings are preferably chosen: Protein weight matrix: BLOSUM 62; gap open: 10; gap extension: 0.2; gap distance: 5; no end gap.

Those positions which turn out to be "identical" (i.e., "conserved") are preferably not subject to a substitution, deletion or insertion. In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same with SEQ ID NO:6 and SEQ ID NO:7, respectively. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul, (1997) Nucl. Acids Res. 25:3389-3402; Altschul (1993) J. Mol. Evol. 36:290-300; Altschul (1990) J. Mol. Biol. 215:403-410). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff (1989) PNAS 89:10915) uses alignments (B) of 50, expectation (E) of 10, M=5, N=4, and a comparison of both strands.

Preferably, the above up to 1, 2, 3, 4, 5, 6, 7, or 8 amino acid substitutions are conservative amino acid substitutions with reference to the sequences of SEQ ID NO:6 and SEQ ID NO:7, respectively.

These "conservative amino acid substitution(s)" refer to substitutions of amino acids in a protein with other amino acids having similar characteristics (e.g., charge, side-chain size, hydrophobicity/hydrophilicity, backbone conformation and rigidity, etc.), such that the changes can frequently be made without altering the biological activity of the protein. Those of skill in this art recognize that, in general, single amino acid substitutions in non-essential regions of a polypeptide do not substantially alter biological activity (see, e.g., Watson Molecular Biology of the Gene, The Benjamin/Cummings Pub. Co. 4th Ed. (1987), 224. In addition, substitutions of structurally or functionally similar amino acids are less likely to disrupt biological activity. Within the context of the present invention the neuropeptide sequence(s) of the present invention comprise polypeptide chains with sequences that include up to 0 (no changes), 1, 2, 3, 4, 5, 6, 7, or 8 conservative amino acid substitutions when compared with the specific amino acid sequences disclosed herein, for example, SEQ ID NO:6 and SEQ ID NO:7, respectively.

Such exemplary substitutions are preferably made in accordance with those set forth in Table 1 as follows:

**TABLE 1**

| Exemplary Conservative Amino Acid Substitutions | |
|---|---|
| Original residue | Conservative substitution |
| Ala (A) | Gly; Ser |
| Arg (R) | Lys; His |
| Asn (N) | Gln; His |
| Asp (D) | Glu; Asn |
| Cys (C) | Ser; Ala |
| Gln (Q) | Asn |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

The neuropeptide sequence which has one or more of the above substitutions, deletions or insertions in comparison to SEQ ID NO:6 and SEQ ID NO:7, respectively may result in a neuropeptide sequence having a similar capability (in terms of properties of being capable of binding to and/or activating the neuropeptide B/W receptor (NPBWR1) and, accordingly, being capable of transducing its signals comparable to a non-modified neuropeptide B and neuropeptide W) as SEQ ID NO:6 and SEQ ID NO:77, respectively, preferably higher capability as SEQ ID NO:6 and SEQ ID NO:7, respectively. The property/capability of a given modified neuropeptide sequence in comparison to SEQ ID NO:6 and SEQ ID NO:7, respectively can easily be determined by the skilled person by methods known in the art. Thus, the property/capability of a given modified neuropeptide sequence in comparison to SEQ ID NO:6 and SEQ ID NO:7, respectively relates to the activity of a certain respective sequence to have the ability to mediate binding to and/or activating the neuropeptide B/W receptor (NPBWR1) and, accordingly, being capable of transducing its signals comparable to a non-modified neuropeptide B and neuropeptide W. The "capability of binding to and/or activating the neuropeptide B/W receptor (NPBWR1) and, accordingly, being capable of transducing its signals comparable to a non-modified neuropeptide B and neuropeptide W" can, e.g., be determined by methods described in the appended examples and as outlined in the following. As regards the capability of a neuropeptide B or W to bind to and/or activate NPBWR1, e.g., quantitative PCR to assay the expression of Bdnf can be used as already explained above. Briefly, as regards the capability of NPBWR1 to bind to neuropeptide B/W, e.g., FRAP (fluorescence recovery after photobleaching) can be used wherein neuropeptide B or neuropeptide W is used as a substrate for the assessment of the binding to NBWBR1. Corresponding assays are known in the art and can easily be performed by the skilled person by utilizing routine methods.

Without being bound by theory, the capability of a neuropeptide B or W to bind to and/or activate NPBWR1 is frequently measured indirectly via the regulation/activation of the expression of the downstream protein Brain derived neurotophic factor (Bdnf). Bdnf RNA-levels can be assessed using quantitative PCR. An increase in the expression of Bdnf is an indication of the inhibition of Npbwr1. Correspondingly, a decrease in the expression of Bdnf is an indication of the activation of Npbwr1.

However, the neuropeptide sequence(s) as used in the present invention is/are not particularly limited to the above specific sequences and the above described deletions, substitutions or insertions but may also relate to a neuropeptide sequence which comprises a sequence which shows (an) amino acid(s) addition(s) in comparison to SEQ ID NO:6 and SEQ ID NO:7, respectively. The addition of amino acid(s) can be flanking or interspersed. Thus, the additional amino acids may be added at the N- and/or C-terminal end of the neuropeptide sequence(s) of the present invention. Alternatively, or in addition to these flanking additional amino acids, the additional amino acids may also be within the amino acid sequence of the neuropeptide sequence(s) of the present invention. The additional amino acid(s) comprise polypeptide chains of up to 0 (no changes), 1, 2, 3, 4, 5, 6, 7, 8, 9 or 10 amino acids, preferably of up to 20 amino acids or even more preferably of up to 30 amino acids. In light of the rationale that the addition of amino acids is likely not to change the above functional properties of the neuropeptide sequence of the invention the addition of the amino acids may also have a length of up to 40, 50, 60, 70, 80, 90, or even 100 amino acids or even more, up to 200, 300, 400 or 500 amino acids as long as these sequences have a similar capability (in terms of in of being capable of binding to and/or activating the neuropeptide B/W receptor (NPBWR1) and, accordingly, being capable of transducing its signals comparable to a non-modified neuropeptide B and neuropeptide W) as SEQ ID NO:6 and SEQ ID NO:7, respectively, preferably higher capability as SEQ ID NO:6 and SEQ ID NO:7, respectively, as defined above.

The neuropeptide sequence(s) of the present invention may be recombinantly or synthetically generated/synthesized by methods known to the person skilled in the art. More specifically, as described further below, the neuropeptide of the present invention may be produced either recombinantly by methods known to the person skilled in the art or may, e.g., be conveniently synthesized on an automated peptide synthesizer using a solid-phase support and standard techniques of repetitive orthogonal deprotection and coupling. Free amino groups in the peptide, that are to be used later for conjugation of moieties or other agents, are advantageously blocked with standard protecting groups such as a Boc group, while N-terminal residues may be acetylated to increase serum stability. Such protecting groups are well known to the skilled person; see Greene and Wuts Protective Groups in Organic Synthesis, 1999 (John Wiley and Sons, N.Y.). When the peptides are prepared for later use within the construct of the present invention, they are advantageously cleaved from the resins to generate the corresponding C-terminal amides, in order to inhibit in vivo carboxypeptidase activity.

In a further preferred embodiment, the present invention relates to a pharmaceutical composition comprising an agonist/activator of neuropeptide B/W receptor (NPBWR1) for use in in a method of treating, ameliorating or preventing a bipolar affective disorder (ICD-10 F31) during the manic phase, appetitive disorders, preferably anorexia or bulimia as described herein above, wherein said agonist/activator is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional activator domain and an epigenetic modification domain capable of activating the expression of NPBWR1.

The feature "a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional activator domain and an epigenetic modification domain" has already described above in the context of the pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders, wherein said antagonist/inhibitor is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional repressor domain and an epigenetic modification domain capable of repressing the expression of NPBWR1.

The only difference in the now-described context of the pharmaceutical composition comprising an agonist/activator of neuropeptide B/W receptor (NPBWR1) (for use in in a method of treating, ameliorating or preventing a bipolar affective disorder (ICD-10 F31) during the manic phase, appetitive disorders , preferably anorexia or bulimia as described herein above, wherein said agonist/activator is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional activator domain and an epigenetic modification domain capable of activating the expression of NPBWR1) is the fact that the Cas-protein is fused to an effector domain selected from the group consisting of a transcriptional activator domain and an epigenetic modification domain capable of activating the expression of NPBWR1 (while, in contrast, in the further above-described context of the pharmaceutical composition comprising an antagonist/inhibitor of neuropeptide B/W receptor (NPBWR1), Cas-protein is fused to an effector domain selected from the group consisting of a transcriptional repressor domain and an epigenetic modification domain capable of repressing the expression of NPBWR1).

Accordingly, with the exception of this only difference, as regards this feature as well as preferred embodiments the same applies, *mutatis mutandis,* to the here defined agonist/activator-arm of the medical setting as has been set forth above in the context of the pharmaceutical composition comprising an antagonist/inhibitor of neuropeptide B/W receptor (NPBWR1) for use in in a method of treating, ameliorating or preventing a mood disorder/affective disorder and/or chronic stress and/or anxiety disorders and/or Parkinson's disease, wherein said antagonist/inhibitor is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional repressor domain and an epigenetic modification domain capable of repressing the expression of NPBWR1.

In particular, suitable effector domains selected from the group consisting of a transcriptional activator domain and an epigenetic modification domain capable of activating the expression of NPBWR1) to be used in the context of the agonistic/activating arm of the medical use has also been defined above.

As outlined above, the pharmaceutical composition comprising the antagonist/inhibitor of neuropeptide B/W receptor (NPBWR1) of the present invention as defined above and the pharmaceutical composition comprising the agonist/activator of neuropeptide B/W receptor (NPBWR1) of the present invention as defined above, respectively, are particularly useful in medical settings.

Thus, in a preferred embodiment, the present invention relates to a pharmaceutical composition comprising the antagonist/inhibitor of neuropeptide B/W receptor (NPBWR1) of the present invention as defined above and a pharmaceutical composition comprising the agonist/activator of neuropeptide B/W receptor (NPBWR1) of the present invention as defined above, respectively, and at least one pharmaceutically acceptable excipient.

Hence, in a preferred embodiment, the present invention relates a pharmaceutical composition comprising the antagonist/inhibitor of neuropeptide B/W receptor (NPBWR1) of the present invention as defined above and a pharmaceutical composition comprising the agonist/activator of neuropeptide B/W receptor (NPBWR1) of the present invention as defined above, respectively, for use as a drug.

The term "treatment" and/or "prevention" and the like are used herein to generally mean obtaining a desired pharmacological and/or physiological effect. Accordingly, the treatment of the present invention may relate to the treatment of (acute) states of a certain disease/disorder but may also relate to the prophylactic treatment in terms of completely or partially preventing a disease/disorder or symptom thereof. Preferably, the term "treatment" is to be understood as being therapeutic in terms of partially or completely curing a disease/disorder and/or adverse effect and/or symptoms attributed to the disease/disorder. "Acute" in this respect means that the subject shows symptoms of the disease or disorder. In other words, the subject to be treated is in actual need of a treatment and the term "acute treatment" in the context of the present invention relates to the measures taken to actually treat the disease/disorder after the onset of the disease/disorder or the outbreak of the disease/disorder. The treatment may also be prophylactic or preventive treatment, i.e., measures taken for disease/disorder prevention.

The pharmaceutical composition or drug of the present invention may be administered via a large range of classes of forms of administration known to the skilled person. Administration may be systemically, locally, orally, through aerosols including but not limited to tablets, needle injection, the use of inhalators, creams, foams, gels, lotions and ointments.

Preferably, the pharmaceutical composition comprising the antagonist/inhibitor of neuropeptide B/W receptor (NPBWR1) of the present invention as defined above and the pharmaceutical composition comprising the agonist/activator of neuropeptide B/W receptor (NPBWR1) of the present invention as defined above, respectively, is to be administered intravenously, topically, intradermally, subcutaneously, intra-cutanously, intramuscular an/or intrathecal.

These routes of administration, i.e., via an intravenously, topically, intradermally, subcutaneously, intra-cutanously, intramuscular an/or intrathecal are known to the skilled person.

An excipient or carrier is an inactive substance formulated alongside the active ingredient, i.e., the pharmaceutical composition comprising the antagonist/inhibitor of neuropeptide B/W receptor (NPBWR1) of the present invention as defined above and the pharmaceutical composition comprising the agonist/activator of neuropeptide B/W receptor (NPBWR1) of the present invention as defined above, respectively, for the purpose of bulking-up formulations that contain potent active ingredients. Excipients are often referred to as "bulking agents," "fillers," or "diluents". Bulking up allows convenient and accurate dispensation of a drug substance when producing a dosage form. They also can serve various therapeutic-enhancing purposes, such as facilitating drug absorption or solubility, or other pharmacokinetic considerations. Excipients can also be useful in the manufacturing process, to aid in the handling of the active substance concerned such as by facilitating powder flowability or non-stick properties, in addition to aiding in vitro stability such as prevention of denaturation over the expected shelf life. The selection of appropriate excipients also depends upon the route of administration and the dosage form, as well as the active ingredient and other factors.

Thus, the pharmaceutical composition comprising the antagonist/inhibitor of neuropeptide B/W receptor (NPBWR1) of the present invention as defined above and the pharmaceutical composition comprising the agonist/activator of neuropeptide B/W receptor (NPBWR1) of the present invention as defined above, respectively, may be in solid, liquid or gaseous form and may be, *inter alia,* in a form of (a) powder(s), (a) tablet(s), (a) solution(s) or (an) aerosol(s). It is preferred that said pharmaceutical composition optionally comprises a pharmaceutically acceptable carrier and/or diluent.

These pharmaceutical compositions can be administered to the subject at a suitable dose. Administration of the suitable compositions may be affected by different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in a lung artery or directly into the lung. The compositions of the invention may also be administered directly to the target site, e.g., by biolistic delivery to an external or internal target site. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 ng and 10 mg/kg body weight per dose; however, doses below or above this exemplary range are envisioned, especially considering the aforementioned factors. If the regimen is a continuous infusion, it should also be in the range of 1 µg to 10 mg units per kilogram of body weight per minute.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose, i.e., in "an effective amount" which can easily be determined by the skilled person by methods known in the art. The dosage regimen will be determined by the attending physician and clinical factors. As is well known in the medical arts, dosages for any one patient depends upon many factors, including the patient's or subject's size, body surface area, age, the particular compound to be administered, sex, time and route of administration, general health, and other drugs being administered concurrently.

Thus, preferably, the antagonist/inhibitor of neuropeptide B/W receptor (NPBWR1) of the present invention as defined above and the agonist/activator of neuropeptide B/W receptor (NPBWR1) of the present invention as defined above, respectively, is/are included in an effective amount. The term "effective amount" refers to an amount sufficient to induce a detectable therapeutic response in the subject to which the pharmaceutical composition is to be administered. In accordance with the above, the content of the antagonist/inhibitor of neuropeptide B/W receptor (NPBWR1) of the present invention as defined above and the agonist/activator of neuropeptide B/W receptor (NPBWR1) of the present invention as defined above, respectively, in the pharmaceutical composition is not limited as far as it is useful for treatment as described above, but preferably contains 0.0000001-10% by weight per total composition. Further, the antagonist/inhibitor of neuropeptide B/W receptor (NPBWR1) of the present invention as defined above and the agonist/activator of neuropeptide B/W receptor (NPBWR1) of the present invention as defined above, respectively, is/are preferably employed in a carrier. Generally, an appropriate amount of a pharmaceutically acceptable salt is used in the carrier to render the composition isotonic. Examples of the carrier include but are not limited to saline, Ringer's solution and dextrose solution. Preferably, acceptable excipients, carriers, or stabilisers are non-toxic at the dosages and concentrations employed, including buffers such as citrate, phosphate, and other organic acids; salt-forming counter-ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol). Suitable carriers and their formulations are described in greater detail in Remington's Pharmaceutical Sciences, 17th ed., 1985, Mack Publishing Co.

Therapeutic progress can be monitored by periodic assessment.

The pharmaceutical composition/drug of the invention may be in sterile aqueous or non-aqueous solutions, suspensions, and emulsions as well as creams and suppositories. Examples of non-aqueous solvents are propylene glycol, polyethylene glycol, vegetable oils such as olive oil, and organic esters such as ethyl oleate. Aqueous carriers include water, alcoholic/aqueous solutions, emulsions or suspensions, including saline and buffered media. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition. Said agents may be, e.g., polyoxyethylene sorbitan monolaurate, available on the market with the commercial name Tween, propylene glycol, EDTA, Citrate, Sucrose as well as other agents being suitable for the intended use of the pharmaceutical composition that are well-known to the person skilled in the art.

In accordance with this invention, the term "pharmaceutical composition" relates to a composition for administration to a patient, preferably a human patient.

The invention also relates to method of treating or preventing a disorder or a disease as defined herein above in a subject, wherein the pharmaceutical composition comprising the antagonist/inhibitor of neuropeptide B/W receptor (NPBWR1) of the present invention as defined above and the pharmaceutical composition comprising the agonist/activator of neuropeptide B/W receptor (NPBWR1) of the present invention as defined above, respectively, is administered to a subject, preferably, in a therapeutically effective amount as defined above .

As regards the preferred embodiments of the method for treatment the same applies, *mutatis mutandis,* as has been set forth above in the context of the antibody or the pharmaceutical composition for use as defined above.

In the present invention, the subject is, in a preferred embodiment, a mammal such as a dog, cat, pig, cow, sheep, horse, rodent, e.g., rat, mouse, and guinea pig, or a primate, e.g., gorilla, chimpanzee, and human. In a most preferable embodiment, the subject is a human.

Other aspects and advantages of the invention will be described in the following examples, which are given for purposes of illustration and not by way of limitation. Each publication, patent, patent application or other document cited in this application is hereby incorporated by reference in its entirety.
- **Figure 1:**: **Acute caffeine injection reverses CVS-induced changes in depressive-like behavior and dendritic spine changes in females.**
**A**) Experimental overview. A subgroup of mice was stereotaxically injected with a GFP-expressing AAV for dendritic spine analysis. **B-E, H, I**) Statistics: 2-way ANOVA Bonferroni posthoc-test. **B-E**) Stress-induced behavioral changes are reversed 24h after caffeine injection. **B**) Forced swim test. n = 9-10 per group; effect of stress: F(1,35) = 10.38, P < 0.01; effect of drug: F(1,35) = 23.36, P < 0.0001; posthoc-test: effect of Caf within Naive: *P < 0.05, within CVS: ***P < 0.001.; effect of stress within Sal: **P < 0.01, within Caf: P > 0.05. **C**) Novelty suppressed feeding, n = 14-15 per group; effect of stress: F(1,54) = 6.21, P < 0.05; effect of drug: F(1,54) = 6.17, P < 0.05; interaction: F(1,54) = 4.28, P < 0.05; posthoc-test: effect of drug within Naive: P > 0.05, within CVS: **P < 0.01; effect of stress within Sal: **P < 0.01, within Caf: P > 0.05. **D**) Splash test. n = 7-8 per group; effect of stress: F(1,26) = 1.05, P = 0.32; effect of drug: F(1,26) = 0.41, P = 0.53; interaction: F(1,26) = 0.57, P = 0.46; posthoc-test: all comparisons: P > 0.05. **E**) Sucrose preference. n = 10-12 per group; effect of stress: F(1,42) = 2.91, P = 0.10; effect of drug: F(1,42) = 0.72, P = 0.40; interaction: F(1,42) = 1.61, P = 0.21; posthoc-test: all comparisons: P > 0.05. **F-N**) CVS-induced changes in NAc dendritic spines are reversed by caffeine. **F**) Overview image of GFP-labelled neurons. **G**) Representative dendrites. Scale bar 10 µm. **H**) Stubby spines. n = 39-58 dendrites from 4-6 mice per group; 2-way ANOVA: effect of stress: F(1,193) = 2.90, P = 0.09; effect of drug: F(1,193) = 5.62, P < 0.05; interaction: F(1,193) = 0.11, P = 0.73; posthoc-test: all comparisons: P > 0.05. **I**) Neck-containing spines. n = 40-58 dendrites from 4-6 mice per group; 2-way ANOVA: effect of stress: F(1,194) = 3.34, P = 0.06; effect of drug: F(1,194) = 4.34, P < 0.05; interaction: F(1,194) = 3.24, P = 0.07; posthoc-test: effect of drug within Naive: P > 0.05, within CVS: *P < 0.05; effect of stress within Sal: *P < 0.05, within Caf: P > 0.05. **J-M**) Cumulative head diameter is increased by CVS and reversed by caffeine; statistics: Gehan-Breslow-Wilcoxon test. **J**) Effect of drug within Naive: χ² = 65.36, degrees of freedom (DF) = 1, ***P < 0.0001. **K**) Effect of drug within CVS: χ² = 51.74, DF = 1, ***P < 0.0001. L) Effect of stress within Saline: χ² = 141.90, DF = 1, ***P < 0.0001. **M**) Effect of stress within Caffeine: χ² = 7.04, DF = 1, **P < 0.01. **B-E, H, I**) Independent data points are plotted and means ± s.e.m. are shown. Scetches were made with biorender.com.

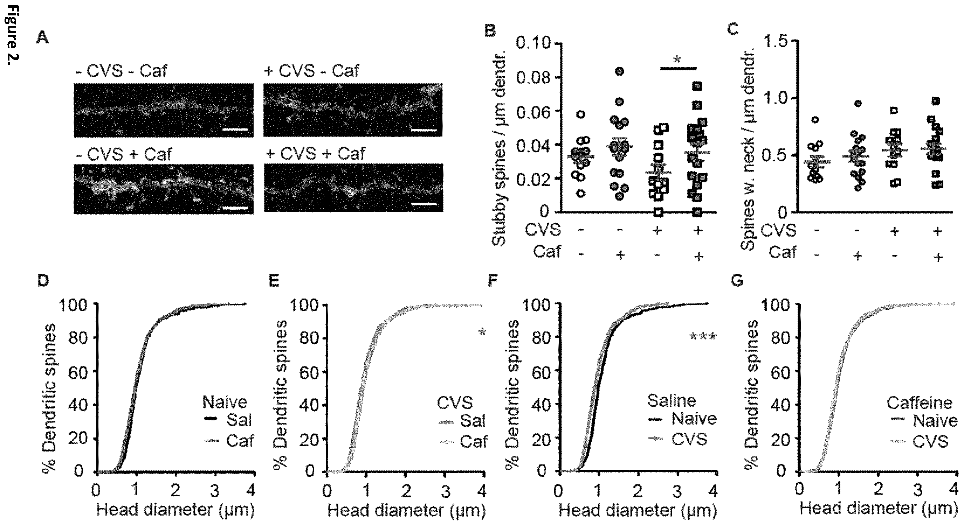

- **Figure 2:**: **CVS induces subtle spine changes in males, which are partially reversed by caffeine.**
**A**) Representative dendrites. Scale bar 10 µm. **B**) Stubby spines. n = 31-40 dendrites from 3-4 mice per group; 2-way ANOVA: effect of stress: F(1,136) = 1.77, P = 0.19; effect of drug: F(1,136) = 6.68, P < 0.05; interaction: F(1,136) = 0.66, P = 0.42; Bonferroni posthoc-test: effect of drug within CVS: *P < 0.05, all other comparisons: P > 0.05. **C**) Neck-containing spines. n = 32-40 dendrites from 3-4 mice per group; 2-way ANOVA: effect of stress: F(1,139) = 0.01, P = 0.91; effect of drug: F(1,139) = 0.13, P = 0.72; interaction: F(1,139) = 2.53, P = 0.11; Bonferroni posthoc-test: all comparisons P > 0.05. **D-G**) Cumulative head diameter is reduced by CVS and reversed by caffeine; statistics: Gehan-Breslow-Wilcoxon test. **D**) Effect of drug within Naive: χ² = 3.61, degrees of freedom (DF) = 1, P = 0.06. **E**) Effect of drug within CVS: χ² = 6.56, DF = 1, *P < 0.05. **F**) Effect of stress within Saline: χ² = 23.17, DF = 1, ***P < 0.0001. **G**) Effect of stress within Caffeine: χ² = 1.48, DF = 1, P = 0.22. **B, C**) Independent data points are plotted and means ± s.e.m. are shown.

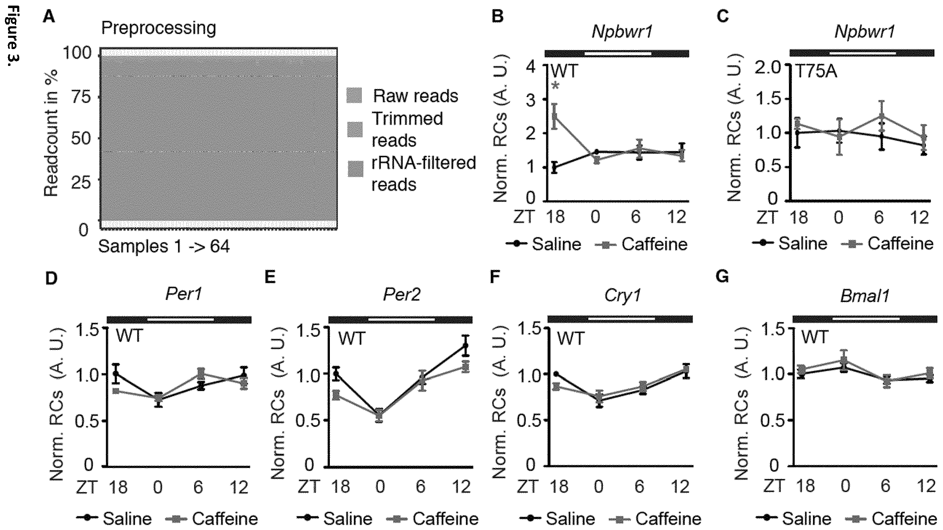

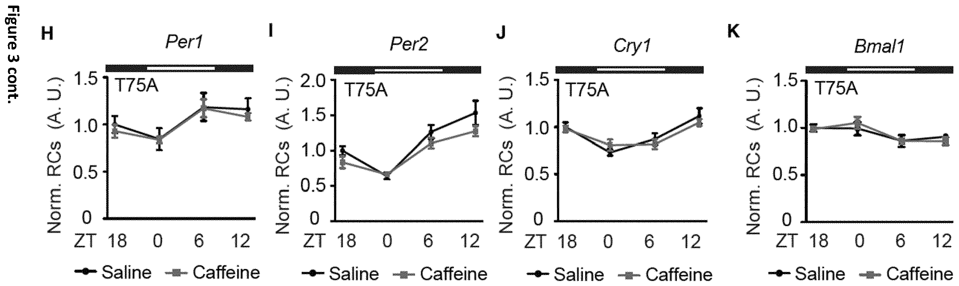

- **Figure 3:**: **Quality control markers and read count distribution of RNA-sequencing.**
**A**) Preprocessing information on RNA-quality. All samples reached at least 90% alignment. **B, C**) Distribution of *Npbwrl* read-counts (normalized for WT ZT18) over time. **B-K**) n = 4 per group; statistics: 2-way ANOVA & Bonferroni posthoc-test. **B**) WT; effect of drug: F(1,24) = 1.38, P < 0.05; effect of time: F(3,24) = 0.71, P = 0.25; interaction: F(3,24) = 3.68, P = 0.55; posthoc-test: effect of caffeine within ZT18: *P < 0.05, within all other ZT: P > 0.05. Sal: ZT18 vs. ZT0: P < 0.001; ZT18 vs. ZT6, 12: P < 0.01; all others: P > 0.05. **C**) T75A; effect of drug: F(1,24) = 0.76, P = 0.39; effect of time: F(3,24) = 0.57, P = 0.63; interaction: F(3,24) = 0.36, P = 0.78; posthoc-test: Effect of caffeine within all ZT: all P > 0.05; effect of time points within sal and caf: all P > 0.05. **D-K**) Normalized read-counts (RCs) for circadian genes. Circadian alterations are in agreement with the literature. Caffeine but not T75A-mutation may slightly flatten the amplitude of circadian oscillations. **D-G**) WT. **D**) *Per1*; effect of drug: F(1,24) = 0.47, P = 0.50; effect of time: F(3,24) = 5.05, P < 0.01; interaction: F(3,24) = 2.25, P = 0.11; posthoc-test: effect of caffeine within all ZT: P > 0.05, Sal: ZT18 vs. ZT0: P < 0.01; ZT0 vs. ZT12: P < 0.05; Caf: ZT0 vs. ZT6: P < 0.05; all others: P > 0.05. **E**) *Per2*; effect of drug: F(1,24) = 5.35, P < 0.05; effect of time: F(3,24) = 23.82, P < 0.0001; interaction: F(3,24) = 1.27, P = 0.31; posthoc-test: Effect of caffeine within all ZT: P > 0.05; Sal: ZT18 vs. ZT0, ZT0 vs. ZT12, ZT6 vs. ZT12: P < 0.001; ZT18 vs. ZT12: P < 0.05; ZT0 vs. ZT6: P < 0.01; Caf: ZT18 vs. 12: P < 0.05; ZT0 vs. ZT6: P < 0.01; ZT0 vs. ZT12: P < 0.001; all others: P > 0.05. **F**) *Cry*; effect of drug: F(1,24) = 0.03, P = 0.87; effect of time: F(3,24) = 13.19, P < 0.0001; interaction: F(3,24) = 1.41, P = 0.27; posthoc-test: Effect of caffeine within all ZT: P > 0.05; Sal: ZT0 vs. ZT6: P < 0.01; ZT0 vs. ZT6, ZT6 vs. ZT12: P < 0.05; Caf: ZT0 vs. ZT12: P < 0.05; ZT0 vs. ZT12: P < 0.001; ZT6 vs. ZT12: P < 0.01; all others: P > 0.05. **G)** *Bmal;* effect of drug: F(1,24) = 1.09, P = 0.31; effect of time: F(3,24) = 3.48, P < 0.05; interaction: F(3,24) = 0.23, P = 0.88; posthoc-test: Effect of caffeine within all ZT: P > 0.05; Caf: ZT0 vs. ZT6: P < 0.05; all others: P > 0.05. **H-K)** T75A. **H**) *Per1*; effect of drug: F(1,24) = 0.40, P = 0.53; effect of time: F(3,24) = 5.14, P < 0.01; interaction: F(3,24) = 0.08, P = 0.97; posthoc-test: effect of caffeine within all ZT: P > 0.05, Sal, Caf: ZT0 vs. ZT6: P < 0.05; all others: P > 0.05. **I**) *Per2*; effect of drug: F(1,24) = 4.9, P < 0.05; effect of time: F(3,24) = 26.49, P < 0.0001; interaction: F(3,24) = 0.88, P = 0.47; posthoc-test: effect of caffeine within all ZT: P > 0.05, Sal: ZT18 vs. ZT0: P < 0.05; ZT18 vs. ZT12, ZT0 vs. ZT6, ZT0 vs. ZT12: P < 0.001; Caf: ZT18 vs. ZT12, ZT0 vs. ZT6: P < 0.01; ZT0 vs. ZT12: P < 0.0001; all others: P > 0.05. **J**) *Cry*; 2-way ANOVA: effect of drug: F(1,24) = 0. 17, P = 0.67; effect of time: F(3,24) = 13.76, P < 0.0001; interaction: F(3,24) = 0.75, P = 0.53; posthoc-test: Effect of caffeine within all ZT: P > 0.05; Sal: ZT18 vs. ZT0, ZT6 vs. ZT12: P < 0.01; ZT0 vs. ZT12: P < 0.001; Caf: ZT0 vs. ZT12: P < 0.01; ZT6 vs. ZT12: P < 0.05; all others: P > 0.05. K) *Bmal;* effect of drug: F(1,24) < 0.01, P = 0.99; effect of time: F(3,24) = 5.73, P < 0.01; interaction: F(3,24) = 0.45, P = 0.72; posthoc-test: Effect of caffeine within all ZT: P > 0.05; Caf: ZT0 vs. ZT6, ZT0 vs. ZT12: P < 0.05; all others: P > 0.05. **B-K**) Means ± s.e.m. are shown.

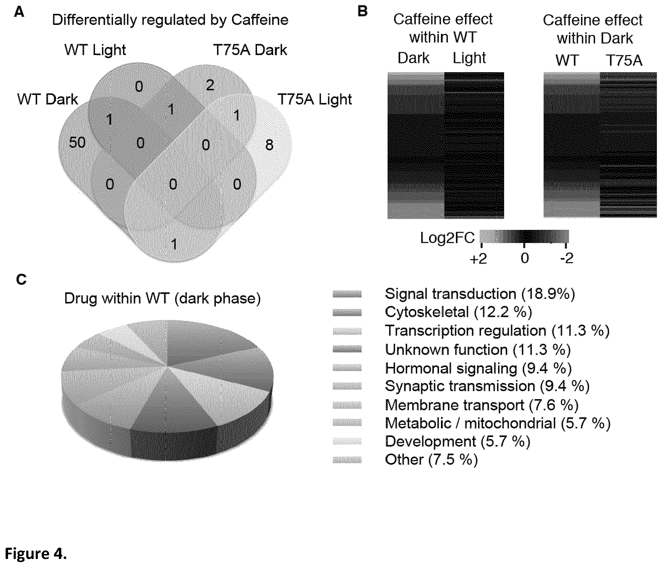

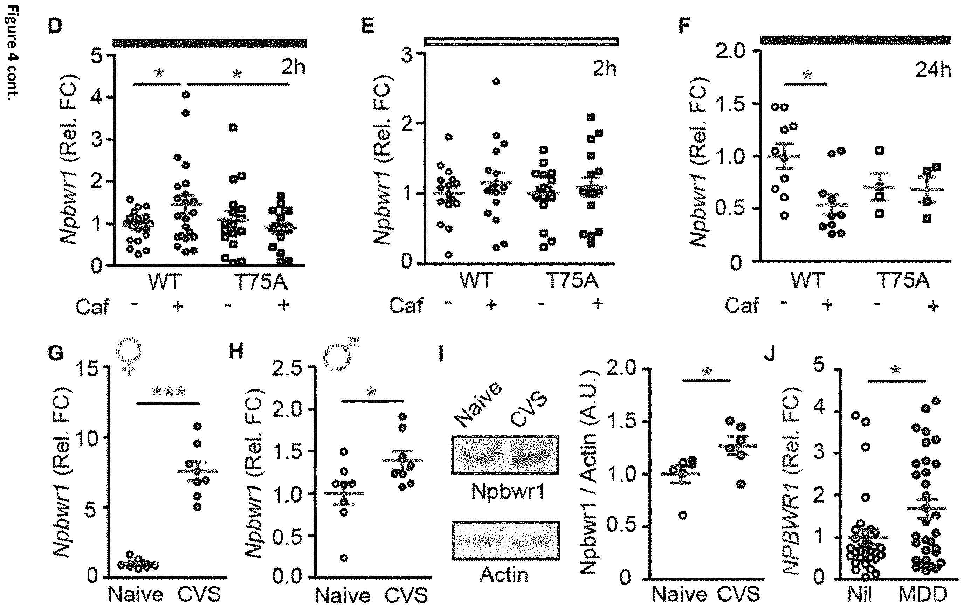

- **Figure 4:**: **RNA-sequencing identifies Npbwr1 as a gene product that is diurnally regulated by the DARPP-32 pathway.**
**A-C**) RNA-sequencing on the NAc of wildtype (WT) and DARPP-32-T75A mutant mice (T75A) at active and inactive phases of the light cycle, 2h after caffeine injection. **A**) Venn diagrams of significant (Padj < 0.05, log2FC 0.5 <> -0.5) gene expression changes between various conditions. These data confirm that the biggest effect of caffeine occurs in WT in the active phase. **B**) Heat maps comparing transcriptional changes (log(FC) in across light-phases and genotypes. **C**) Pie chart of annotated pathways in the WT-group (dark phase). **D-F**) Validation in a different cohort by qPCR. **D**) 2h after caffeine, dark-phase. n = 16-22 per group; 2-way ANOVA: effect of genotype: F(1,73) = 0.91, P = 0.34; effect of drug: F(1,73) = 1.53, P = 0.22; interaction: F(1,73) = 4.57, P < 0.05; Bonferroni posthoc-test: effect of drug within WT: *P < 0.05, within T75A: P > 0.05; effect of genotype within Sal. P > 0.05, within Caf: *P < 0.05. **E**) 2h after caffeine, light-phase. n = 12-16 per group; 2-way ANOVA: effect of genotype: F(1,53) = 0.05, P = 0.82; effect of drug: F(1,53) = 0.93, P = 0.34; interaction: F(1,53) = 0.06, P = 0.80. Bonferroni posthoc-test: all P > 0.05. **F**) 24h after caffeine, dark-phase. n = 4-10 per group; 2-way ANOVA: effect of genotype: F(1,24) < 0.01, P = 0.98; effect of drug: F(1,24) = 2.02, P = 0.17; interaction: F(1,24) = 1.53, P = 0.23; Bonferroni posthoc-test: effect of drug within WT: *P < 0.05, within T75A: P > 0.05.; effect of genotype within Sal: P > 0.05, within Caf: P > 0.05. **G, H**) *Npbwr1*-levels are increased after chronic variable stress (CVS). **G**) Females: n = 8 per group; t-test: t = 9.71, df = 14; ***P < 0.0001 **H**) Males: n = 8 per group; t-test: t = 2.27 df = 14; *P < 0.05. **I**) Npbwr1 protein levels are elevated after CVS: n = 6 per group; t-test: t = 2.27 df = 10; *P < 0.05. **J**) *NPBWR1* is increased in depressed patients (MDD) vs. controls (Nil): n = 30-32 per group; t-test: t = 2.36, df = 60; *P < 0.05. **D-J**) Independent data points are plotted and means ± s.e.m. are shown.
- **Figure 5:**: **Demographics of postmortem samples and separation by cause of death.**
**A**) Patient information. **B**) Analysis split by cause of death and diagnosis. n = 11-21 per group; 2-way ANOVA: effect of depr: F(1,65) = 5.45, ^{#}P < 0.05; effect of cause of death: F(1,65) = 0.38, P = 0.36; interaction: F(1,65) = 0.66, P = 0.42; Bonferroni posthoc-test: all comparisons P > 0.05. Independent data points are plotted and means ± s.e.m. are shown. Nil: controls. Depr.: Depression.

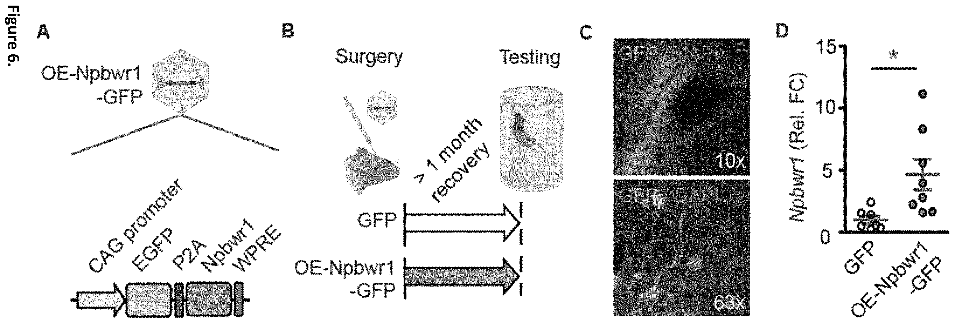

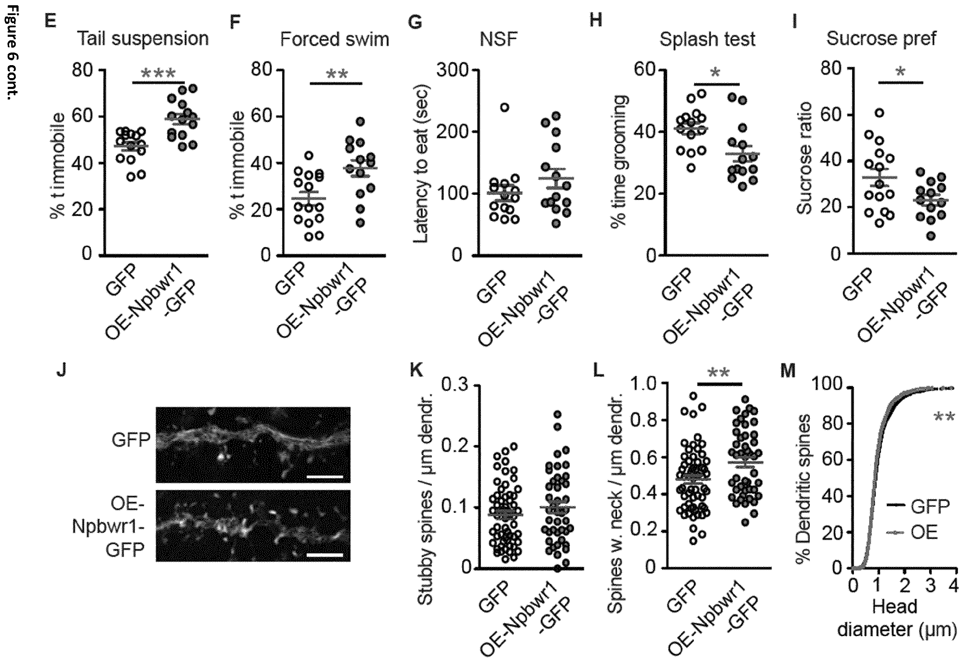

- **Figure 6:**: **Overexpression of *Npbwr1* mimics stress-effects on behavior and dendritic spines.**
**A**) Schematic of the OE-Npbwr1-GFP AAV. **B**) Experimental plan. **C**) Overview image of viral injection into the NAc. **D**) qPCR. n = 7-8 per group; Student's t-test: t₁₃ = 2.71, *P < 0.05. **E-I**) Depressive-like behaviors are increased by overexpression (OE) of *Npbwr1*; statistics: Student's t-test. **E**) Tail suspension test. n = 14 per group; t₂₆ = 4.17, ***P < 0.001. **F**) Forced swim test. n = 13-15 per group; t₂₆ = 3.00, **P < 0.01. **G**) Novelty suppressed feeding, n = 14 per group; t₂₆ = 1.21, P = 0.24. **H**) Splash test. n = 14 per group; t₂₆ = 2.68, *P < 0.05. **I**) Sucrose preference. n = 13-15 per group; t₂₆ = 2.15, P > 0.05. **J-M**) Dendritic spines in the NAc are altered by OE-Npbwr1 in a manner reminiscent of CVS. **J**) Representative dendrites. Scale bar 10 µm. **K**) Stubby spines. n = 46-57 from 5-6 mice per group; t₉₈ = 1.13, P = 0.26. **L**) Neck-containing spines. n = 48-59 from 5-6 mice per group; t₁₀₂ = 2.76, **P < 0.01. **M**) Cumulative head diameter. Gehan-Breslow-Wilcoxon test: χ² = 7.83, DF = 1, **P < 0.01. **D-I, K, L**) Independent data points are plotted and means ± s.e.m. are shown. Scetches were made with biorender.com.

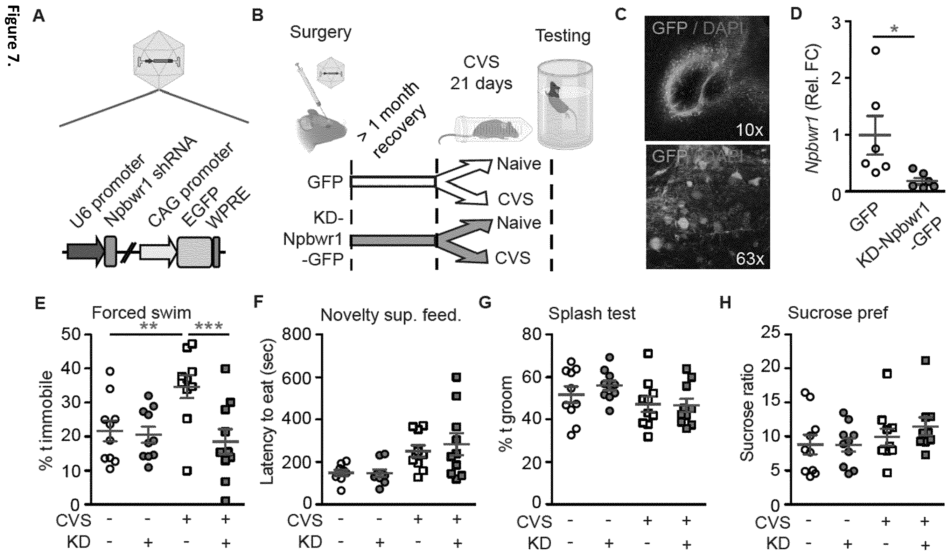

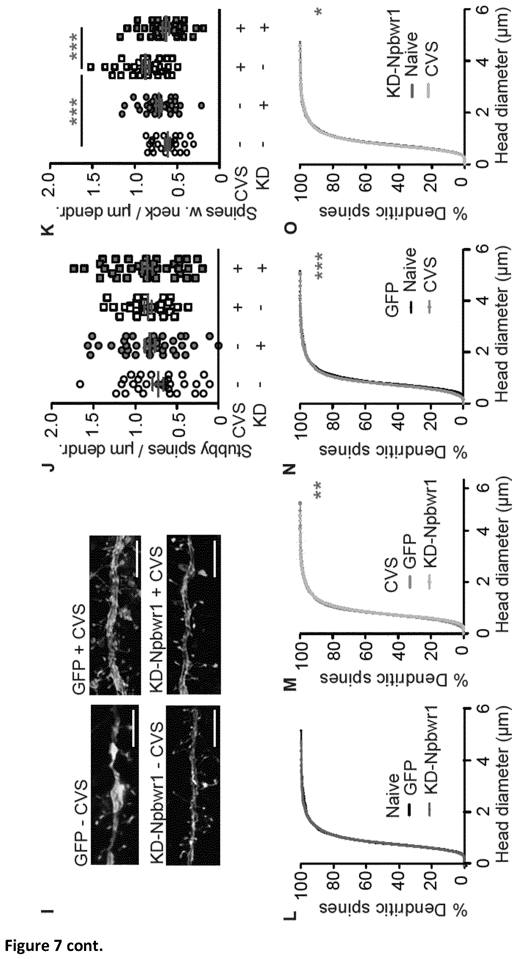

- **Figure 7:**: **Knockdown of Npbwr1 blocks effects of CVS on behavior and dendritic spines.**
**A**) Schematic of the KD-Npbwr1-GFP AAV. **B**) Experimental plan. **C**) Overview image of viral injection into the NAc. **D**) qPCR (naïve mice): n = 6 per group; Student's t-test: t₁₀ = 2.33, *P < 0.05. **E-H**) Depressive-like behaviors are partially blocked by KD-Npbwr1. **E**) Forced swim test. n = 10 per group; 2-way ANOVA: effect of stress: F(1,36) = 3.41, P = 0.07; effect of AAV: F(1,36) = 8.40, P < 0.01; interaction: F(1,36) = 6.42, P < 0.05; Bonferroni posthoc-test: effect of AAV within Naive: P > 0.05, within CVS: ***P < 0.001.; effect of stress within GFP: **P < 0.01, within KD: P > 0.05. **F**) Novelty suppressed feeding, n = 10 per group; 2-way ANOVA: effect of stress: F(1,36) = 4.51, P < 0.05; effect of AAV: F(1,36) = 0.03, P = 0.87; interaction: F(1,36) = 0.45, P = 0.51; Bonferroni posthoc-test: all comparisons: P > 0.05. **G**) Splash test. n = 10 per group; 2-way ANOVA: effect of stress: F(1,36) = 0.37, P = 0.55; effect of AAV: F(1,36) = 5.13, P < 0.05; interaction: F(1,36) = 0.62, P = 0.43; Bonferroni posthoc-test: all comparisons: P > 0.05. **H**) Sucrose preference. n = 10 per group; 2-way ANOVA: effect of stress: F(1,36) = 0.24, P = 0.55; effect of AAV: F(1,36) = 3.71, P = 0.06; interaction: F(1,36) = 1.55, P = 0.22; Bonferroni posthoc-test: all comparisons: P > 0.05. **I-O**) Stress-induced spine changes are reversed by KD-Npbwr1. **I**) Representative dendrites. Scale bar 10 µm. **J**) Stubby spines. n = 30-39 dendrites from 3-4 mice per group; 2-way ANOVA: effect of stress: F(1,152) = 2.24, P = 0.14; effect of AAV: F(1,152) = 3.38, P = 0.21; interaction: F(1,152) = 2.24, P = 0.07; Bonferroni posthoc-test: all comparisons: P > 0.05. **K**) Neck-containing spines. n = 28-37 dendrites from 3-4 mice per group; 2-way ANOVA: effect of stress: F(1,132) = 6.10, P < 0.05; effect of AAV: F(1,132) = 3.70, P = 0.06; interaction: F(1,132) = 23.11, P < 0.0001; Bonferroni posthoc-test: effect of AAV within Naive: P > 0.05, within CVS: ***P > 0.001; effect of stress within GFP: ***P < 0.001, within KD: P > 0.05. **L-O**) The cumulative head diameter, is reduced by CVS and rescued by KD-Npbwr1; statistics: Gehan-Breslow-Wilcoxon test. **L**) Effect of AAV within Naive: χ² < 0.01, DF = 1, P = 0.96. **M**) Effect of AAV within CVS: χ² = 7.71, DF = 1, **P < 0.01. **N**) Effect of stress within GFP: χ² = 20.05, DF = 1, ***P < 0.0001. **O**) Effect of stress within KD: χ² = 4.58, DF = 1, *P < 0.05. **E-H, J, K**) Independent data points are plotted and means ± s.e.m. are shown. Scetches were made with biorender.com.

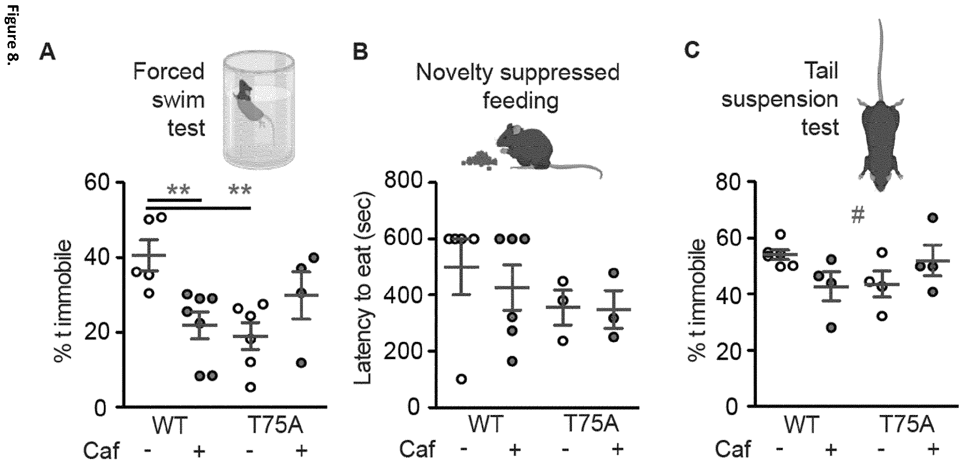

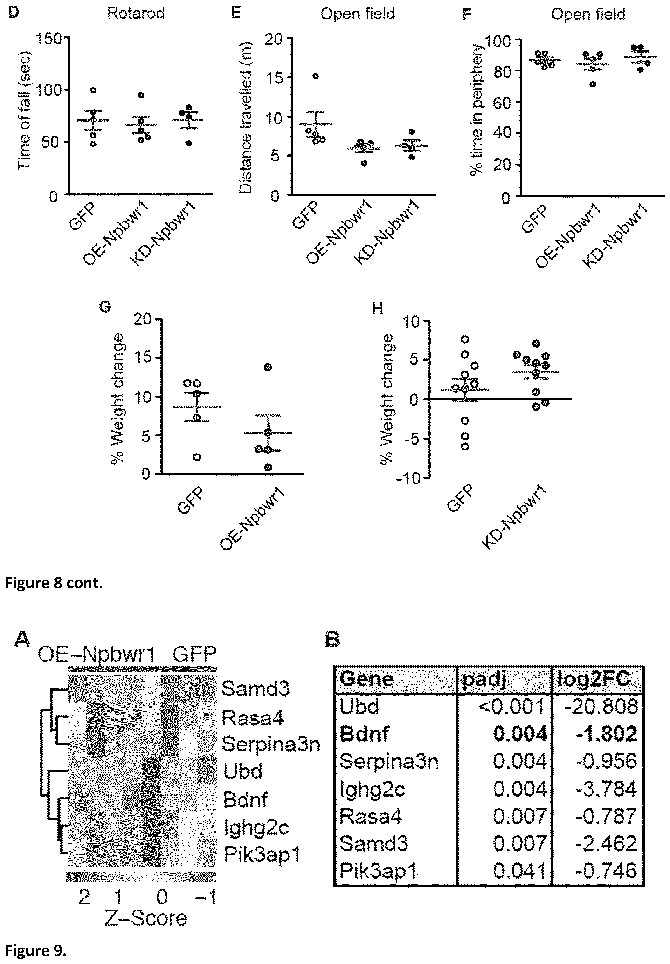

- **Figure 8:**: **Escape behaviors are altered in naïve WT mice 24h after an acute dose of caffeine.**
**A-C**) Naive WT and T75A mutants received 7.5 mg/kg of caffeine vs. saline in the dark phase and were tested 24h later. **A**) Forced swim test: n = 5-7 per group; 2-way ANOVA: effect of drug: F(1,18) = 0.83, P = 0.37; effect of genotype: F(1,18) = 2.49, P = 0.13; interaction: F(1,18) = 11.73, P < 0.01; Bonferroni posthoc-test: effect of Caf within WT: **P < 0.01, within T75A: P > 0.05.; effect of genotype within Sal: **P < 0.01, within Caf: P > 0.05. **B**) Novelty suppressed feeding: n = 3-5 per group; 2-way ANOVA: effect of drug: F(1,13) = 0.18, P = 0.68; effect of genotype: F(1,13) 1.40, P = 0.26; interaction: F(1,13) = 0.13, P = 0.73; Bonferroni posthoc-test: all P > 0.05. **C**) Tail suspension test: n = 4-6 per group; 2-way ANOVA: effect of drug: F(1,14) = 0.12, P = 0.73; effect of genotype: F(1,14) = 0.02, P = 0.89; interaction: F(1,14) = 5.73, ^{#}P < 0.05; Bonferroni posthoc-test: all P > 0.05. **A-C**) Independent data points are plotted and means ± s.e.m. are shown.
- **Figure 9:**: **Differentially expressed genes after overexpression of Npbwr1.**
**A, B**) NAc from mice that were infected with AAVs expressing GFP or OE-Npbwr1-GFP was submitted to RNA-sequencing. **A**) Heatmap of the significantly altered gene products. **B**) Table with the same gene products sorted for adjusted P-value (Padj) and log 2 fold change (log2FC).
- **Figure 10:**: **Npbwr1-ligands alter Bdnf-signaling and depressive-like behavior.**
**A**) Overview over ligand binding of the agonist neuropeptide B (NPB) and the antagonist CYM50769 to Npbwr1. **B**) 1nmolar NPB or **C,D**) 1 µmolar CYM50769 or was microinfused and tissue was collected 24 h (**B, C**) or 7 days later (**D**) and analysed by qPCR. Statistics: Student's t-test. **B-D**) n = 6-7. **B**) NPB decreases *Bdnf.* P < 0.01**. **C**) CYM50769 increases *Bdnf.* P < 0.05^{∗}. ) CYM50769 effect on *Bdnf* persists at the 7 day time point. P < 0.01**. **E-G**) Mice underwent CVS (vs. naive controls) and were microinfused in the end of the active phase with NPB or CYM50769 after the last stress induction. Tests for depressive-like behavior were conducted app. 24 h later in the dark phase. **E**) Forced swim test. NPB increases immobility time in naive mice, while CYM50769 blocks CVS-effects. n = 8-9 per group; 2-way ANOVA: CVS-effect: F(1,51) = 8.57, P = 0.01; ligand effect: F(2,51) = 26.96, P < 0.01; interaction: F(2,51) = 4.70, P = 0.01; Bonferroni posthoc-test: CVS-effect within ACSF: ***P < 0.001; NPB-effect within naive: **P < 0.01; CYM50769-effect wihtin CVS: ***P < 0.001; all others n.s. **F**) Splash test. NPB may reduce grooming in naive mice, while CYM50769 blocks CVS-effects. n = 8-9 per group; 2-way ANOVA: interaction: F(2,46) = 3.17, P = 0.05. **G**) Sucrose preference. NPB and CVS reduce the ratio of sucrose solution consumed, while CYM50769 rescues CVS-effects. n = 9-10 per group; 2-way ANOVA: effect of ligands: F(2,52) = 13.68, P < 0.0001; interaction: F(2,52) = 9.29, P < 0.01; Bonferroni posthoc-test: CVS-effect within ACSF: ***P < 0.001; NPB-effect within naive: **P < 0.01; CYM50769-effect within CVS: ***P < 0.001; all others n.s. **B-G**) Independent data points are plotted and means ± s.e.m. are shown. Scetches were made with biorender.com.

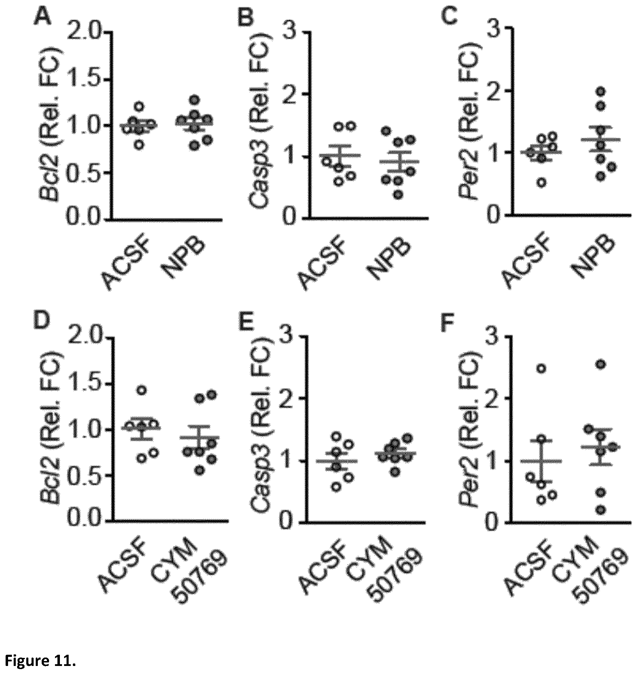

- **Figure 11:**: **Acute microinfusion of Npbwr1-ligands is selective and non-toxic.**
**A-C**) 1nmolar NPB **D-F**) 1 µmolar CYM50769 was microinfused into the NAc. Tissue was collected 24 h later and analysed by qPCR. Statistics: Student's t-test. n = 6-7. Average +/- s.e.m. shown. **A**) No effect of NPB on *Bcl2.* P = 0.882. **B**) NPB does not alter *Casp3.* P = 0.66. **C**) *Per2* is not affected by NPB. P = 0.38. **D**) No effect on *Bcl2.* P = 0.58. **E**) CYM50769 does not alter *Casp3.* P = 0.42. **F**) *Per2* is not affected by CYM50769. P = 0.63.

### Examples

### Materials and Methods

Animals and Licenses. Mice were housed in accordance with the ethical guidelines of the Thuringer Landesamt für Verbraucherschutz (TLV). Experiments were conducted under Animal license UKJ-18-036 and UKJ-21-12 (Germany), which are complying to the EU Directive 2010/63/EU guidelines for animal experiments. Experiments with genetically modified organisms were performed according to S1-regulations according to the GenTAufzV. C57Bl/6J mice were bred in the animal facility (FZL) of the Universitätsklinikum Jena, Germany and purchased from Janvier labs (Saint Berthevin Cedex, France). Both sexes were used as stated. Mice were housed in a 12L:12D light-cycle.

Drugs and chemicals. Mice were intraperitoneally (i.p.) injected with 7.5 mg/kg caffeine (#, C0750, Sigma) or saline at an injection volume of 10ml/kg body weight and tested 2 or 24 h later. NPB ((#CSB-MP015971HU-100, Cusabio) was injected at doses of 1, 3 and 10 nM into the NAc.CYM50769 (#1067-25mg, Sigma Aldrich) was injected into the NAc at doses of 0.1-10 µM.

RNA purification and quantification. RNA was purified by resuspension in Trizol and chloroform-precipitation. RNA was washed in isopropanol and 75% Ethanol. After cDNA-conversion with a GoScript^{™} Reverse Transcriptase kit (#A5001, Promega), quantitative realtime-PCR was performed on a Bio-rad CFX96 Real-time system. Primer sequences are listed in the supplementary material. Quantitative PCR results were processed as described ²².

AAVs, stereotaxic surgery and microinjection. Bilateral stereotaxic surgery into the NAc was essentially performed as described ¹³. The following three viruses were utilized: pAAV.1-CAG-GFP (#37825, Addgene), OE-Npbwr1-GFP: pAAV-CAG-GFP-P2A-Npbwr1-WPRE1, KD-Npbwr1-GFP: pAAV-U6-shRNA-Npbwrl#1-CAG-GFP-P2A-WPR3 (NPBWR1-AAVs custom-made by Charitè viral vector core, Berlin, Germany).

Behavioral tests and CVS. The acute behavioral tests forced swim test, tail suspension test, sucrose preference, splash test and novelty suppressed feeding were performed as described ^{13,23}. After CVS, no tail suspension test was conducted, as tail suspension is part of the stress-induction protocol. For CVS-groups, caffeine was injected at the last day of CVS (day 21), just prior to the stressor. The CVS-protocol was performed as described ¹³. In brief, mice received 21 days of stress with one of three stressors presented in a semi-random order, where the same stressor does not occur on two consecutive days. Stressors consist of one hour of either, tube restraint, tail suspension or 100 mild electric random food shocks. If only female experimenters were present, a used male t-shirt was wrapped in clean protective clothing from the animal unit and placed into the experimental room in order to avoid variability due to sex-specific scents of the scientists ²⁴. All experiments were conducted in the light phase of the light-cycle to allow comparability with previous experiments ²².

Dendritic spine analysis. The analysis was based on detecting the AAVs' GFP-fluorophores. Photos of 40 µm PFA-fixed brain sections were taken with a Zeiss LSM 880 confocal microscope using the AiryScan method. Maximum intensity projections were obtained using Zen Black and Zen Blue software and analysed in NeuronStudio (CNIC, Mount Sinai School of Medicine). The total density of spines, proportions of thin, mushroom and stubby spines as well as the cumulative neck-length were determined in Graph Prism. Neck-containing mushroom density refer to the sum of thin and mushroom spine densities.

Next-generation RNA-sequencing. An in-house RNA-sequencing analysis pipeline was applied as described ²⁵. After artificial depletion of ribosomal RNA (rRNA) transcripts from the data set, more than 90% of reads aligned to the reference genome GRCm38, indicating excellent sample quality. Only changes that reached an adjusted P-value < 0.05 and a log2FC 0.5 <> - 0.5 were further considered.

Postmortem samples. Experiments were conducted in agreement with the Ethics committee of Jena University Hospital, Germany (Reg.-Nr. 2020-1862-Material). Groups were balanced forage and postmortem interval. Samples with age < 22-80 < years and postmortem interval > 130h were excluded.

Statistics. Statistical analysis was performed in GraphPrism. Two-tailed Student's t-test was used for comparison of two groups. Two-way ANOVA was used, when two factors were varied. Additionally, the Bonferroni post-hoc test was used. The cumulative head diameter of dendritic spines was analyzed using the Gehan-Breslow-Wilcoxon test ²⁶. Outliers were removed when the for data points that were more than two standard deviations away from the average. During behavioral tests and dendritic spine analysis, the experimenters were blind to groups.

### Example 1: Caffeine rapidly ameliorates effects of chronic stress.

Rapid mood-elevating effects of caffeine were previously observed in naive mice ¹⁴. Hence, it was hypothesized that caffeine may also improve symptoms induced by chronic variable stress (CVS) in mice, a superior model for sex-specific transcriptional changes of MDD ^{12,13}. It was observed that an acute dose of caffeine 24h prior to testing improved stress-induced behavioral changes **(****Figure 1****).**

Furthermore, mice were stereotaxically injected with a GFP-expressing AAV in order to assess NAc dendritic spine morphology **(****Figure 1****).** In agreement with the literature, CVS increased the density of neck-containing spines and the cumulative head diameter in the NAc of female mice **(****Figure 1****)** ²⁷. Male mice showed only subtle stress-induced changes in neuromorphology **(****Figure 2****).** Importantly, 24h after injection, stress-induced spine changes were reversed by caffeine back to naive levels **(****Figure 1****).** This suggests that caffeine can rapidly reverse stress effects on behavior and neuromorphology.

### Example 2: Npbwr1 is altered by caffeine in a diurnal and T75-DARPP-32 dependent manner.

It was recently revealed that a caffeine-induced diurnal signaling cascade in the NAc. Caffeine alters transcription via Thr75-DARPP-32, which directly binds to the CLOCK/BMAL1 transcriptional complex and thereby regulates gene expression ¹⁴. Accordingly, mood-elevating effects of caffeine only occurred in the active (dark) phase in both sexes and were blocked by the T75A-DARPP-32 knock-in mutation (T75A) ¹⁴. They started at the earliest measured time point (45 min) and persisted at least until 24h later ¹⁴. Hence, it was hypothesized that the described pathway mediates rapid mood-elevating effects of caffeine.

To determine caffeine-regulate transcripts, next-generation RNA-sequencing 2h after caffeine-injection on NAc of wildtype (WT) and T75A mice were performed. Tissue was collected at four time points along the diurnal clock ¹⁴. As expected, significant changes in circadian markers were observed, which oscillated in the expected manner across the four measured time points (*Per1, Per2, Cry1, Bmal),* both in WT and T75A-DARPP-32 mutant mice **(****Figure 3**). Consistent with previous data, it has been observed that most caffeine-induced transcriptional changes occurred in WT in the active (dark) phase **(****Figure 4A**, B). Altered transcripts belonged to a variety of cellular signaling pathways, including synaptic transmission, transcriptional regulation and hormonal signaling **(****Figure 4C****).**

*Npbwr1* was increased by caffeine in the end of the dark phase in WT mice but not T75A mutants (**Figure 3**). This was confirmed in an independent cohort **(****Figure 4D**, E). Surprisingly, while caffeine increased *Npbwr1*-expression 2h after injection, Npbwrl-levels were decreased 24h later **(****Figure 4F****).** This suggests a time-course specific correlation between caffeine, mood and Npbwr1.

Next it was assessed whether *Npbwr1* is affected by chronic stress. The expression of *Npbwr1* was increased after CVS in both sexes. The effect was more pronounced in females **(****Figure 4G,** H). Additionally, Npbwr1 protein levels were increased after CVS **(****Figure 4I****,** **Figure 2****).** Moreover, transcription of *NPBWR1* was increased in the NAc of postmortem tissue from depressed patients of both sexes **(****Figure 4J****,** **Figure 5**). These data suggest that *Npbwr1* is associated with mood and MDD and can be altered using the CVS-model.

### Example 3: Npbwr1 causally mediates symptoms of stress.

In order to investigate whether Npbwr1 is causally linked to morphological and behavioral consequences of stress, genetically modified AAVs to overexpress (OE) or knockdown (KD) Npbwr1 in the NAc were obtained. AAVs induce stable long-term changes in gene products, allowing a manipulation of Npbwr1 over the entire time course of CVS. Experiments were only continued in female mice, given the stronger effect size.

OE of Npbwr1 mimicked chronic stress-effects on behavior and dendritic spines **(****Figure 6**) Specifically, OE-Npbwr1 reduced escape behavior in tail suspension and forced swim tests **(****Figure 6D**, E), decreased the time spent grooming in the splash test **(****Figure 6G****)** and the amount of sucrose solution vs. water consumed **(****Figure 6H****).** The density of neck-containing spines, but not of stubby spines, was increased (Figure 6J-K). The cumulative head diameter was reduced **(****Figure 6L****),** suggesting an increase of neck-containing spines with small head diameters ("thin" spines).

In turn, KD of Npbwr1 prevented certain consequences of CVS on behavior and dendritic spines **(****Figure 7****).** In this cohort, the forced swim test was most sensitive to CVS and showed a reversal by KD of Npbwr1 (Figure 7D-G). Additionally, KD reversed CVS-effects on neck-containing spines and cumulative head diameter (Figure 7H-N), consistent with the observations from **Figure 2****.** OE or KD of Npbwr1 did not affect the body weight of mice or measures of anxiety and locomotor skills **(****Figure 8****).**

Next, Npbwrl-downstream signaling was assessed, which is virtually unknown. To that end RNA-sequencing on NAc tissue that was injected with OE-Npbwrl AAV or a GFP-expressing control was performed. Surprisingly, only 7 genes were significantly altered **(****Figure 9**). However, among them was Brain derived neurotrophic factor (Bdnf), which is highly associated with MDD and antidepressant response ²¹. Taken together, these data demonstrate a causal link between Npbwr1 and symptoms of stress. However, the AAV-based experimental approach cannot reflect the fast-acting nature of Npbwr1 changes as observed after caffeine administration.

### Example 4: Microinjection of Npbwrl ligands rapidly affects depressive-like behavior.

In order to address the rapid acting aspects of the Npbwrl-pathway, ligands were microinjected into the NAc that activate (NPB) or inhibit (CYM50769) Npbwr1. While NPB is a natural neurotransmitter, CYM50769 is a synthetic ligand, which has not been tested *in vivo.* Hence, dosing, selectivity and toxicity were assessed first. Doses of 0.1 µM, 1 µM and 10 µM CYM50769 were injected and mice were scored for health markers for 7 days. No effects on body weight, state of fur, breathing, posture, stereotypies or movement were observed (data not shown, all mice scored "0").

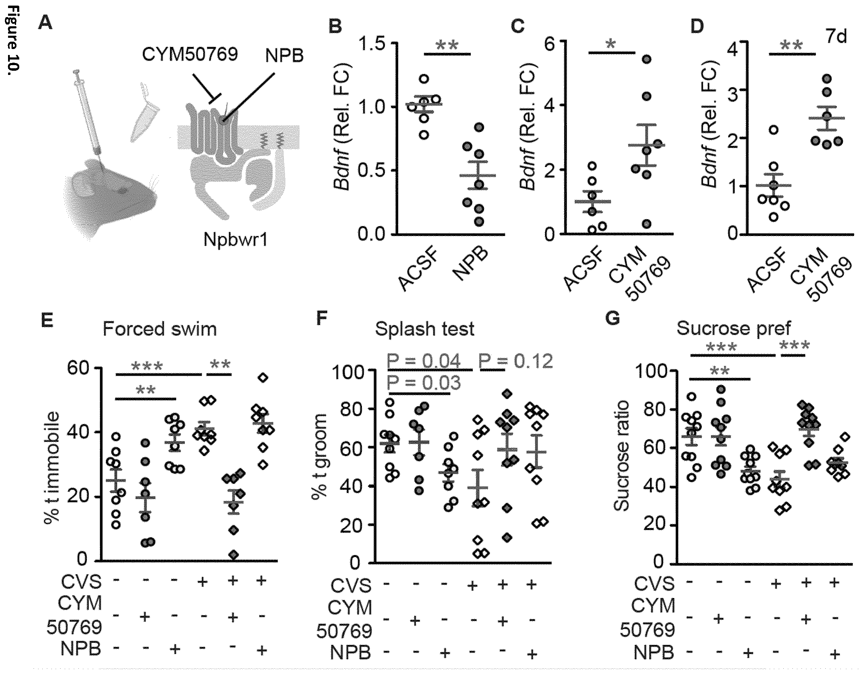

Both, CYM50769 and NPB altered *Bdnf*-levels, albeit as expected in opposite directions. The ligand concentrations with the clearest effects on *Bdnf* were tested further (1 µm CYM50769, 1 nM NPB; Figure 10A-D). Neither NPB nor CYM50769 affected the apoptosis markers *Bcl2* and *Cosp3* **(****Figure 11****).** Moreover, they did not alter levels of the circadian gene *Per2* **(****Figure 11****),** suggesting a selectivity for the *Bdnf-*pathway.

Next, the rapid effects of Npbwr1 activity were tested on depressive-like behaviors *in vivo.* To that end, mice underwent CVS and were microinjected with 1 nM NPB and 1 µm CYM50769 after the last session of stress induction. Behavioral tests were conducted 24 h later. It was observed that NPB elicited depressive-like behaviors in naïve mice in forced swim, splash and sucrose preference tests while not enhancing the behavioral effects of CVS further (Figure 10E-G). In contrast, CYM50769 had no effects in naïve mice. However, it rescued CVS-effects in all tests (Figure 10E-G). These data are consistent with the effects of viral-mediated OE and KD of *Npbwr1* levels **(****Figures 6**, 7), pointing towards a pro-depressive effect of *Npbwrl1* which is ameliorated when levels or activity of the receptor are reduced. Importantly, *Npbwr1* ligands demonstrated fast-acting effects on behavior, with the antagonist CYM50769 showing promise in rapidly reversing effects of chronic stress.

### Summary and Discussion

This study describes a previously unknown pathway, which mediates rapid effects on depressive-like behaviors and stress response via *Npbwr1.* Moreover, selective alterations on the levels of *Bdnf* and dendritic spine morphology were shown. Viral-mediated gene transfer stably altered levels of *Npbwrl.* While overexpression of *Npbwrl* mimicked depressive-like symptoms, knockdown prevented chronic stress effects. This dichotomous effect may be explained with relatively low baseline levels of *Npbwrl* in the NAc as observed by Cq-values of Npbwr1 during qPCR of more than 25 in naive mice. Hence, effects on Npbwrl-regulation may only become apparent when Npbwrl-levels are upregulated, e.g., after CVS. Consistently, regulation of Npbwrl-activity via microinjection of ligands altered depression-related symptoms. Stimulation of Npbwr1 via NPB increased depressive-like behaviors while inhibition with CYM50769 blocked those within the CVS-cohort. Taken together, these data provide evidence that Npbwrl-signaling rapidly alters depressive-like symptoms in a causal manner.

The *Npbwr1*-pathway is rapidly regulated by caffeine, which in mice has acute resilience promoting effects. However, caffeine was used in the experiments above as a research tool since it is already widely used in the human population and can have adverse effects on health in some individuals. The seemingly contradictory observed effects of caffeine on Npbwr1 after 2 vs. 24 h are consistent with a report, where introcerebroventricular injection of NPB into mouse brain induced hyperphagia after 2 h, followed by hypophagia ¹⁵.

In agreement with previous findings, caffeine effects on *Npbwr1*-signaling occurred in a diurnal manner ¹⁴. This is consistent with the literature, in which diurnal effects of NPW in feeding behavior have been reported as well with effects only occurring during the active phase ²⁹.

CYM50769 has been developed as a selective antagonist of Npbwr1 *in vitro.* Here, this compound was further explored for the first time in an *in vivo*-model. It has been shown that an acute dose of this drug is selective and well tolerated.

Taken together, this study demonstrates that *Npbwrl* as a key mediator of depression and stress response and that corresponding antagonists like, e.g., CYM50769 are potential compounds to rapidly modulate this pathway in a beneficial manner.

### Literature

1. Bebbington, P. The World Health Report 2001 - Mental Health: New Understanding, New Hope. Soc. Psychiatry Psychiatr. Epidemiol. 36, 473-4 (2001).
2. Vos, T. et al. Global, regional, and national incidence, prevalence, and years lived with disability for 328 diseases and injuries for 195 countries, 1990-2016: A systematic analysis for the Global Burden of Disease Study 2016. Lancet 390, 1211-59 (2017).
3. Galea, S., Merchant, R. M. & Lurie, N. The Mental Health Consequences of COVID-19 and Physical Distancing: The Need for Prevention and Early Intervention. JAMA Intern Med Epub ahead, (2020).
4. Torales, J., O'Higgins, M., Castaldelli-Mala, J. & Ventriglio, A. The outbreak of COVID-19 coronavirus and its impact on global mental health. Int J Soc Psychiatry 2076402091, (2020).
5. Sanchez-Roige, S. & Palmer, A. A. Emerging phenotyping strategies will advance our understanding of psychiatric genetics. Not. Neurosci. 23, 475-80 (2020).
6. Pappa, S. et al. Prevalence of depression, anxiety, and insomnia among healthcare workers during the COVID-19 pandemic: A systematic review and meta-analysis. Brain. Behav. Immun. 88, 901-7 (2020).
7. Vindegaard, N. & Eriksen Benros, M. COVID-19 pandemic and mental health consequences: Systematic review of the current evidence. Brain. Behav. Immun. 89, 531-42 (2020).
8. Dubey, S. et al. Psychosocial impact of COVID-19. Diabetes Metab. Syndr. Clin. Res. Rev. 14, 779-88 (2020).
9. Rajkumar, R. P. COVID-19 and mental health: A review of the existing literature. Asian J. Psychiatr. 52, 102066 (2020).
10. Tomonaga, Y. et al. The economic burden of depression in Switzerland. Pharmacoeconomics 31, 237-50 (2013).
11. Greenberg, P. E., Fournier, A. A., Sisitsky, T., Pike, C. T. & Kessler, R. C. The economic burden of adults with major depressive disorder in the United States (2005 and 2010). J. Clin. Psychiatry 76, 155-62 (2015).
12. Scarpa, J. et al. Shared Transcriptional Signatures in Major Depressive Disorder and Mouse Chronic Stress Models. Biol. Psychiatry (2020).
13. Labonte, B. et al. Sex-Specific Transcriptional Signatures in Human Depression. Not. Med. 23, 1102-1111 (2017).
14. Trautmann, C., Burek, D., Huebner, C., Girault, J.-A. & Engmann, O. A regulatory pathway linking caffeine action, mood and the diurnal clock. Neuropharmacology 172, 108133 (2020).
15. Tanaka, H. et al. Characterization of a family of endogenous neuropeptide ligands for the G protein-coupled receptors GPR7 and GPR8. Proc. Natl. Acad. Sci. U. S. A. 100, 6251-6 (2003).
16. Eipper-Mains, J. E. et al. Effects of cocaine and withdrawal on the mouse nucleus accumbens transcriptome. Genes, Brain Behav. (2013). doi:10.1111/j.1601-183X.2012.00873.x
17. Dvorakova, M. C. Distribution and function of neuropeptides W/B signaling system. Front. Physiol. 9, 981 (2018).
18. Uchio, N. et al. Circadian characteristics of mice depleted with GPR7. Biomed. Res. (2009). doi:10.2220/biomedres.30.357
19. Dun, S. L. et al. Neuropeptide B immunoreactivity in the central nervous system of the rat. Brain Res. (2005). doi:10.1016/j.brainres.2005.03.024
20. Singh, G., Maguire, J. J., Kuc, R. E., Fidock, M. & Davenport, A. P. Identification and cellular localisation of NPW1 (GPR7) receptors for the novel neuropeptide W-23 by [125I]-NPW radioligand binding and immunocytochemistry. Brain Res. (2004). doi:10.1016/j.brainres.2004.03.079
21. Cubillos, S., Engmann, O. & Brancato, A. BDNF as a Mediator of Antidepressant Response: Recent Advances and Lifestyle Interactions. Int. J. Mol. Sci. 23, (2022).
22. Trautmann, C., Bock, A., Urbach, A., Hübner, A. & Engmann, O. Acute vitamin B12 supplementation evokes antidepressant response and alters Ntrk-2. Neuropharmacology 171, 108112 (2020).
23. Scott, M. M. et al. Hcrtr1 and 2 signaling differentially regulates depression-like behaviors. Behav. Brain Res. (2011).
24. Sorge, R. E. et al. Olfactory exposure to males, including men, causes stress and related analgesia in rodents. Not. Methods 11, 629-32 (2014).
25. Lott, S. C. et al. Customized workflow development and data modularization concepts for RNA-Sequencing and metatranscriptome experiments. J. Biotechnol. 261, 85-96 (2017).
26. Engmann, O. et al. DARPP-32 interaction with adducin may mediate rapid environmental effects on striatal neurons. Not. Commun. 6, 10099 (2015).
27. Duman, C. & Duman, R. Spine synapse remodeling in the pathophysiology and treatment of depression. Neurosci Lett 601, 20-29 (2015).
28. Koo, J. W., Chaudhury, D., Han, M. H. & Nestler, E. J. Role of Mesolimbic Brain-Derived Neurotrophic Factor in Depression. Biol. Psychiatry 86, 738-748 (2019).
29. Aikawa, S., Ishii, M., Yanagisawa, M., Sakakibara, Y. & Sakurai, T. Effect of neuropeptide B on feeding behavior is influenced by endogenous corticotropin-releasing factor activities. Regul. Pept. (2008). doi:10.1016/j.regpep.2008.08.001

## Claims

1. A pharmaceutical composition comprising an antagonist/inhibitor of neuropeptide B/W receptor (NPBWR1)
for use in in a method of treating, ameliorating or preventing a mood disorder/affective disorder and/or chronic stress and/or anxiety disorders and/or Parkinson's disease.

2. Pharmaceutical composition according to claim 1, wherein the mood disorder/affective disorder is selected from the group consisting of:
(i) Manic episode (ICD-10 F30);
(ii) Bipolar affective disorder (ICD-10 F31);
(iii) Depressive episode (ICD-10 F32);
(iv) Recurrent depressive disorder (ICD-10 F33);
(v) Persistent mood affective disorders (ICD-10 F34).
(vi) Other mood affective disorders (ICD-10 F38); and
(vii) Unspecified mood affective disorder (ICD-10 F39).

3. Pharmaceutical composition according to claim 1, wherein the chronic stress is selected from the group consisting of:
(i) Reaction to severe stress, and adjustment disorders (ICD-10 F43);
(ii) Acute stress reaction (ICD-10 F43.0);
(iii) Post-traumatic stress disorder (ICD-10 F43.1);
(iv) Adjustment disorders (ICD-10 F43.2);
(v) Other reactions to severe stress (ICD-10 F43.8); and
(vi) Reaction to severe stress, unspecified (ICD-10 F43.9).

4. Pharmaceutical composition according to claim 1, wherein the anxiety disorder is selected from the group consisting of:
(i) Phobic anxiety disorders (ICD-10 F40); and
(ii) Other anxiety disorders (ICD-10 F41.1).

5. Pharmaceutical composition according to any one of claims 1 to 4, wherein said antagonist/inhibitor is selected from an NPBWR1 inhibitory peptide, NPBWR1 inhibitory small binding molecule, RNAi, siRNA, shRNA, aptamers and intramers specifically directed against NPBWR1, anti-NPBWR1 antisense molecules.

6. Pharmaceutical composition according to any one of claims 1 to 5, wherein said antagonist/inhibitor is an NPBWR1 inhibitory small binding molecule and has the following chemical structure represented by Formula (1) 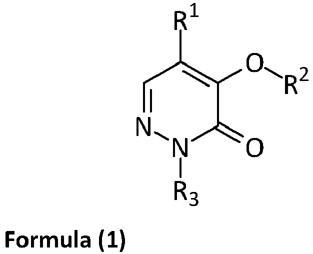 wherein:
R¹ is selected from F, Cl, Br, I and CN;
R² is selected from -(heterocyclyl with 5 to 10 ring atoms and optionally one or more substituents R^{2a}), and -(carbocyclyl with 6 to 10 ring atoms and optionally one or more substituents R^{2a});
R³ is selected from -(heterocyclyl with 5 to 20 ring atoms and optionally one or more substituents R^{3a}), -(C₁₋₄ alkylene which is optionally substituted with one or more substituents R^{Alk})-(heterocyclyl with 5 to 20 ring atoms and optionally one or more substituents R^{3a}), -(carbocyclyl with 6 to 20 ring atoms and optionally one or more substituents R^{3a}), and -(C₁₋₄ alkylene which is optionally substituted with one or more substituents R^{Alk})-(carbocyclyl with 6 to 20 ring atoms and optionally one or more substituents R^{3a}),
wherein
each R^{2a} is independently selected from the group consisting of -halogen, -CN, -CF₃, -CHF₂, -CH₂F, -OCF₃, -OCHF₂, -OCH₂F, -NR^{∗}R^{∗}, -NR*COR*, -NR*C(O)NR*R*, - NR^{∗}S(O₂)NR^{∗}R^{∗}, -C(O)OR*, -C(O)NR*R*, -OH, or -O-C₁₋₆ alkyl, wherein each R* is independently selected from H or C₁₋₆ alkyl or C₁₋₆ cycloalkyl;
each R^{3a} is independently selected from the group consisting of -halogen, -CN, -CF₃, -CHF₂, -CH₂F, -OCF₃, -OCHF₂, -OCH₂F, -NR*R*, -NR*COR*, -NR*C(O)NR*R*, - NR^{∗}S(O₂)NR^{∗}R^{∗}, -C(O)OR*, -C(O)NR*R*, -OH, or -O-C₁₋₆ alkyl, wherein each R* is independently selected from H or C₁₋₆ alkyl or C₁₋₆ cycloalkyl;
each R^{Alk} is independently selected from the group consisting of-halogen and -CN;
or a pharmaceutically acceptable salt, solvate or prodrug thereof.

7. The pharmaceutical composition according to claim 6, wherein one or more of the following are fulfilled in Formula (1):
a) R¹ is selected from F, Cl, Br and CN; preferably from F, Cl and CN; more preferably from F and Cl; even more preferably from Cl;
b) R² is selected from -(heteroaryl with 5 to 10 ring atoms and optionally one or more substituents R^{2a}), and -(aryl with 6 to 10 ring atoms and optionally one or more substituents R^{2a}); preferably from -(heteroaryl with 5 to 7 ring atoms and optionally one or more substituents R^{2a}), and -(aryl with 6 or 10 ring atoms and optionally one or more substituents R^{2a}); more preferably from benzene, naphthalene, pyrrole, furan, imidazole, pyrazole, oxazole, thiazole and pyridine, any of which may optionally be substituted with one or more substituents R^{2a}; even more preferably from benzene, naphthalene, imidazolidine, oxazole and pyridine, any of which may optionally be substituted with one or more substituents R^{2a}; still more preferably from benzene, imidazolidine, oxazole and pyridine, any of which may optionally be substituted with one or more substituents R^{2a}; even still more preferably from benzene which may optionally be substituted with one or more substituents R^{2a}; still even more preferably from benzene which substituted with one or more substituents R^{2a}; most preferably from benzene being substituted at 4-position with R^{2a};
c) R³ is selected from -(heterocyclyl with 5 to 14 ring atoms and optionally one or more substituents R^{3a}), -(C₁₋₄ alkylene which is optionally substituted with one or more substituents R^{Alk})-(heterocyclyl with 5 to 14 ring atoms and optionally one or more substituents R^{3a}), -(carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); preferably from -(carbocyclyl with 6 to 14 ring atoms and optionally one or more substituents R^{3a}), and -(C₁₋₄ alkylene which is optionally substituted with one or more substituents R^{Alk})-(carbocyclyl with 6 to 14 ring atoms and optionally one or more substituents R^{3a}); more preferably from -(aryl with 6 to 14 ring atoms and optionally one or more substituents R^{3a}), and -(C₁₋₄ alkylene which is optionally substituted with one or more substituents R^{Alk})-(aryl with 6 to 14 ring atoms and optionally one or more substituents R^{3a}); even more preferably from -(aryl with 6 to 10 ring atoms and optionally one or more substituents R^{3a}), and -(C₁₋₄ alkylene which is optionally substituted with one or more substituents R^{Alk})-(aryl with 6 to 10 ring atoms and optionally one or more substituents R^{3a}); still more preferably from -(aryl with 6 or 10 ring atoms and optionally one or more substituents R^{3a}), and -(C₁₋₄ alkylene which is optionally substituted with one or more substituents R^{Alk})-(aryl with 6 or 10 ring atoms and optionally one or more substituents R^{3a}); even still more preferably from -(phenyl having one or more substituents R^{3a}), and -(C₁₋₄ alkylene which is optionally substituted with one or more substituents R^{Alk})-(naphthyl with optionally one or more substituents R^{3a}); still even more preferably 2,5-dimethylphenyl or 1-naphthylmethyl;
d) C₁₋₄ alkylene is preferably methylene or ethylene, more preferably methylene;
e) there are none, one, two or three substituents R^{2a;} preferably none, one or two substituents R^{2a}; more preferably one substituent R^{2a}; even more preferably one substituent R^{2a} in 4-position;
f) there are none, one, two or three substituents R^{3a;} preferably none, one or two substituents R^{3a}; more preferably two substituents R^{3a} in case R³ does not contain the -(C₁₋₄ alkylene) group, and more preferably one or no substituent R^{3a} in case R³ contains the -(C₁₋₄ alkylene) group;
g) there are none, one, two or three substituents R^{Alk;} preferably none, one or two substituents R^{Alk}; more preferably noen or one substituent R^{Alk}; even more preferably no substituent R^{Alk};
h) each R^{2a} is independently selected from the group consisting of -halogen, -CN, C₁₋₆ alkyl, C₁₋₆ haloalkyl, C₁₋₆ cycloalkyl, -NR*R*, -NR*COR*, -NR*C(O)NR*R*, - NR^{∗}S(O₂)NR^{∗}R^{∗}, -C(O)OR*, -C(O)NR*R*, -OH, -O-C₁₋₆ alkyl or -O-C₁₋₆ haloalkyl, wherein each R* is independently selected from H or C₁₋₆ alkyl or C₁₋₆ cycloalkyl; preferably each R^{2a} is independently selected from the group consisting of -halogen, - CN, C₁₋₃ alkyl, C₁₋₃ haloalkyl, cyclopropyl, -NR*R*, -NR*COR*, -NR*C(O)NR*R*, - C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; more preferably each R^{2a} is independently selected from the group consisting of-halogen, -CN, C₁₋₃ alkyl, -NR*R*, -NR*COR*, -C(O)OR*, -C(O)NR*R*, -OH, -O-C₁₋₃ alkyl or -O-C₁₋₃ haloalkyl, wherein each R* is independently selected from H or C₁₋₃ alkyl or cyclopropyl; even more preferably each R^{2a} is independently selected from the group consisting of -halogen, - CN, C₁₋₃ alkyl or -O-C₁₋₃ alkyl; still more preferably each R^{2a} is independently selected from the group consisting of -halogen, -CN, or -O-C₁₋₃ alkyl; even more preferably each R^{2a} is independently -O-C₁₋₃ alkyl; still even more preferably each R^{2a} is independently methoxy or ethoxy, most preferably methoxy.
i) each R^{3a} is independently selected from the group consisting of -halogen, -CN, C₁₋₆ alkyl, C₁₋₆ haloalkyl, C₃₋₆ cycloalkyl, -NR*R*, -NR*COR*, -NR*C(O)NR*R*, - NR^{∗}S(O₂)NR^{∗}R^{∗}, -C(O)OR*, -C(O)NR*R*, -OH, -O-C₁₋₆ alkyl or -O-C₁₋₆ haloalkyl, wherein each R* is independently selected from H or C₁₋₆ alkyl or C₃₋₆ cycloalkyl; preferably each R^{3a} is independently selected from the group consisting of -halogen, - CN, C₁₋₆ alkyl, C₁₋₆ haloalkyl, C₃₋₆ cycloalkyl, -O-C₁₋₆ alkyl or -O-C₁₋₆ haloalkyl; more preferably each R^{3a} is independently selected from the group consisting of -halogen, - CN, C₁₋₆ alkyl, C₁₋₆ haloalkyl, or C₃₋₆ cycloalkyl; even more preferably each R^{3a} is independently selected from the group consisting of -halogen, -CN, C₁₋₃ alkyl, or C₁₋₃ haloalkyl; still more preferably each R^{3a} is independently selected from the group consisting of -halogen and C₁₋₃ alkyl; even still more preferably each R^{3a} is independently selected from the group consisting of C₁₋₃ alkyl, such as methyl or ethyl, more preferably methyl;
j) each R^{Alk} is independently selected from the group consisting of -F and -Cl; preferably each R^{Alk} is -F; more preferably each R^{Alk} is absent; and/or
k) the NPBWR1 inhibitory small binding molecule has the following chemical structure represented by Formula (1) or a pharmaceutically acceptable salt or solvate thereof.

8. Pharmaceutical composition according to any one of claims 1 to 7, wherein said antagonist/inhibitor is an NPBWR1 inhibitory small binding molecule and has one of the following chemical structures represented by Formulae (2) to (9): 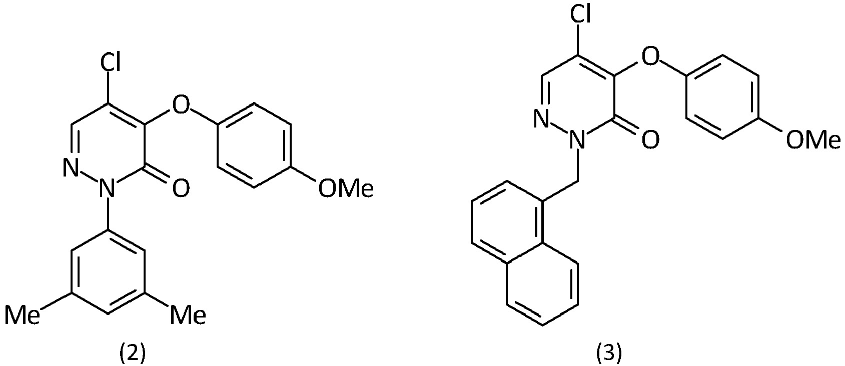 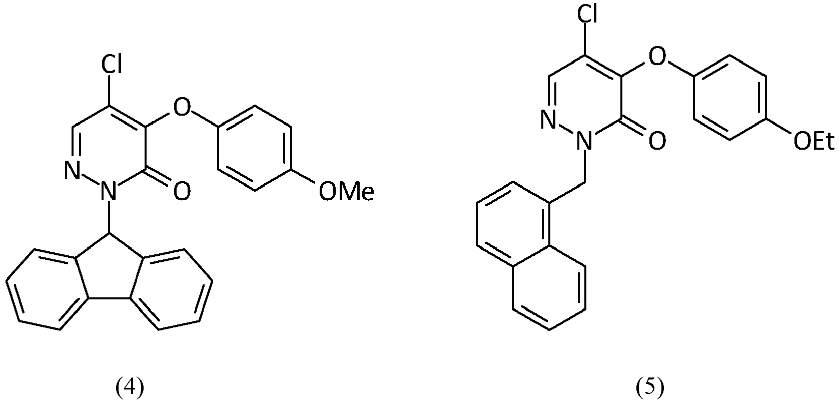 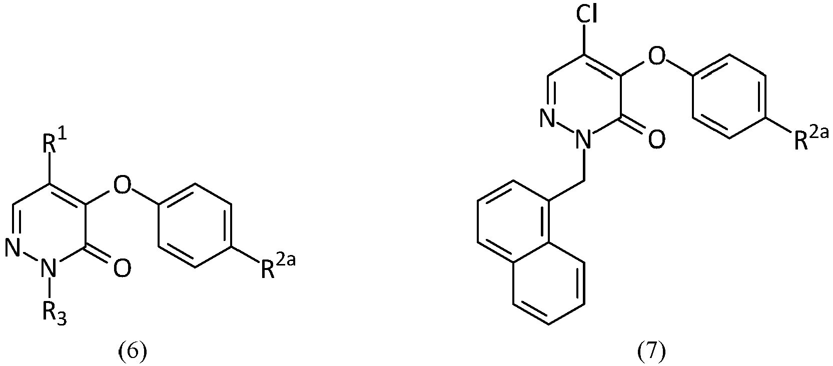 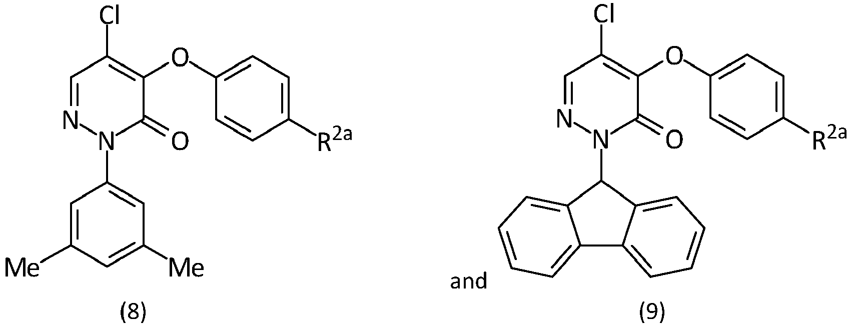 wherein R¹, R^{2a}, and R³ are as defined in claim 6 or 7.

9. Pharmaceutical composition according to any one of claims 1 to 3, wherein said antagonist/inhibitor is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional repressor domain and an epigenetic modification domain capable of repressing the expression of NPBWR1.

10. A pharmaceutical composition comprising an agonist/activator of neuropeptide B/W receptor (NPBWR1)
for use in in a method of treating, ameliorating or preventing a bipolar affective disorder (ICD-10 F31) during the manic phase, appetitive disorders , preferably anorexia or bulimia.

11. Pharmaceutical composition according to claim 10, wherein said agonist/activator is selected from an NPBWR1 activating peptide, NPBWR1 activating small binding molecule and NPBWR1 RNA molecules.

12. Pharmaceutical composition according to claim 10 or 11, wherein said agonist/activator is neuropeptide B or neuropeptide W.

13. Pharmaceutical composition according to any one of claims 9 to 12; wherein said agonist/activator is a CRISPR/Cas system specifically directed against NPBWR1, wherein the Cas-protein of said CRISPR/Cas system is modified to lack its nuclease activity and said Cas-protein is fused to an effector domain selected from the group consisting of a transcriptional activator domain and an epigenetic modification domain capable of activating the expression of NPBWR1.
